# EUROPEAN PATENT APPLICATION

(11) **EP 4 345 101 A1**
(43) Date of publication of application: **03.04.2024**
(21) Application number: 22198899.1
(22) Date of filing: 29.09.2022
(51) Int. Cl.: C07D 471/20, A61K 31/438, A61P 35/00, A61P 35/02, A61P 35/04, A61P 9/00, A61P 37/00, A61P 37/02, A61P 37/06, A61P 11/00, A61P 27/00

(54) **AZOLE DERIVATIVES AS SHP2 INHIBITORS**

(71) Applicant: IRBM S.P.A., 00071 Pomezia (RM) (IT); C.N.C.C.S. S.c.a.r.l. Collezione Nazionale Dei Composti Chimici e Centro Screening, 00071 Pomezia (RM) (IT)
(72) Inventor: PETROCCHI, Alessia, 00071 Pomezia (RM) (IT); MONTALBETTI, Christian, 00071 Pomezia (RM) (IT); DI FABIO, Romano, 00071 Pomezia (RM) (IT); CIAMMAICHELLA, Alina, 00071 Pomezia (RM) (IT); ROSSETTI, Ilaria, 00071 Pomezia (RM) (IT)
(74) Representative: Pace Napoleone, Maria

(57) **Abstract**

The present invention relates to new compounds capable of inhibiting the activity of SHP2 phosphatase. Compounds of the invention can be used for the treatment of disorders associated with SHP2 deregulation. The present invention also relates to pharmaceutical compositions containing said compounds and to their method of synthesis.

## Description

### FIELD OF THE INVENTION

The present invention relates to new compounds capable of inhibiting the activity of SHP2 phosphatase. Compounds of the invention can be used for the treatment of disorders associated with SHP2 deregulation. The present invention also relates to pharmaceutical compositions containing said compounds and to their method of synthesis.

### BACKGROUND OF THE INVENTION

Src homology phosphotyrosine phosphatase 2 (SHP2) encoded by *PTPN11* is a non-receptor protein tyrosine phosphatase (PTP) composed of a C-terminal domain, a PTP domain, and two N-terminal Src homology (N-SH2) domains, that contributes to multiple cellular functions including proliferation, differentiation, cell cycle maintenance and migration. SHP2 is a positive regulator of signalling downstream of several receptor tyrosine kinases through the Ras-mitogen-activated protein kinase, the JAK-STAT or the phosphoinositol-3-kinase-AKT pathways. The protein exists in an inactive, self-inhibited conformation, stabilized by a binding network involving residues from both the N-SH2 domains and the catalytic PTP domain. Recruitment of SHP2 to an activated receptor releases the self-inhibitory conformation and leads to catalytic activation of its phosphatase domain. In addition to its function as a phosphatase, SHP2 also serves as a docking protein to recruit other signalling intermediates through its two amino terminus N-SH2 domains. Since SHP2 is a positive regulator of cellular signalling leading to proliferation, differentiation, and survival, its constitutive activation is associated with oncogenesis. SHP2 emerged as an attractive target for therapeutic targeting in the treatment of various diseases, such as Noonan Syndrome, Leopard Syndrome, juvenile myelocytic leukemias, glioblastoma, neuroblastoma, melanoma, acute myeloid leukemia and cancers of the breast, lung and colon.

Both academic institutions and pharmaceutical companies have disclosed drug discovery programs exploiting SHP2 inhibitors based on different heterocyclic scaffolds.

WO2015/107493, WO2015/107494 and WO2015/107495 from Novartis disclose compounds of general formula (A) as indicated below:

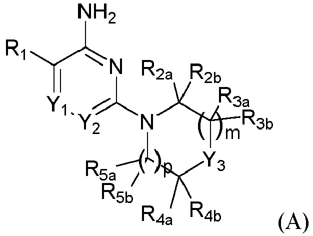

Still from Novartis the compounds of general structures (B), (C), (D) and (E) indicated below are disclosed in, respectively, WO2016/203404, WO2016/203405 and WO2017/216706:

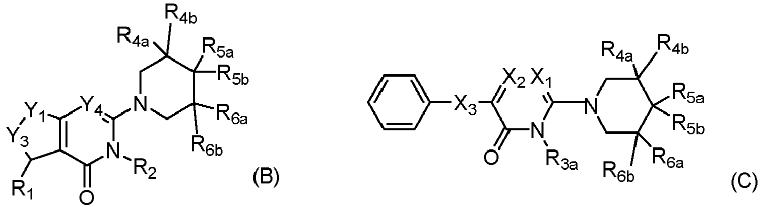

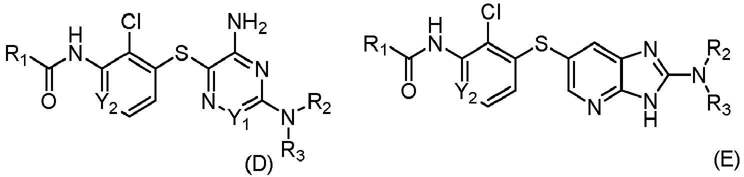

The general structure E disclosed in WO2017/216706 and the compounds therein identified are 2-amino-3H-imidazo[4,5-b]pyridine 5- or 6- thiol derivatives.

Jacobio Pharmaceuticals disclosed in WO2017/211303 and in WO2018/172984 pyrazine derivatives of structures (F) and (G) as indicated below:

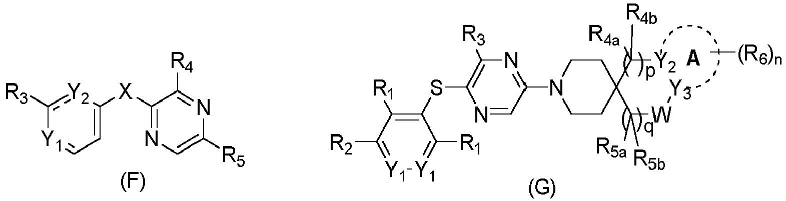

Further pyridine, pyrazine and triazine compounds as allosteric SHP2 inhibitors have been recently disclosed by Revolution Medicines in WO2018/013597, WO2018/136264 and WO2019/075265. WO2018/136265 and WO2019/118909 both relate to bicyclic heteroaromatic scaffolds comprising imidazopyrazines, triazolopyrazines, pyrazolopyridine, imidazopyrimidines of general structure (H):

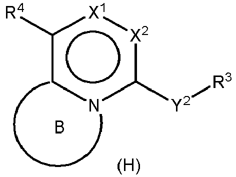

Pyrazolopyrazines and ring-fused pyrimidin-4-ones have been disclosed by the Board of Regents, University of Texas System, in WO2017/210134 and in WO2017/156397, respectively. Pyrazolopyrazines were further disclosed by Relay Therapeutics in WO2018/081091, WO2018/218133, WO2018/057884 and WO2019/067843.

Imidazopyrimidine indicated below are disclosed by Gilead in WO2020/072656:

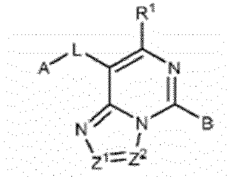

SHP2 therefore represents a highly attractive target for the development of novel therapies for the treatment of various diseases where it is involved. Therefore, there is the need to develop novel therapeutic agents that act as SHP2 inhibitors. The compounds of the present invention fulfil such need since they are small molecules capable of inhibiting the activity of SHP2.

Many of the compounds disclosed in the cited patent and patent application share an heterocyclic core substituted with a tricyclic amine with a general structure indicated below, wherein C-ring is an aromatic or heteroaromatic ring:

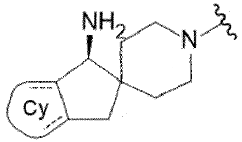

Unexpectedly, the introduction of a suitable azole ring, such as a pyrazole or a pyrrole, with one nitrogen is at the bridgehead in the left-hand side of the molecule combined with a specific selection of central heterocyclic cores and substituents, enabled the identification of potent and selective SHP2 inhibitors, endowed with superior pharmacokinetics in pre-clinical animal species, exhibiting remarkable exposure in plasma after oral administration (both in terms of Cmax and AUC). The compounds of the invention, possibly due also to the presence of the azole moiety either substituted or unsubstituted, are endowed with improved ligand-biological target kinetics, greatly lengthening SHP2 inhibitors residence time (time a ligand spent in contact with its biological target) by reducing its koff. Moreover, many of the present compounds are SHP2 inhibitors capable of penetrating the BBB to reach their target and can be used in the treatment of tumors arising from or disseminating into the CNS.

### DESCRIPTION OF THE INVENTION

The present invention relates to heterocyclic compounds useful as SHP2 inhibitors and for the treatment of conditions mediated by SHP2.

The inventors have found that compounds with a general formula including a five-membered fused heteroaromatic ring with specific nitrogen pattern on the left side of the molecule, act as particularly potent SHP2 inhibitors, as evidenced by both enzymatic and cellular IC₅₀ values for SHP2 inhibition in the low nanomolar or micromolar range. More specifically, compounds of the invention are characterized by a suitable azole ring, such as a pyrazole or a pyrrole, wherein one nitrogen is at the bridgehead in the left-hand side of the molecule combined with a specific selection of central heterocyclic cores and substituents.

It is therefore an object of the present invention a compound of general formula (I): 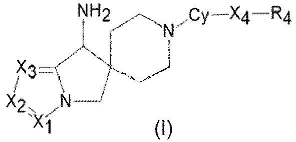 wherein:
X₁ is CR₁ or N; X₂ is CR₂; X₃ is CR₃;
R₁, R₂ and R₃ are each optionally selected from H, halogen, OC₁₋₃alkyl or C₁₋₃alkyl;
X₄ is O, S or X₄ is a bond;
R₄ is an aryl, heteroaryl, partially unsaturated aryl, partially unsaturated heteroaryl ring, each of said aryl, heteroaryl, partially unsaturated aryl, partially unsaturated heteroaryl ring being optionally substituted with one or more substituents independently selected from C(O)CH₃, C(O)OCH₃, OH, halogen, NH₂, NH-C₁-₆alkyl, N(C₁₋₆alkyl)₂, C₁₋₆alkyl, C₃₋₅cycloalkyl, haloC₁₋₆alkyl, hydroxyC₁₋₆alkyl, CONR'R", CN, haloC₁₋₆alkoxy, C₁₋₆alkoxy, saturated 5- or 6-membered heterocyclic ring, aryl or heteroaryl wherein each of said saturated 5- or 6-membered heterocyclic ring, aryl or heteroaryl is optionally substituted with one or more CH₃, NH₂, halogen or OH and wherein R' and R" are each independently H or C₁₋₆alkyl, preferably H or CH₃;
Cy-X₄-R₄ corresponds to anyone of general formulae (A) - (F) as indicated below: 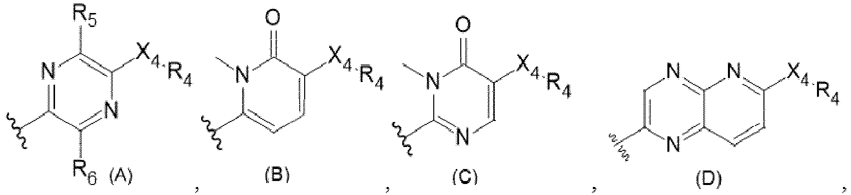 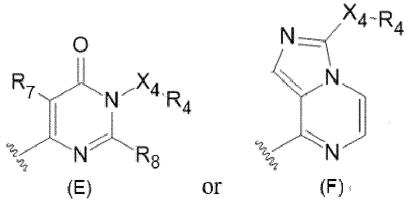 wherein:
   - R₅ is selected from H, C₁₋₃alkyl and NH₂; preferably from H, CH₃ and NH₂;
   - R₆ R₇, R₈ are each independently selected from H and C₁₋₃alkyl;
   or a pharmaceutically acceptable salt, solvate, or stereoisomer thereof.

Preferably, Cy-X₄-R₄ corresponds to the general formula (A) wherein R₅ and R₆ are H.

In a preferred embodiment the compound of formula (I) according to claim 1 is a compound of general formula (IA) or (IB):

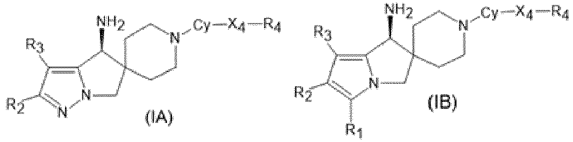

Preferably R₁, R₂ and R₃ are each independently selected from H, Br, Cl, F and CH₃.

Preferably R₄ is selected from: phenyl, pyridine, pyrimidine, pyrazine, quinoline, imidazo[1,2-a]pyridine, indole, 2H-indazole, 2,3-dihydroindole, 2,3,3a,4-tetrahydro-1H-pyrrolo[2,1-c][1,4]benzoxazine, 6a,7,8,9-tetrahydro-6H-pyrido[3,2-b]pyrrolo[1,2-d][1,4]oxazine, 2,3-dihydro-1H-pyrrolo[2,3-b]pyridine, 1,8-napthyridine, triazolo[4,3-a]pyridine, imidazo[1,2-a]pyridin-2(3H)-one, each of said ring being optionally substituted with one or more substituents independently selected from C(=O)CH₃, C(=O)OCH₃, OH, halogen, NH₂, CF₃, CHF₂, C₁₋₃alkyl, C₁₋₃alkoxy, C(=O)NH₂, C(=O)NHC₁₋₃alkyl, C(=O)N(C₁₋₃alkyl)₂, CN, C₃₋₅cycloalkyl, aryl or heteroaryl wherein each of said aryl or heteroaryl is optionally substituted with one or more halogen, C(=O)NH₂, C(=O)NHCH₃, C(=O)N(CH₃)₂, C₁₋₃alkyl and NHC(=O)CF₃. More preferably R₄ is quinoline optionally substituted with one or more substituents independently selected from OH, OC₁-₃alkyl, halogen, NH₂, CF₃, CHF₂, C₁₋₃alkyl, C(=O)NH₂, C(=O)NHC₁₋₃alkyl, C(=O)N(C₁₋₃alkyl)₂, CN, C₃₋₅cycloalkyl.

More preferably R₄ is selected from the group consisting of: 2,3-dichlorophenyl, 3-fluoro-2-methylpyridin-4-yl, 2,3-difluorophenyl, 5-(4-fluorophenyl)pyrazin-2-yl, 2-chloro-3-(1H)-pyrazolyl, 2-fluoro-3-methylphenyl, 4-fluorophenyl, 2-chloro-3-fluorophenyl, 3-chloro-2-methylphenyl, 2-chloro-6-fluoro-3-methylphenyl, 4-(trifluoromethoxy)phenyl, 4-(trifluoromethyl)phenyl, 3-chlorophenyl, 2-fluorophenyl, 2,4-dichlorophenyl, 3,4-dichlorophenyl, tolyl, phenyl, pyridyl, 2,4-difluorophenyl, 2-chloro-4-fluorophenyl, 2-chloro-3-methylphenyl, 8-methoxyquinolin-4-yl, 2,3-dihydrobenzofuran-4-yl, 5-quinolin-4-yl, 4-chloro-2-methylphenyl, 2-(trifluoromethyl)phenyl, 2,3-dihydrobenzofuran-5-yl, trifluoromethylbenzonitrile, 2-chloro-3-(1H-imidazol-1-yl)phenyl, 2,4-difluoro-3-methylphenyl, 2,3-dihydro-1H-inden-4-yl, 2,3-dimethylphenyl, 2,5-difluorophenyl, 2,3,4-trifluorophenyl, 3,5-difluorophenyl, 3-fluorophenyl, 1,3-dihydroisobenzofuran-4-yl, 3-chloro-2-(trifluoromethyl)pyridin-4-yl, 2,4-difluoro-3-methoxyphenyl, 3-chloro-2-(trifluoromethyl)phenyl, 3-fluoro-2-methylphenyl, 3-(difluoromethyl)-2-fluorophenyl, 4-(difluoromethyl)-2-fluorophenyl, 2-fluoro-3-methoxyphenyl, quinolinyl, 5-chloro-4-methylpyridin-3-yl, 2-chlorophenyl, 2-fluoro-4-methoxyphenyl, 1-methyl-1H-benzo[d]imidazol-6-yl, 8-fluoroquinolin-5-yl, 3-cyclopropyl-2-fluorophenyl, 8-chloroquinolin-5-yl, 2-(difluoromethyl)-3-fluorophenyl, 2-(difluoromethyl)phenyl, 8-(difluoromethoxy)quinolin-5-yl, 5-(difluoromethyl)-2-fluorophenyl, 2,6-difluorophenyl, 3-fluorobenzonitrile, 2,4,5-trifluorophenyl, 4-chloro-2-fluorophenyl, 3-fluoro-2-(1H-pyrazol-1-yl)phenyl, 2-fluoro-3-(1H-pyrazol-1-yl)phenyl, 2-chloro-3-(2-(difluoromethyl)-1H-imidazol-1-yl)phenyl, 7-fluoro-2-methyl-2H-indazol-6-yl, 4-chloro-2-methyl-2H-indazol-5-yl.

Preferably, in the compound of formula (I) Cy-X₄-R₄ corresponds to the general formula (A) wherein R₅ and R₆ are H and R₄ is selected from phenyl, pyridine, pyrimidine, pyrazine, quinoline, each of said ring being optionally substituted with one or more substituents independently selected from C(=O)CH₃, C(=O)OCH₃, OH, OC₁₋₃alkyl, halogen, NH₂, CF₃, CHF₂, C₁₋₃alkyl, C(=O)NH₂, C(=O)NHC₁₋₃alkyl, C(=O)N(C₁₋₃alkyl)₂, CN, C₃₋₅cycloalkyl.

Preferably, in the compound of formula (I) Cy-X₄-R₄ corresponds to the general formula (A) wherein R₂, R₃, R₅ and R₆ are H and R₄ is selected from phenyl, pyridine, pyrimidine, pyrazine, quinoline, each of said ring being optionally substituted with one or more substituents independently selected from C(=O)CH₃, C(=O)OCH₃, OH, OC₁₋₃alkyl, halogen, NH₂, CF₃, CHF₂, C₁₋₃alkyl, C(=O)NH₂, C(=O)NHC₁₋₃alkyl, C(=O)N(C₁₋₃alkyl)₂, CN, C₃₋₅cycloalkyl.

Preferably, in the compound of formula (I) Cy-X₄-R₄ corresponds to the general formula (A) wherein R₂, R₃, R₅ and R₆ are H and R₄ is quinoline optionally substituted with one or more substituents independently selected from OH, OC₁₋₃alkyl, halogen, NH₂, CF₃, CHF₂, C₁₋₃alkyl, C(=O)NH₂, C(=O)NHC₁₋₃alkyl, C(=O)N(C₁₋₃alkyl)₂, CN, C₃₋₅cycloalkyl.

It is a further object of the invention a compound of formula (I) as defined above, selected from the group consisting of:
- (S)-6-(4'-amino-3'-fluoro-4'H,6'H-spiro[piperidine-4,5'-pyrrolo[1,2-b]pyrazol]-1-yl)-3-(2,3- dichlorophenyl)-2-methylpyrimidin-4(3H)-one;
- (S)-1-(5-(2,3-dichlorophenoxy)pyrazin-2-yl)-4'H,6'H-spiro[piperidine-4,5'-pyrrolo[1,2-b]pyrazol]-4'-amine;
- (S)-6-(4'-amino-4'H,6'H-spiro[piperidine-4,5'-pyrrolo[1,2-b]pyrazol]-1-yl)-3-(2,3- dichlorophenyl)-2,5-dimethylpyrimidin-4(3H)-one;
- (S)-1-(5-((3-fluoro-2-methylpyridin-4-yl)thio)-6-methylpyrazin-2-yl)-4'H,6'H-spiro[piperidine-4,5'-pyrrolo[1,2-b]pyrazol]-4'-amine;
- (S)-2-(4'-amino-4'H,6'H-spiro[piperidine-4,5'-pyrrolo[1,2-b]pyrazol]-1-yl)-5-(2,3- difluorophenyl)-3-methylpyrimidin-4(3H)-one;
- (S)-1-(5-((2,3-dichlorophenyl)thio)-6-methylpyrazin-2-yl)-4'H,6'H-spiro[piperidine-4,5'-pyrrolo[1,2-b]pyrazol]-4'-amine;
- (S)-1-(5-(4-fluorophenoxy)pyrazin-2-yl)-4'H,6'H-spiro[piperidine-4,5'-pyrrolo[1,2-b]pyrazol]-4'-amine;
- (S)-1-(6-(2,3-difluorophenyl)pyrido[2,3-b]pyrazin-2-yl)-4'H,6'H-spiro[piperidine-4,5'-pyrrolo[1,2-b]pyrazol]-4'-amine;
- (S)-1-(6-(2,3-dichlorophenyl)pyrido[2,3-b]pyrazin-2-yl)-4'H,6'H-spiro[piperidine-4,5'-pyrrolo[1,2-b]pyrazol]-4'-amine;
- (S)-1-(5-((2-chloro-3-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-6-(4'-amino-4'H,6'H-spiro[piperidine-4,5'-pyrrolo[1,2-b]pyrazol]-1-yl)-3-(2,3 - dichlorophenyl)-1-methylpyridin-2(1H)-one;
- (S)-1-(3-(2-fluoro-3-methylphenyl)imidazo[1,5-a]pyrazin-8-yl)-4'H,6'H-spiro[piperidine-4,5'-pyrrolo[1,2-b]pyrazol]-4'-amine;
- (S)-1-(5-((4-fluorophenyl)thio)pyrazin-2-yl)-4'H,6'H-spiro[piperidine-4,5'-pyrrolo[1,2-b]pyrazol]-4'-amine;
- (S)-1-(3-(2-chloro-3-fluorophenyl)imidazo[1,5-a]pyrazin-8-yl)-4'H,6'H-spiro[piperidine-4,5'-pyrrolo[1,2-b]pyrazol]-4'-amine;
- (S)-1-(3-(3-chloro-2-methylphenyl)imidazo[1,5-a]pyrazin-8-yl)-4'H,6'H-spiro[piperidine-4,5'-pyrrolo[1,2-b]pyrazol]-4'-amine;
- (S)-1-(3-(2-chloro-6-fluoro-3-methylphenyl)imidazo[1,5-a]pyrazin-8-yl)-4'H,6'H-spiro[piperidine-4,5'-pyrrolo[1,2-b]pyrazol]-4'-amine;
- (S)-1-(5-((2,3-dichlorophenyl)thio)-3-methylpyrazin-2-yl)-4'H,6'H-spiro[piperidine-4,5'-pyrrolo[1,2-b]pyrazol]-4'-amine;
- (S)-1-(5-((4-(trifluoromethoxy)phenyl)thio)pyrazin-2-yl)-4'H,6'H-spiro[piperidine-4,5'-pyrrolo[1,2-b]pyrazol]-4'-amine;
- (S)-1-(5-((4-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((3-chlorophenyl)thio)pyrazin-2-yl)-4'H,6'H-spiro[piperidine-4,5'-pyrrolo[1,2-b]pyrazol]-4'-amine;
- (S)-1-(5-((2-fluorophenyl)thio)pyrazin-2-yl)-4'H,6'H-spiro[piperidine-4,5'-pyrrolo[1,2-b]pyrazol]-4'-amine;
- (S)-1-(5-((2,4-dichlorophenyl)thio)pyrazin-2-yl)-4'H,6'H-spiro[piperidine-4,5'-pyrrolo[1,2-b]pyrazol]-4'-amine;
- (S)-1-(5-((3,4-dichlorophenyl)thio)pyrazin-2-yl)-4'H,6'H-spiro[piperidine-4,5'-pyrrolo[1,2-b]pyrazol]-4'-amine;
- (S)-1-(5-(phenylthio)pyrazin-2-yl)-4'H,6'H-spiro[piperidine-4,5'-pyrrolo[1,2-b]pyrazol]-4'-amine;
- (S)-1-(5-(pyridin-4-ylthio)pyrazin-2-yl)-4'H,6'H-spiro[piperidine-4,5'-pyrrolo[1,2-b]pyrazol]-4'-amine;
- (S)-1-(5-(o-tolylthio)pyrazin-2-yl)-4'H,6'H-spiro[piperidine-4,5'-pyrrolo[1,2-b]pyrazol]-4'-amine;
- (S)-1-(5-((2-isopropylphenyl)thio)pyrazin-2-yl)-4'H,6'H-spiro[piperidine-4,5'-pyrrolo[1,2-b]pyrazol]-4'-amine;
- (S)-1-(5-((2,3-dichlorophenyl)thio)pyrazin-2-yl)-1'H,3'H-spiro[piperidine-4,2'-pyrrolizin]-1'-amine;
- (S)-1-(5-((2,3-dichlorophenyl)thio)pyrazin-2-yl)-7'-fluoro-1'H,3'H-spiro[piperidine-4,2'-pyrrolizin]-1'-amine;
- (S)-1-(5-((2-chloro-3-fluorophenyl)thio)pyrazin-2-yl)-4'H,6'H-spiro[piperidine-4,5'-pyrrolo[1,2-b]pyrazol]-4'-amine;
- (S)-1-(5-(2,3-dichlorophenyl)pyrazin-2-yl)-4'H,6'H-spiro[piperidine-4,5'-pyrrolo[1,2-b]pyrazol]-4'-amine;
- (S)-1-(5-((2,4-difluorophenyl)thio)pyrazin-2-yl)-4'H,6'H-spiro[piperidine-4,5'-pyrrolo[1,2-b]pyrazol]-4'-amine;
- (S)-1-(5-((3-chloro-2-methylphenyl)thio)pyrazin-2-yl)-4'H,6'H-spiro[piperidine-4,5'-pyrrolo[1,2-b]pyrazol]-4'-amine;
- (S)-1-(5-((2-chloro-4-fluorophenyl)thio)pyrazin-2-yl)-4'H,6'H-spiro[piperidine-4,5'-pyrrolo[1,2-b]pyrazol]-4'-amine;
- (S)-1-(5-((2-chloro-3-methylphenyl)thio)pyrazin-2-yl)-4'H,6'H-spiro[piperidine-4,5'-pyrrolo[1,2-b]pyrazol]-4'-amine;
- (S)-1-(5-((2-fluoro-3-methylphenyl)thio)pyrazin-2-yl)-4'H,6'H-spiro[piperidine-4,5'-pyrrolo[1,2-b]pyrazol]-4'-amine;
- (S)-1-(5-((8-methoxyquinolin-4-yl)thio)pyrazin-2-yl)-4'H,6'H-spiro[piperidine-4,5'-pyrrolo[1,2-b]pyrazol]-4'-amine;
- (S)-1-(5-((2,3-dihydrobenzofuran-4-yl)thio)pyrazin-2-yl)-4'H,6'H-spiro[piperidine-4,5'-pyrrolo[1,2-b]pyrazol]-4'-amine;
- (S)-1-(5-(quinolin-4-ylthio)pyrazin-2-yl)-4'H,6'H-spiro[piperidine-4,5'-pyrrolo[1,2-b]pyrazol]-4'-amine;
- (S)-1-(5-((4-chloro-2-methylphenyl)thio)pyrazin-2-yl)-4'H,6'H-spiro[piperidine-4,5'-pyrrolo[1,2-b]pyrazol]-4'-amine;
- (S)-1-(5-((2-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((2,3-dihydrobenzofuran-5-yl)thio)pyrazin-2-yl)-4'H,6'H-spiro[piperidine-4,5'-pyrrolo[1,2-b]pyrazol]-4'-amine;
- (S)-3-((5-(4'-amino-4'H,6'H-spiro[piperidine-4,5'-pyrrolo[1,2-b]pyrazol]-1-yl)pyrazin-2-yl)thio)-2-(trifluoromethyl)benzonitrile;
- (S)-1-(5-((2-chloro-3-(1H-imidazol-1-yl)phenyl)thio)pyrazin-2-yl)-4'H,6'H-spiro[piperidine-4,5'-pyrrolo[1,2-b]pyrazol]-4'-amine;
- (S)-1-(5-((2,4-difluoro-3-methylphenyl)thio)pyrazin-2-yl)-4'H,6'H-spiro[piperidine-4,5'-pyrrolo[1,2-b]pyrazol]-4'-amine;
- (S)-1-(5-((2,3-dihydro-1H-inden-4-yl)thio)pyrazin-2-yl)-4'H,6'H-spiro[piperidine-4,5'-pyrrolo[1,2-b]pyrazol]-4'-amine;
- (S)-1-(5-((2,3-dimethylphenyl)thio)pyrazin-2-yl)-4'H,6'H-spiro[piperidine-4,5'-pyrrolo[1,2-b]pyrazol]-4'-amine;
- (S)-1-(5-((2,5-difluorophenyl)thio)pyrazin-2-yl)-4'H,6'H-spiro[piperidine-4,5'-pyrrolo[1,2-b]pyrazol]-4'-amine;
- (S)-1-(5-((2,3,4-trifluorophenyl)thio)pyrazin-2-yl)-4'H,6'H-spiro[piperidine-4,5'-pyrrolo[1,2-b]pyrazol]-4'-amine;
- (S)-1-(5-((3,5 difluorophenyl)thio)pyrazin-2-yl)-4'H,6'H-spiro[piperidine-4,5'-pyrrolo[1,2-b]pyrazol]-4'-amine;
- (S)-1-(5-((3-fluorophenyl)thio)pyrazin-2-yl)-4'H,6'H-spiro[piperidine-4,5'-pyrrolo[1,2-b]pyrazol]-4'-amine;
- (S)-1-(5-((1,3-dihydroisobenzofuran-4-yl)thio)pyrazin-2-yl)-4'H,6'H-spiro[piperidine-4,5'-pyrrolo[1,2-b]pyrazol]-4'-amine;
- (S)-1-(5-((2,3-difluorophenyl)thio)pyrazin-2-yl)-4'H,6'H-spiro[piperidine-4,5'-pyrrolo[1,2-b]pyrazol]-4'-amine;
- (S)-1-(5-((3-chloro-2-(trifluoromethyl)pyridin-4-yl)thio)pyrazin-2-yl)-4'H,6'H-spiro[piperidine-4,5'-pyrrolo[1,2-b]pyrazol]-4'-amine;
- (S)-1-(5-((2,4-difluoro-3-methoxyphenyl)thio)pyrazin-2-yl)-4'H,6'H-spiro[piperidine-4,5'-pyrrolo[1,2-b]pyrazol]-4'-amine;
- (S)-1-(5-((3-chloro-2-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((3-fluoro-2-methylphenyl)thio)pyrazin-2-yl)-4'H,6'H-spiro[piperidine-4,5'-pyrrolo[1,2-b]pyrazol]-4'-amine;
- (S)-1-(5-((3-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((4-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((2-fluoro-3-methoxyphenyl)thio)pyrazin-2-yl)-4'H,6'H-spiro[piperidine-4,5'-pyrrolo[1,2-b]pyrazol]-4'-amine;
- (S)-1-(5-(quinolin-5-ylthio)pyrazin-2-yl)-4'H,6'H-spiro[piperidine-4,5'-pyrrolo[1,2-b]pyrazol]-4'-amine;
- (S)-1-(5-((5-chloro-4-methylpyridin-3-yl)thio)pyrazin-2-yl)-4'H,6'H-spiro[piperidine-4,5'-pyrrolo[1,2-b]pyrazol]-4'-amine;
- (S)-1-(5-((2-chlorophenyl)thio)pyrazin-2-yl)-4'H,6'H-spiro[piperidine-4,5'-pyrrolo[1,2-b]pyrazol]-4'-amine;
- (S)-1-(5-((2-fluoro-4-methoxyphenyl)thio)pyrazin-2-yl)-4'H,6'H-spiro[piperidine-4,5'-pyrrolo[1,2-b]pyrazol]-4'-amine;
- (S)-1-(5-((1-methyl-1H-benzo[d]imidazol-6-yl)thio)pyrazin-2-yl)-4'H,6'H-spiro[piperidine-4,5'-pyrrolo[1,2-b]pyrazol]-4'-amine;
- (S)-5-((5-(4'-amino-4'H,6'H-spiro[piperidine-4,5'-pyrrolo[1,2-b]pyrazol]-1-yl)pyrazin-2-yl)thio)quinoline-8-carbonitrile;
- (S)-1-(5-((8-(trifluoromethyl)quinolin-5-yl)thio)pyrazin-2-yl)-4'H,6'H-spiro[piperidine-4,5'-pyrrolo[1,2-b]pyrazol]-4'-amine;
- (S)-1-(5-((8-fluoroquinolin-5-yl)thio)pyrazin-2-yl)-4'H,6'H-spiro[piperidine-4,5'-pyrrolo[1,2-b]pyrazol]-4'-amine;
- (S)-1-(5-((3-cyclopropyl-2-fluorophenyl)thio)pyrazin-2-yl)-4'H,6'H-spiro[piperidine-4,5'-pyrrolo[1,2-b]pyrazol]-4'-amine;
- (S)-1-(5-((8-chloroquinolin-5-yl)thio)pyrazin-2-yl)-4'H,6'H-spiro[piperidine-4,5'-pyrrolo[1,2-b]pyrazol]-4'-amine;
- (S)-1-(5-((2-(difluoromethyl)-3-fluorophenyl)thio)pyrazin-2-yl)-4'H,6'H-spiro[piperidine-4,5'-pyrrolo[1,2-b]pyrazol]-4'-amine;
- (S)-1-(5-((2-(difluoromethyl)phenyl)thio)pyrazin-2-yl)-4'H,6'H-spiro[piperidine-4,5'-pyrrolo[1,2-b]pyrazol]-4'-amine;
- (S)-1-(5-((8-(difluoromethoxy)quinolin-5-yl)thio)pyrazin-2-yl)-4'H,6'H-spiro[piperidine-4,5'-pyrrolo[1,2-b]pyrazol]-4'-amine;
- (S)-1-(5-((5-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((2,6-difluorophenyl)thio)pyrazin-2-yl)-4'H,6'H-spiro[piperidine-4,5'-pyrrolo[1,2-b]pyrazol]-4'-amine;
- (S)-4-((5-(4'-amino-4'H,6'H-spiro[piperidine-4,5'-pyrrolo[1,2-b]pyrazol]-1-yl)pyrazin-2-yl)thio)-3 -fluorobenzonitrile;
- (S)-1-(5-((2,4,5-trifluorophenyl)thio)pyrazin-2-yl)-4'H,6'H-spiro[piperidine-4,5'-pyrrolo[1,2-b]pyrazol]-4'-amine;
- (S)-1-(5-(2-fluorophenoxy)pyrazin-2-yl)-4'H,6'H-spiro[piperidine-4,5'-pyrrolo[1,2-b]pyrazol]-4'-amine;
- (S)-1-(5-(4-chloro-2-fluorophenoxy)pyrazin-2-yl)-4'H,6'H-spiro[piperidine-4,5'-pyrrolo[1,2-b]pyrazol]-4'-amine;
- (S)-1-(5-((3-fluoro-2-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-1-(5-((2-fluoro-3-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-1 -(5-((2-chloro-3-(2-(difluoromethyl)- 1H-imidazol- 1 -yl)phenyl)thio)pyrazin-2-yl)-4'H,6'H-spiro[piperidine-4,5'-pyrrolo[1,2-b]pyrazol]-4'-amine;
- (S)-1-(5-((7-fluoro-2-methyl-2H-mdazol-6-yl)thio)pyrazm-2-yl)-4'H,6'H-spiro[piperidine-4,5'-pyrrolo[1,2-b]pyrazol]-4'-amine;
- (S)-1-(5-((4-chloro-2-methyl-2H-indazol-5-yl)thio)pyrazin-2-yl)-4'H,6'H-spiro[piperidine-4,5'-pyrrolo[1,2-b]pyrazol]-4'-amine;
or a pharmaceutically acceptable salt, solvate, or stereoisomer thereof.

It is also within the scope of the present invention a compound of formula (I^{∗}): 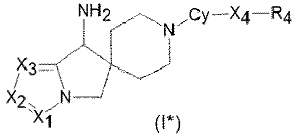 wherein X₁, X₂, X₃, X₄, Cy and R₄ are as defined above.

Preferably the compound has the following stereochemistry:

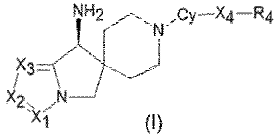

Compounds of the invention may be used in the form of prodrugs. A prodrug may be a pharmacologically inactive derivative of a biologically active substance (the "parent drug" or "parent molecule", i.e. the compound of the invention) that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the invention. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods and the preparations described in the Descriptions and in the Examples hereafter using appropriate isotopic variations of suitable reagents.

The present invention includes within its scope solvates of the compounds of Formula (I) or of the relative salts, for example, hydrates, alcoholates and the like.

The compounds disclosed herein may exist in different isomeric forms, all of which are encompassed by the present invention. In particular, any reference to the compound of the present invention is intended to include all its possible resonance forms.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers and atropoisomers, all such stereoisomers being included in the present invention. The present invention includes racemic mixtures, relative and absolute stereoisomers, and mixtures of relative and absolute stereoisomers.

Atropoisomers are stereoisomers arising because of hindered rotation about a single bond: compounds 1, and 3 are specific examples of atropoisomers according to the invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. For instance, enantiomers may be separated from each other by the selective crystallization of their diastereomeric salts or by chromatographic techniques using chiral stationary phases. Pure stereoisomeric forms may also be derived from the corresponding pure stereoisomeric forms of the appropriate starting materials, provided that the reaction occurs in stereospecific way. The term "enantiomerically pure" shall be interpreted in a similar way, having regard to the enantiomeric ratio.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure result. The phrase "optionally substituted" should be taken to be equivalent to the phrase "unsubstituted or substituted with one or more substituents" and in such cases the preferred embodiment will have from zero to three substituents. More particularly, there are zero to two substituents.

The expression "one or more substituents" refers in particular to 1, 2, 3, 4 or more substituents, in particular to 1, 2, 3 or 4 substituents, more in particular 1, 2 or 3 substituents.

As used herein "X₄ is a bond" indicates that, in the general Formula (I), Cy is directly linked via a single bond to R₄.

As used herein, "alkyl" is intended to include both branched and straight-chain saturated aliphatic hydrocarbon groups having the specified number of carbon atoms. For example, "C₁₋₆alkyl" is defined to include groups having 1, 2, 3, 4, 5 or 6 carbons in a linear or branched arrangement and specifically includes methyl, ethyl, *n*-propyl, *i*-propyl, *n*-butyl, *t*-butyl, *i*-butyl, pentyl, hexyl, and so on. Preferably, "C₁₋₆alkyl" refers to "C₁₋₄alkyl" or "C₁₋₃alkyl". "C₁₋₄alkyl" is defined to include groups having 1, 2, 3 or 4 carbons in a linear or branched arrangement. For example, "C₁₋₄ alkyl" specifically includes methyl, ethyl, *n*-propyl, *i*-propyl, *n*-butyl, *t*-butyl, *i*-butyl, and so on. "C₁₋₃alkyl" is defined to include groups having 1, 2, or 3 carbons in a linear or branched arrangement. For example, "C₁₋₃ alkyl" specifically includes methyl, ethyl, *n*-propyl, *i*-propyl, and so on. Preferred alkyl groups are methyl, ethyl, *i*-propyl, *t*-butyl or *i*-butyl.

As used herein, "alkoxy" represents an alkyl group of indicated number of carbon atoms attached through an oxygen bridge. "Alkoxy" therefore encompasses the definitions of alkyl above. C₁₋₆ alkoxy group is preferably a linear or branched C₁₋₄alkoxy group, more preferably a C₁₋₃alkoxy group, still more preferably a C₁₋₂ alkoxy group. Examples of suitable alkoxy groups include, but are not limited to methoxy, ethoxy, *n*-propoxy, *i*-propoxy, *n*-butoxy, *s*-butoxy or *t*-butoxy. Preferred alkoxy groups include methoxy, ethoxy and *t*-butoxy.

As used herein, the terms "haloC₁₋₆alkyl", "haloC₁₋₆alkoxy" and variants thereof such as "C₁₋₆haloalkyl" mean a C₁₋₆alkyl or C₁₋₆alkoxy group in which one or more (in particular, 1 to 3) hydrogen atoms have been replaced by halogen atoms, especially fluorine or chlorine atoms. HaloC₁₋₆alkoxy group is preferably a linear or branched haloC₁₋₄alkoxy group, more preferably a haloC₁₋₃alkoxy group, still more preferably a haloC₁₋₂alkoxy group, for example OCF₃, OCHF₂, OCH₂F, OCH₂CH₂F, OCH₂CHF₂ or OCH₂CF₃, and most especially OCF₃ or OCHF₂. HaloC₁₋₆alkyl group is preferably a linear or branched haloC₁₋₃alkyl group, more preferably a haloC₁₋₂alkyl group for example, CF₃, CHF₂, CH₂F, CH₂CH₂F, CH₂CHF₂, CH₂CF₃ or CH(CH₃)CF₃, and most especially CF₃, CHF₂ or CH(CH₃)CF₃.

As used herein, the term "aryl" or "aromatic ring" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Preferably, the ring component of aryl or heteroaryl groups comprises 5 or 6 members (i.e. atoms). Still preferably, aryl or heteroaryl groups are polycyclic aromatic rings. Illustrative examples of aryl groups are optionally substituted phenyls. Illustrative examples of heteroaryl groups according to the invention include optionally substituted thiophene, oxazole, thiazole, thiadiazole, imidazole, pyrazole, pyrimidine, pyrazine, pyridine and pyridine N-oxide. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzoxadiazolyl, benzoisoxazolyl, benzoisothiazolyl, indolyl, indolinyl, indolizinyl, indazolyl, isobenzofuranyl, isoindolyl, isoindolinyl, isoquinolyl, quinazolinyl, quinolyl, quinoxalinyl, quinolizinyl, naphtyl, naphthyridinyl and phthalazinyl. Other examples of polycyclic heteroaromatic rings according to the invention are 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine, 1,5-naphthyridine, imidazo[1,2-a]pyridine. A preferred aryl according to the present invention is phenyl. A preferred heteroaryl according to the present invention is pyridyl.

The expressions "optionally substituted aryl", "optionally substituted heteroaryl", "optionally substituted aryloxy", "optionally substituted heteroaryl-C₁₋₆alkyl", "optionally substituted heteroaryl-C₁₋₆alkoxy" generically refer to aryl, heteroaryl or aryloxy groups wherein the aromatic or heteroaromatic ring may be substituted with one or more substituents. Examples of said substituents include alkyl, alkoxy, amino, trifluoromethyl, aryl, heteroaryl, hydroxyl, carboxyalkyl and the like.

Aryl or heteroaryl rings can also have a partially unsaturated structure and can thus be derived from the partially hydrogenated analogues of the before-listed aryl or heteroaryl groups but also from an aryl or heteroaryl ring fused with a cycloalkyl or heterocycloalkyl ring. Said rings might also contain a group selected from SO, SO₂ and C=O. Examples of said partially unsaturated aryl or heteroaryl derivatives include 2,3-dihydro-1H-indene, 2,3-dihydro-1H-inden-1-one, 2,3-dihydroisoindol-1-one, indoline, 1,2,3,4-tetrahydroquinoline, 1,2,3,4-tetrahydroisoquinoline, isoindoline, 1-methyl-indol-2-one, dihydroquinazoline, dihydroquinoxaline, 2,3-dihydrobenzofuran, benzo[d][1,3]dioxole, 1,3-dihydroisobenzofuran, 3,4-dihydro-2H-benzo[b][1,4]oxazine, 2,3,4,5-tetrahydrobenzo[f][1,4]oxazepine, quinazolin-4(3H)-one, 4,5,6,7-tetrahydro-1H-indazole, 4,5,6,7-tetrahydropyrazolo[1,5-a]pyrazine, 2,3,4,5-tetrahydro-1H-benzo[d]azepine, 6',7'-dihydrospiro[azetidine-3,5'-pyrrolo[1,2-a]imidazole], 2,3-dihydrobenzo[b][1,4]dioxine, benzo[d]oxazol-2(3H)-one, 2H-benzo[b][1,4]oxazin-3(4H)-one, indolin-2-one, 1,2,3,4-tetrahydro-1,5-naphthyridine, 3',4'-dihydro-2'H-spiro[azetidine-3,1'-pyrrolo[1,2-a]pyrazine], 3,4-dihydroquinolin-2(1H)-one, 4-methyl-2H-benzo[b][1,4]oxazin-3(4H)-one, quinoxalin-2(1H)-one, 4H-pyrido[1,2-a]pyrimidin-4-one, (6aS)-6a,7,8,9-tetrahydro-6H-pyrido[3,2-b]pyrrolo[1,2-d][1,4 ]oxazine, 3,4-dihydro-2H-1,5-naphthyridin-1-yl, dihydrofuro[2,3-b]pyridinyl and the like.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, the expression "NH-C₁₋₆alkyl", "N(C₁₋₆alkyl)₂" refer to an amino substituent, wherein anyone of the two hydrogens is substituted by a C₁₋₆alkyl chain.

As used herein, the expression "saturated 5- or 6-membered heterocyclic ring" refer to a saturated 5 or six membered ring containing at least one heteroatom selected from S, N or O, preferably N. Examples of said saturated 5- or 6-membered heterocyclic ring are pyrrolidine, piperidine, morpholine, piperazine, tetrahydrofurane, tetrahydropyrane and the like.

Included in the instant invention is the free base of compounds of Formula (I) as well as the pharmaceutically acceptable salts and stereoisomers thereof. Some of the specific compounds exemplified herein are the protonated salts of amine compounds. Compounds containing one or more N atoms may be protonated on anyone, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of Formula (I). The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Conventional non-toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of Formula (I) and 1, 2 or 3 equivalent of an inorganic or organic acid or base. More particularly, pharmaceutically acceptable salts of this invention are the tartrate, trifluoroacetate or the chloride salts.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

Preferably, compounds of the present invention, including salts, stereoisomers and solvates thereof, are SHP2 inhibitors, meaning that for example they can inhibit the activity or function of SHP2. Then, the present invention relates to compounds for use as inhibitors of at least one SHP2 function and to a method of inhibiting at least one SHP2 function comprising the step of contacting SHP2 with a compound as described herein.

"SHP2" means "Src Homology-2-phosphatase" and is also known as SH-PTP2, SH-PTP3, Syp, PTPID, PTP2C, SAP-2 or PTPN11.

The functions of SHP2 are varied as SHP2 is involved in multiple signaling processes, such as RAS-ERK, JAK-STAT, PI3K-AKT, NF-κB, and mTOR pathways. SHP2 regulates cancer cell survival and proliferation primarily by activating the RAS-ERK signaling pathway (T. Matozaki, Y. Murata, Y. Saito, H. Okazawa, H. Ohnishi, Cancer Sci, 100 (2009), pp. 1786-1793). In the RAS-ERK pathway, SHP2 acts as a positive regulator at upstream to promote RAS-RAF-ERK kinase cascade signaling transduction. Therefore, SHP2 inhibition leads to dephosphorylation of ERK and suppression of the pro-oncogenic function of RAS-RAF-ERK pathway, resulting in cell growth inhibition and apoptosis induction in cancer cells. Recently, Chen et al. (Y.N. Chen, M.J. LaMarche, H.M. Chan, P. Fekkes, J. Garcia-Fortanet, M.G. Acker et al., Nature, 535 (2016), pp. 148-152) found that cancer cell lines sensitive to SHP2 depletion were also sensitive to EGFR depletion, which validated reports that RTK-driven cancer cells depend on SHP2 for survival. Furthermore, recent studies have shown that SHP2 is required for the growth of mutant KRAS-driven cancers while wild-type KRAS-amplified gastroesophageal cancer can be controlled through combined SHP2 and MEK inhibition (S. Mainardi, A. Mulero-Sanchez, A. Prahallad, G. Germano, A. Bosma, P. Krimpenfort, et al. Nat Med, 24 (2018), pp. 961-9; D.A. Ruess, G.J. Heynen, K.J. Ciecielski, J. Ai, A. Berninger, D. Kabacaoglu, et al. Nat Med, 24 (2018), pp. 954-960; G.S. Wong, J. Zhou, J.B. Liu, Z. Wu, X. Xu, T. Li, et al. Nat Med, 24 (2018), pp. 968-977). As a downstream target of several receptors, SHP2 is also involved in signaling in T-cells (M. Tajan, A. de Rocca Serra, P. Valet, T. Edouard, A. Yart, Eur J Med Genet, 58 (2015), pp. 509-525; R.J. Salmond, D.R. Alexander, Trends Immunol, 27 (2006), pp. 154-160). It is a downstream molecule in the PD-1 signaling pathway which not only suppresses T-cell activation but also causes T-cell anergy. SHP2-deficiency in T-cells triggered an anti-tumor immune response against colitis-associated cancer in mice (W. Liu, W. Guo, L. Shen, Z. Chen, Q. Luo, X. Luo, et al. Oncotarget, 8 (2017), pp. 7586-7597). Therefore, targeting SHP2 may restore or even enhance T-cell functions.

SHP2 inhibition may be assessed or measured by: the cell phenotype (as for example the phenotypes of proliferation and resistance to EGFR and c-MET co-inhibition, the mesenchymal phenotype in BTBC cells), cell proliferation, activity of SHP2, change in biochemical output produced by active SHP2, expression of SHP2, or binding of SHP2 with a natural binding partner may be monitored as a measure of SHP2 inhibition. In particular, inhibition of SHP2 activity or function can be measured by the IC₅₀ (concentration of inhibitor which reduces the activity of SHP2 to half-maximal level), as described in the assays hereinbelow or in the biochemical assays for SHP2 inhibition reported for example by Chen et al., Nature (535) 2016 or by Bagdanoffet al., J. Med. Chem. 2019, 62, 1781-1792. Preferably, compounds of the invention exhibit an IC₅₀ towards SHP2 lower than or equal to 10 µM. Preferred compounds exhibit an enzymatic IC₅₀ towards SHP2, as defined hereinbelow, lower than or equal to 3 µM (preferably lower than or equal to 0.5 µM or between 0.5 µM and 3 µM) and/or inhibition of SHP2 in cell-based assays, as defined hereinbelow, with IC₅₀ lower than or equal to 5 µM (preferably lower than or equal to 1 µM or between 1 µM and 5 µM). Then, the compounds of the present invention, including salts, tautomers, stereoisomers and solvates thereof, may be for use in a method of inhibiting SHP2 activity. In other words, they may be for use in the prevention and/or treatment of any condition that would be ameliorated by SHP2 inhibition. In a preferred embodiment, the compound or a pharmaceutically acceptable salt, solvate, or stereoisomer thereof as defined above is for use in inhibiting SHP2 activity. Inhibition of SHP2 activity may be measured with respect to a proper control, such as a subject affected by a disease or disorder mediated by the activity of SHP2 or a subject throughout the course of a therapy for a disease or disorder mediated by the activity of SHP2. Preferably, compounds of the invention inhibit SHP2 activity by at least 10%, 20%, 30%, 40%, 50%, 60%, 70%, 80%, 90% in respect to a proper control. More preferably, compounds of the invention inhibit SHP2 activity by approximately 10%, 20%, 30%, 40%, 50%, 60%, 70%, 80%, 90% in respect to a proper control.

Yet more preferably, compounds of the invention inhibit SHP2 activity by more than 90%, for instance by approximately 92%, 94%, 95%, 98%, 99% or 100% in respect to a proper control.

Therefore, the compounds of the invention can be used for the treatment of diseases and for carrying out biological assays, cellular assays, biochemical assays or the like.

It is an object of the invention a compound or a pharmaceutically acceptable salt, solvate, or stereoisomer thereof as defined above for medical use.

Preferably, the compound or the pharmaceutically acceptable salt, solvate, or stereoisomer thereof as defined above is for use in inhibiting SHP2 activity. Inhibition of SHP2 activity further leads to dephosphorylation of ERK and suppression of the pro-oncogenic function of RAS-RAF-ERK pathway. Then, inhibition of SHP2 activity may be measured by ERK dephosphorylation, wherein ERK phosphorylation may be evaluated by any method known in the art, for instance as described in the Examples below. Dephosphorylation of ERK may be measured with reference to any proper control. More preferably, the compound or the pharmaceutically acceptable salt, solvate, or stereoisomer thereof as defined above is for use in the treatment and/or prevention of a disease or disorder mediated by the activity of SHP2. Preferably, the disease or disorder mediated by the activity of SHP2 is selected from the group consisting of: cancer, cardiovascular disease, immunological disorder, fibrosis, an ocular disorder, systemic lupus erythematosus, diabetes, neutropenia, and combinations thereof. Yet preferably, the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably any of said cancers is a primary cancer or a cancer metastasis.

A disease or disorder mediated by the activity of SHP2 indicates a condition in a subject in which modulation, in particular inhibition, of SHP2 activity can prevent, inhibit, ameliorate, slow down or eradicate the condition and/or the symptomology thereof. Treatment of said disease or disorder might comprise administering to the subject in need thereof a therapeutically effective amount of a compound of Formula (I) according to the invention.

In diseases or disorders mediated by the activity of SHP2, mutations are often observed at the N-SH2/PTP interface (e.g. E76D/E76K), resulting in constitutively active protein and abnormal proliferation. Cancers harboring "PTPN11 mutations" include but are not limited to: N58Y; D61Y, V; E69K; A72V, T, D; E76G, Q, K (ALL); G60A; D61Y; E69V; F71K; A72V; T73I; E76G, K; R289G; G503V (AML); G60R, D61Y, V, N; Y62D; E69K; A72T, V; T73I; E76K, V, G, A, Q; E139D; G503A, R; Q506P (JMML); G60V; D61V; E69K; F71L; A72V; E76A (MDS); Y63C (CMML); Y62C; E69K; T507K (neuroblastoma); V46L; N58S; E76V (Lung cancer); R138Q (melanoma); E76G (colon cancer). The compounds of the invention can exhibit affinity at low concentrations for wild type SHP2 and can also be active against mutant forms of the protein.

Another aspect of the present invention relates to a compound of the invention, including any pharmaceutically acceptable salt, solvate or stereoisomer thereof, as defined hereinabove for use in a method of preventing/treating an SHP2-mediated disorder and/or disorders mediated by the pro-oncogenic function of RAS-RAF-ERK pathway.

Another aspect of the present invention relates to a method of preventing/treating an SHP2-mediated disorder comprising the step of administering to a patient in need thereof a therapeutically effective amount of a compound of the invention, including any pharmaceutically acceptable salt, solvate or stereoisomer thereof, as defined hereinabove. In another aspect, the present invention relates to a method of preventing/treating an SHP2-mediated disorder comprising the step of administering to a patient in need thereof a therapeutically effective amount of a chemotherapeutic agent, as further defined below, in combination with a therapeutically effective amount of a compound of the invention.

Another aspect of the present invention relates to the use of compounds of the invention, including any pharmaceutically acceptable salts, solvate or stereoisomer thereof, as defined hereinabove in preventing/treating an SHP2-mediated disorder.

In yet another aspect of the present invention, there are provided the compounds, salt, solvate, stereoisomer as defined above for use in the treatment and/or prevention of a disease or disorder selected from the group consisting of: cancer, cardiovascular disease, immunological disorder, fibrosis, an ocular disorder, systemic lupus erythematosus, diabetes, neutropenia and combinations thereof. Preferably, the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably, the cancer is a primary cancer or a cancer metastasis.

In certain embodiments the present invention relates to the aforementioned use/method, wherein said disorder is selected from Noonan Syndrome (NS) and Leopard Syndrome (LS).

In further embodiments, the present invention relates to the aforementioned use/method, wherein said SHP2-mediated disorders are those due to dysregulated cellular proliferation, including cancer. The cancer may be hormone-dependent or hormone-resistant, such as in the case of breast cancers. Preferably, the cancer is RTK-driven or KRAS-driven, such as KRAS amplified gastroesophageal cancer. In certain embodiments, the cancer is a solid tumor. In other embodiments, the cancer is a lymphoma or leukemia or a glioma. In certain embodiments, the cancer is a drug resistant phenotype of a cancer disclosed herein or known in the art. The cancer may be primary or metastatic. Tumor invasion, tumor growth, tumor metastasis, and angiogenesis may also be treated using the compositions and methods disclosed herein. Precancerous neoplasia may also be treated using the compositions and methods disclosed herein. Compounds of the invention can be used for the treatment of cancers selected from, but not limited to: Juvenile Myelomonocytic Leukemias (JMML); Acute Myeloid Leukemia (AML); Myelodysplastic Syndrome (MDS); B cell acute lymphoblastic leukemia (B-ALL); neuroblastoma; esophageal; breast cancer; lung cancer; colon cancer; gastric cancer, head and neck cancer; ovarian cancer; prostate cancer; cancers of the oral cavity and pharynx (lip, tongue, mouth, larynx, pharynx), stomach, small intestine, large intestine, colon, rectum, liver and biliary passages; pancreas, bone, connective tissue, skin, cervix, uterus, corpus endometrium, testis, bladder, kidney and other urinary tissues, including renal cell carcinoma (RCC); gastroesophageal cancer (preferably KRAS-amplified gastroesophageal cancer), cancers of the eye, brain, spinal cord, and other components of the central and peripheral nervous systems, as well as associated structures such as the meninges; thyroid and other endocrine glands, Hodgkin's disease, non-Hodgkin's lymphomas, multiple myeloma and hematopoietic malignancies including leukemias Chronic Lymphocytic Leukemia (CLL), Acute Lymphocytic Leukemia (ALL), Chronic Myelogenous Leukemia (CML), Acute Myelogenous Leukemia (AML), Chronic Myelomonocytic Leukemia (CMML), and lymphomas including lymphocytic, granulocytic and monocytic. Additional types of cancers which may be treated using the compounds and methods of the invention include, but are not limited to, adenocarcinoma, angiosarcoma, astrocytoma, acoustic neuroma, anaplastic astrocytoma, basal cell carcinoma, blastoglioma, chondrosarcoma, choriocarcinoma, chordoma, craniopharyngioma, cutaneous melanoma, cystadenocarcinoma, endotheliosarcoma, embryonal carcinoma, ependymoma, Ewing's tumor, epithelial carcinoma, fibrosarcoma, gastric cancer, genitourinary tract cancers, glioblastoma multiforme, hemangioblastoma, hepatocellular carcinoma, hepatoma, Kaposi's sarcoma, large cell carcinoma, leiomyosarcoma, leukemias, liposarcoma, lymphatic system cancer, lymphomas, lymphangiosarcoma, lymphangioendotheliosarcoma, medullary thyroid carcinoma, medulloblastoma, meningioma mesothelioma, myelomas, myxosarcoma neuroblastoma, neurofibrosarcoma, oligodendroglioma, osteogenic sarcoma, epithelial ovarian cancer, papillary carcinoma, papillary adenocarcinomas, paraganglioma, parathyroid tumours, pheochromocytoma, pinealoma, plasmacytomas, retinoblastoma, rhabdomyosarcoma, sebaceous gland carcinoma, seminoma, skin cancers, melanoma, small cell lung carcinoma, non-small cell lung carcinoma, squamous cell carcinoma, sweat gland carcinoma, synovioma, thyroid cancer, uveal melanoma, Wilm's tumor, anaplastic large-cell lymphoma, colitis associated cancer. The compounds of the present invention may be useful in the treatment of any other disease or condition related to the aberrant activity of SHP2. Thus, as a further aspect, the invention relates to a method of treatment of a patient, which is preferably a human, affected by a disorder selected from: NS; LS; JMML; AML; MDS; B-ALL; neuroblastoma; esophageal; breast cancer; lung cancer; colon cancer; gastric cancer; head and neck cancer. The invention also relates to the use of the compounds of the invention in the manufacture of a medicament for the treatment of a disorder selected from: NS; LS; JMML; AML; MDS; B-ALL; neuroblastoma; esophageal; breast cancer; lung cancer; colon cancer; gastric cancer; head and neck cancer.

In a further embodiment of the invention, the compounds of Formula (I) are SHP2 inhibitors capable of penetrating the BBB to reach their target and treating humans suffering from brain tumors and/or brain metastases. This is particularly relevant as poor survival rates of patients with tumors arising from or disseminating into the brain are attributed to a lack of blood-brain barrier (BBB) penetration of chemotherapies/targeted agents and an intrinsic tumor radio-/chemo-resistance. The BBB, while serving a critical role in brain homeostasis, also significantly impedes the penetration of most small molecule inhibitors. With growing interest in developing selective and potent inhibitors for the treatment of brain tumors and brain metastases, there is an urgent need to overcome the challenging aspect of crossing the BBB. In a preferred embodiment, this invention is directed to SHP2 inhibitors capable of penetrating the BBB to reach their target and treating humans suffering with tumors arising from or disseminating into the brain.

A compound of the present invention, including any pharmaceutically acceptable salt, tautomer, solvate, or stereoisomer thereof, may be usefully combined with any another known therapy that is useful for the prevention/treatment of a disease or disorder mediated by the activity of SHP2. Such therapy may include radiotherapy. Such therapy may also comprise the administration of another pharmacologically active compound, or of two or more other pharmacologically active compounds, particularly compound(s) active in the prevention/treatment of cancer, also referred to as "anti-cancer drug(s)" or "chemotherapy agents". For example, a compound of the invention, including any pharmaceutically acceptable salt, tautomer, solvate, or stereoisomer thereof as defined above, may be administered simultaneously, sequentially or separately in combination with any one or more other pharmacologically active compound. For simultaneous administration, the compound of the present invention and the other one or more pharmacologically active compound may be formulated in the same composition.

Classes of anti-cancer drugs that may be combined with the compounds of the invention include, but are not limited to: alkylating agents, anti-metabolites, antimitotics, checkpoint inhibitors, plant alkaloids and terpenoids, topoisomerase inhibitors, cytotoxic antibiotics, aromatase inhibitors, angiogenesis inhibitors, anti-steroids and anti-androgens, mTOR inhibitors, tyrosine kinase inhibitors, and others. Chemotherapy agents include, for example, mitotic inhibitors such as a taxane, a vinca alkaloid, paclitaxel, docetaxel, vincristine, vinblastine, vinorelbine or vinflunine, and other anticancer agents, e.g. cisplatin, 5-fluorouracil or 5-fluoro-2-4(1 H,3H)-pyrimidinedione (5FU), flutamide or gemcitabine. Such combinations may offer significant advantages, including synergistic activity, in therapy.

Alkylating agents are compounds that work by adding an alkyl group to the guanine base of the DNA molecule, preventing the strands of the double helix from linking as they should thus causing breakage of the DNA strands and affecting the ability of the cancer cells to multiply. Antimetabolites are drugs that interfere with one or more enzymes or their reactions that are necessary for DNA synthesis. An antimitotic agent is a type of drug that blocks cell growth by stopping mitosis. Checkpoint inhibitors are a type of immunotherapy which block proteins that stop the immune system from attacking the cancer cells. Topoisomerase inhibitors are chemical compounds that block the action of topoisomerase (topoisomerase I and II), which is a type of enzyme that controls the changes in DNA structure by catalyzing the breaking and rejoining of the phosphodiester backbone of DNA strands during the normal cell cycle. Aromatase inhibitors are a class of drugs that work by inhibiting the action of the enzyme aromatase, which converts androgens into estrogens by a process called aromatization. Angiogenesis inhibitors are substances that inhibit the growth of new blood vessels and are used to treat cancers and other diseases that involve a proliferation of blood vessels. mTOR inhibitors are a class of drugs that inhibit the mammalian target of rapamycin (mTOR), which is a serine/threonine-specific protein kinase that belongs to the family of phosphatidylinositol-3 kinase (PI3K) related kinases (PIKKs). mTOR regulates cellular metabolism, growth, and proliferation by forming and signaling through two protein complexes, mTORC1 and mTORC2. The most established mTOR inhibitors are so-called rapalogs (rapamycin and its analogs), which have shown tumor responses in clinical trials against various tumor types.

It is thus a preferred object of the invention a compound as defined above or the pharmaceutically acceptable salt, solvate, or stereoisomer thereof, for use in combination with at least one further therapeutic agent.

In any case, the multiple therapeutic agents (at least one of which is a compound disclosed herein) may be administered in any order or even simultaneously.

Preferably, said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide (TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumor necrosis factor receptor superfamily member 9), TIM3 (T-cell immunoglobulin and mucin-domain containing-3) and/or OX40 (tumor necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (A V ASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone (HALOTESTIN); estrogens such as estradiol, diethylstilbestrol (DES), and dienestrol; progestins such as medroxyprogesterone acetate (MPA) and megestrol; testosterone; 5-fluoro-2-4(1 H,3H)-pyrimidinedione and combinations thereof.

The invention further provides pharmaceutical preparations comprising the compounds of the invention. In particular, it is a further object of the invention a pharmaceutical composition comprising the compound or the pharmaceutically acceptable salt, solvate, or stereoisomer thereof as defined above, alone or in combination with at least one further therapeutic agent, and at least one pharmaceutically acceptable excipient. Preferably, said at least one further therapeutic agent in the pharmaceutical composition is selected among those indicated above.

In a preferred embodiment, the pharmaceutical combination or the composition of the invention is for use in the treatment and/or prevention of a disease or disorder as herein defined, in particular a disease or disorder mediated by the activity of SHP2 and/or a disease or disorder selected from the group consisting of: cancer, cardiovascular disease, immunological disorder, fibrosis, an ocular disorder, systemic lupus erythematosus, diabetes, neutropenia and combinations thereof.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions of the invention may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated, or they may be coated by known techniques to mask the unpleasant taste of the drug or delay disintegration and absorption in the gastrointestinal tract and thereby provide a sustained action over a longer period. For example, a watersoluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS^{™} model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound(s) of the invention are employed. For purposes of this application, topical application shall include mouth washes and gargles.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol. The compounds of the invention may be presented in a liposome or other micro particulate or other nanoparticles designed to target the compound. Acceptable liposomes can be neutral, negatively, or positively charged, the charge being a function of the charge of the liposome components and pH of the liposome solution. Liposomes can be normally prepared using a mixture of phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms. Generally, dosage levels on the order of from about 0.01 mg/kg to about 150 mg/kg of body weight are useful in the treatment of the above indicated conditions.

As used herein, the term "composition" is intended to encompass a product comprising the specified ingredients in the specified amounts, as well as any product which results, directly or indirectly, from combination of the specified ingredients in the specified amounts.

Another object of the present invention relates to an *in vitro* method of inhibiting SHP2 with the compound of the present invention. This may be useful, for instance, to evaluate whether any given compound is an inhibitor/activator of SHP2 and therefore acts also on the ERK pathway.

A further object of the present invention concerns a kit comprising at least one pharmaceutically acceptable vial or container of other type, containing one or more doses of a compound of the invention, including any pharmaceutically acceptable salt, solvate or stereoisomer thereof, or of a pharmaceutical composition of the invention and optionally a) instructions for use thereof in mammals and/or b) an infusion bag or container containing a pharmaceutically acceptable diluent.

In certain embodiments, the compound or the composition of the invention is administered parenterally, intramuscularly, intravenously, subcutaneously, orally, pulmonary, intrathecally, topically, intranasally, or systemically.

In certain embodiments, the patient who is administered the compound or the composition of the invention is a mammal, preferably a primate, more preferably a human.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

As used herein, the term "prevention" means no disorder or disease development if none had occurred, or no further disorder or disease development if there had already been development of the disorder or disease. Also considered is the ability of one to prevent some or all of the symptoms associated with the disorder or disease. As used herein, any reference to "treatment"/"treating" includes the amelioration of at least one symptom of the disease/disorder to be treated. Such amelioration is to be evaluated in comparison to the same symptom prior to administration of the compound or composition of the invention.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The present invention will be described by means of the following non-limiting examples and biological data.

### MATERIALS AND METHODS

### Chemistry

As used herein, the following abbreviations have the following meanings. If an abbreviation is not defined, it has its generally accepted meaning.

### Abbreviations

AcOH: Acetic acid; AlCl₃: Aluminium chloride; BnBr: Benzyl bromide; BOP: Benzotriazol-1-yloxytris(dimethylamino)phosphonium hexafluorophosphate; Boc₂O: Di-tert-butyl dicarbonate; 1-BuOH: 1-Butanol; Cs₂CO₃: Cesium carbonate; CuI: Copper Iodide; DAST: *N*,*N*-diethyl-1,1,1-trifluoro-14-sulfanamine; DBU: 1,8-diazabiciclo[5.4.0]undec-7-ene; DCM: Dichloromethane; DIPEA: *N,N-*Diisopropylethylamine; DMA: Dimethylacetamide; DME: Dimethoxyethane; DMF: Dimethylformamide; DMP: Dess Martin periodinane, DMSO: Dimethylsulfoxide; ES⁺: Electrospray Positive Ionisation; Et₂O: Diethylether; EtOAc: Ethyl acetate; EtOH: Ethanol; EtONa: Sodium ethoxide; HBTU: (2-(1H-benzotriazol-1-yl)-1,1,3,3-tetramethyluronium hexafluorophosphate; HCl: hydrochloric acid; h: Hour; H₂O: Water; HPLC: High Performance Liquid Chromatography; IPA: Isopropyl alcohol; K₂CO₃: potassium carbonate; KOH: Potassium hydroxide; K₃PO₄: Potassium phosphate; LCMS: Liquid Chromatography Mass Spectrometry; LiAlH₄: Lithium aluminum hydride; LDA: Lithium diisopropylamide; MeCN: Acetonitrile; MeI: Iodomethane; MeOH: Methanol; min: Minutes; MsCl: Methanesulfonyl chloride; MW: Microwave; NaBH₄: Sodium borohydride; NaHCO₃: Sodium bicarbonate; NaH: Sodium hydride; NaNO₂: Sodium nitrite; NaOH: Sodium hydroxide; NaOMe: Sodium methoxide; Na₂SO₄: Sodium sulfate; Na₂S₂O₃: Sodium thiosulfate; NH₄HCO₂: Ammonium formate; N_{2:} Nitrogen; NMP: 1-metil-2-pirrolidone; Pd/C: Palladium on carbon; Pd₂(dba)₃: Tris(dibenzylideneacetone)dipalladium(0); Pd(dppf)Cl₂: [1,1'-Bis(diphenylphosphino)ferrocene]dichloropalladium(II); Selectfluor: (1-chloromethyl-4-fluoro-1,4-diazoniabicyclo[2.2.2]octane bis(tetrafluoroborate); POCl₃: Phosphoryl chloride; RP: Reverse Phase; RT: Retention time; rt: Room temperature; Rochelle salt solution: L(+)-Tartaric acid potassium sodium salt; TEA: Triethylamine; TFA: Trifluoroacetic acid; THF: Tetrahydrofuran; Ti(OEt)₄: Titanium ethoxide; Sat.: Saturated; Sol.: Solution; TsCl: 4-Toluenesulfonyl chloride; UPLC: Ultra High Performance Liquid Chromatography; Xantphos: 4,5-Bis(diphenylphosphino)-9,9-dimethylxanthene.

The LC-MS analyses were performed by UPLC Acquity Waters System equipped with the SQD spectrometer, single quadrupole mass detector, and a TUV detector, using column 1: ACQUITY UPLC BEH C18 (2.1×50 mm, id=1.7 µm); column 2: ACQUITY UPLC HSS T3, RP18 (2.1×50 mm, id=1.8 µm). Column temperature 40°C or 33°C. Sample temperature 25°C or 33°C. Phase A was composed by H2O (HiPerSolv Chromanorm Water VWR for HPLC-MS) + 0.1% HCOOH; Phase B by MeCN (HiPerSolv Chromanorm Acetonitrile SuperGradient VWR, suitable for UPLC/UHPLC instruments) + 0.1% HCOOH; flow rate: 0.5 mL/min; UV detection (DIODE array) 200 nm; ESI+ and ESI- detection in the 100-1000 m/z range.

Method 1: column 1, run time: 3 minutes, run gradient: 5%B to 100%B in 2.8 min + 100%B for 0.2 min, equilibration time: 0.8 min, ionization mode: ESI+.

Method 2: column 2, run time: 3 minutes, run gradient: 0%B to 45%B in 2.8 min + 100%B for 0.2 min, equilibration time: 0.8 min, ionization mode: ESI⁺.

| **Example** # | **Structure** | **IUPAC Name** | **MS [M+H]⁺** |
|---|---|---|---|
| 1 | 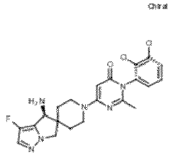 | (S)-6-(4'-amino-3'-fluoro-4'H,6'H-spiro[piperidine-4,5'-pyrrolo[1,2-b]pyrazol]-1-yl)-3-(2,3-dichlorophenyl)-2-methylpyrimidin-4(3H)-one | 463.12 |
| 2 | 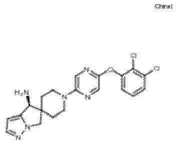 | (S)-1-(5-(2,3-dichlorophenoxy)pyrazin-2-yl)-4'H,6'H-spiro[piperidine-4,5'-pyrrolo[1,2-b]pyrazol]-4'-amine | 431.11 |
| 3 | 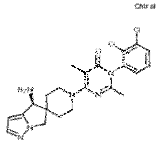 | (S)-6-(4'-amino-4'H,6'H-spiro[piperidine-4,5'-pyrrolo[1,2-b]pyrazol]-1-yl)-3-(2,3-dichlorophenyl)-2,5-dimethylpyrimidin-4(3H)-one | 459.14 |
| 4 | 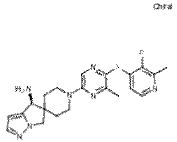 | (S)-1-(5-((3-fluoro-2-methylpyridin-4-yl)thio)-6-methylpyrazin-2-yl)-4'H,6'H-spiro[piperidine-4,5'-pyrrolo[1,2-b]pyrazol]-4'-amine | 426.18 |
| 5 | 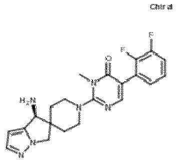 | (S)-2-(4'-amino-4'H,6'H-spiro[piperidine-4,5'-pyrrolo[1,2-b]pyrazol]-1-yl)-5-(2,3-difluorophenyl)-3-methylpyrimidin-4(3H)-one | 413.19 |
| 6 | 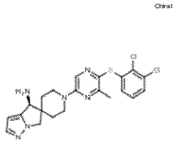 | (S)-1-(5-((2,3-dichlorophenyl)thio)-6-methylpyrazin-2-yl)-4'H,6'H-spiro[piperidine-4,5'-pyrrolo[1,2-b]pyrazol]-4'-amine | 461.1 |
| 7 | 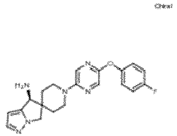 | (S)-1-(5-(4-fluorophenoxy)pyrazin-2-yl)-4'H,6'H-spiro[piperidine-4,5'-pyrrolo[1,2-b]pyrazol]-4'-amine | 381.18 |
| 8 | 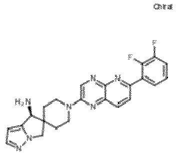 | (S)-1 -(6-(2,3 -difluorophenyl)pyrido [2,3 -b]pyrazin-2-yl)-4'H,6'H-spiro[piperidine-4,5'-pyrrolo[1,2-b]pyrazol]-4'-amine | 434.19 |
| 9 | 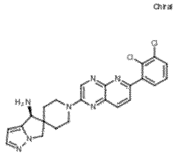 | (S)-1 -(6-(2,3 -dichlorophenyl)pyrido [2,3 -b]pyrazin-2-yl)-4'H,6'H-spiro[piperidine-4,5'-pyrrolo[1,2-b]pyrazol]-4'-amine | 466.13 |
| 10 | 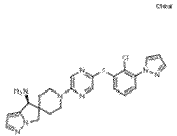 | (S)-1-(5-((2-chloro-3-(1H-pyrazol-1-yl)phenyl)thio)pyrazin-2-yl)-4'H,6'H-spiro[piperidine-4,5'-pyrrolo[1,2-b]pyrazol]-4'-amine | 479.15 |
| 11 | 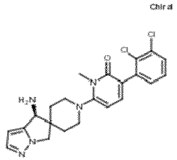 | (S)-6-(4'-amino-4'H,6'H-spiro[piperidine-4,5'-pyrrolo[1,2-b]pyrazol]-1-yl)-3-(2,3-dichlorophenyl)-1-methylpyridin-2(1H)-one | 444.13 |
| 12 | 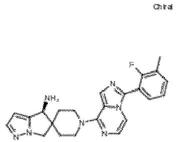 | (S)-1-(3-(2-fluoro-3-methylphenyl)imidazo[1,5-a]pyrazin-8-yl)-4'H,6'H-spiro[piperidine-4,5'-pyrrolo[1,2-b]pyrazol]-4'-amine | 418.21 |
| 13 | 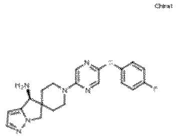 | (S)-1-(5-((4-fluorophenyl)thio)pyrazin-2-yl)-4'H,6'H-spiro[piperidine-4,5'-pyrrolo[1,2-b]pyrazol]-4'-amine | 397.16 |
| 14 | 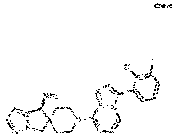 | (S)-1-(3-(2-chloro-3-fluorophenyl)imidazo[1,5-a]pyrazin-8-yl)-4'H,6'H-spiro[piperidine-4,5'-pyrrolo[1,2-b]pyrazol]-4'-amine | 438.16 |
| 15 | 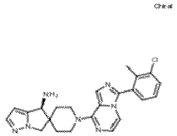 | (S)-1-(3-(3-chloro-2-methylphenyl)imidazo[1,5-a]pyrazin-8-yl)-4'H,6'H-spiro[piperidine-4,5'-pyrrolo[1,2-b]pyrazol]-4'-amine | 434.18 |
| 16 | 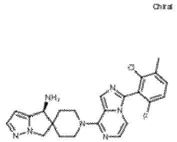 | (S)-1-(3-(2-chloro-6-fluoro-3-methylphenyl)imidazo[1,5-a]pyrazm-8-yl)-4'H,6'H-spiro[piperidine-4,5'-pyrrolo[1,2-b]pyrazol]-4'-amine | 452.17 |
| 17 | 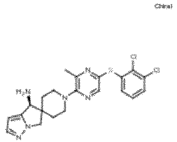 | (S)-1-(5-((2,3-dichlorophenyl)thio)-3-methylpyrazin-2-yl)-4'H,6'H-spiro[piperidine-4,5'-pyrrolo[1,2-b]pyrazol]-4'-amine | 461.11 |
| 18 | 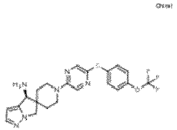 | (S)-1-(5-((4-(trifluoromethoxy)phenyl)thio)pyrazin-2-yl)-4'H,6'H-spiro[piperidine-4,5'-pyrrolo[1,2-b]pyrazol]-4'-amine | 463.15 |
| 19 | 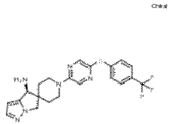 | (S)-1-(5-((4-(trifluoromethyl)phenyl)thio)pyrazin-2-yl)-4'H,6'H-spiro[piperidine-4,5'-pyrrolo[1,2-b]pyrazol]-4'-amine | 447.15 |
| 20 | 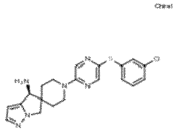 | (S)-1-(5-((3-chlorophenyl)thio)pyrazin-2-yl)-4'H,6'H-spiro[piperidine-4,5'-pyrrolo[1,2-b]pyrazol]-4'-amine | 413.13 |
| 21 | 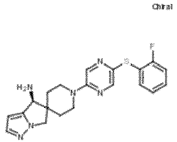 | (S)-1-(5-((2-fluorophenyl)thio)pyrazin-2-yl)-4'H,6'H-spiro[piperidine-4,5'-pyrrolo[1,2-b]pyrazol]-4'-amine | 397.16 |
| 22 | 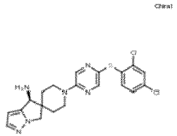 | (S)-1-(5-((2,4-dichlorophenyl)thio)pyrazin-2-yl)-4'H,6'H-spiro[piperidine-4,5'-pyrrolo[1,2-b]pyrazol]-4'-amine | 447.09 |
| 23 | 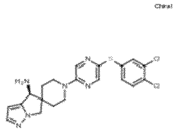 | (S)-1-(5-((3,4-dichlorophenyl)thio)pyrazin-2-yl)-4'H,6'H-spiro[piperidine-4,5'-pyrrolo[1,2-b]pyrazol]-4'-amine | 447.09 |
| 24 | 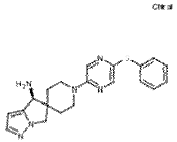 | (S)-1-(5-(phenylthio)pyrazin-2-yl)-4'H,6'H-spiro[piperidine-4,5'-pyrrolo[1,2-b]pyrazol]-4'-amine | 379.17 |
| 25 | 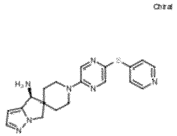 | (S)-1-(5-(pyridin-4-ylthio)pyrazin-2-yl)-4'H,6'H-spiro[piperidine-4,5'-pyrrolo[1,2-b]pyrazol]-4'-amine | 380.16 |
| 26 | 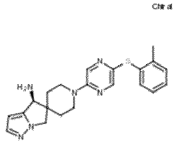 | (S)-1-(5-(o-tolylthio)pyrazin-2-yl)-4'H,6'H-spiro[piperidine-4,5'-pyrrolo[1,2-b]pyrazol]-4'-amine | 393.18 |
| 27 | 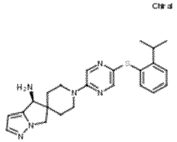 | (S)-1-(5-((2-isopropylphenyl)thio)pyrazin-2-yl)-4'H,6'H-spiro[piperidine-4,5'-pyrrolo[1,2-b]pyrazol]-4'-amine | 421.21 |
| 28 | 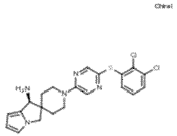 | (S)-1-(5-((2,3-dichlorophenyl)thio)pyrazin-2-yl)-1'H,3'H-spiro[piperidine-4,2'-pyrrolizin]-1'-amine | 446.32 |
| 29 | 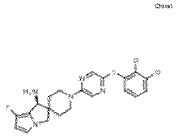 | (S)-1-(5-((2,3-dichlorophenyl)thio)pyrazin-2-yl)-7'-fluoro-1'H,3'H-spiro[piperidine-4,2'-pyrrolizin]-1'-amine | 464.15 |
| 30 | 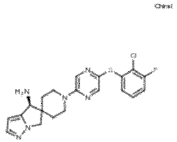 | (S)-1-(5-((2-chloro-3-fluorophenyl)thio)pyrazin-2-yl)-4'H,6'H-spiro[piperidine-4,5'-pyrrolo[1,2-b]pyrazol]-4'-amine | 431.12 |
| 31 | 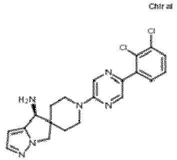 | (S)-1-(5-(2,3-dichlorophenyl)pyrazin-2-yl)-4'H,6'H-spiro[piperidine-4,5'-pyrrolo[1,2-b]pyrazol]-4'-amine | 415.12 |
| 32 | 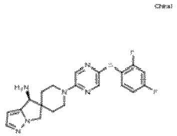 | (S)-1-(5-((2,4-difluorophenyl)thio)pyrazin-2-yl)-4'H,6'H-spiro[piperidine-4,5'-pyrrolo[1,2-b]pyrazol]-4'-amine | 415.15 |
| 33 | 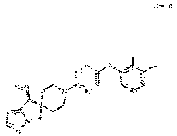 | (S)-1-(5-((3-chloro-2-methylphenyl)thio)pyrazin-2-yl)-4'H,6'H-spiro[piperidine-4,5'-pyrrolo[1,2-b]pyrazol]-4'-amine | 427.14 |
| 34 | 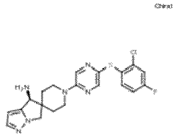 | (S)-1-(5-((2-chloro-4-fluorophenyl)thio)pyrazin-2-yl)-4'H,6'H-spiro[piperidine-4,5'-pyrrolo[1,2-b]pyrazol]-4'-amine | 431.12 |
| 35 | 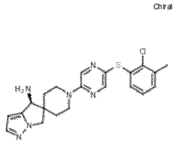 | (S)-1-(5-((2-chloro-3-methylphenyl)thio)pyrazin-2-yl)-4'H,6'H-spiro[piperidine-4,5'-pyrrolo[1,2-b]pyrazol]-4'-amine | 427.14 |
| 36 | 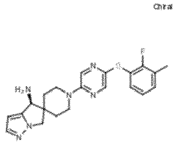 | (S)-1-(5-((2-fluoro-3-methylphenyl)thio)pyrazin-2-yl)-4'H,6'H-spiro[piperidine-4,5'-pyrrolo[1,2-b]pyrazol]-4'-amine | 411.17 |
| 37 | 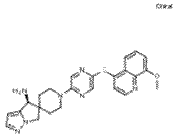 | (S)-1-(5-((8-methoxyquinolm-4-yl)thio)pyrazin-2-yl)-4'H,6'H-spiro[piperidine-4,5'-pyrrolo[1,2-b]pyrazol]-4'-amine | 460.19 |
| 38 | 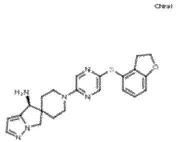 | (S)-1 -(5 -((2,3 -dihydrobenzofuran-4-yl)thio)pyrazin-2-yl)-4'H,6'H-spiro[piperidine-4,5'-pyrrolo[1,2-b]pyrazol]-4'-amine | 421.18 |
| 39 | 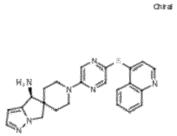 | (S)-1-(5-(qunolin-4-ylthio)pyrazin-2-yl)-4'H,6'H-spiro[piperidine-4,5'-pyrrolo[1,2-b]pyrazol]-4'-amine | 430.18 |
| 40 | 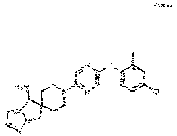 | (S)-1-(5-((4-chloro-2-methylphenyl)thio)pyrazin-2-yl)-4'H,6'H-spiro[piperidine-4,5'-pyrrolo[1,2-b]pyrazol]-4'-amine | 427.14 |
| 41 | 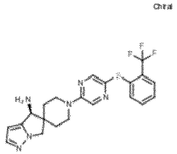 | (S)-1-(5-((2-(trifluoromethyl)phenyl)thio)pyrazin-2-yl)-4'H,6'H-spiro[piperidine-4,5'-pyrrolo[1,2-b]pyrazol]-4'-amine | 447.15 |
| 42 | 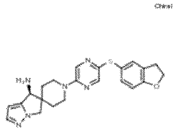 | (S)-1-(5 -((2,3 -dihydrobenzofuran-5 -yl)thio)pyrazin-2-yl)-4'H,6'H-spiro[piperldine-4,5'-pyrrolo[1,2-b]pyrazol]-4'-amine | 421.18 |
| 43 | 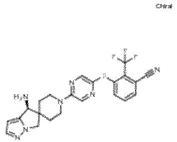 | (S)-3-((5-(4'-amino-4'H,6'H-spiro[piperidine-4,5'-pyrrolo[1,2-b]pyrazol]-1-yl)pyrazin-2-yl)thio)-2-(trifluoromethyl)benzonitrile | 472.15 |
| 44 | 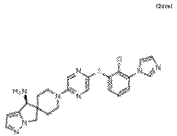 | (S)-1-(5-((2-chloro-3-(1H-imidazol-1-yl)phenyl)thio)pyrazin-2-yl)-4'H,6'H-spiro[piperidine-4,5'-pyrrolo[1,2-b]pyrazol]-4'-amine | 479.11 |
| 45 | 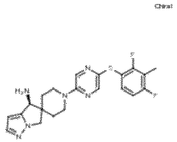 | (S)-1-(5-((2,4-difluoro-3-methylphenyl)thio)pyrazin-2-yl)-4'H,6'H-spiro[piperidine-4,5'-pyrrolo[1,2-b]pyrazol]-4'-amine | 429.16 |
| 46 | 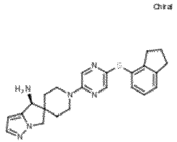 | (S)-1-(5-((2,3-dihydro-1H-inden-4-yl)thio)pyrazin-2-yl)-4'H,6'H-spiro[piperidine-4,5'-pyrrolo[1,2-b]pyrazol]-4'-amine | 419.2 |
| 47 | 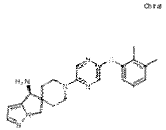 | (S)-1-(5-((2,3-dimethylphenyl)thio)pyrazin-2-yl)-4'H,6'H-spiro[piperidine-4,5'-pyrrolo[1,2-b]pyrazol]-4'-amine | 407.2 |
| 48 | 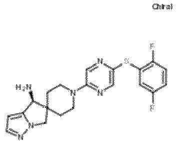 | (S)-1-(5-((2,5-difluorophenyl)thio)pyrazin-2-yl)-4'H,6'H-spiro[piperidine-4,5'-pyrrolo[1,2-b]pyrazol]-4'-amine | 415.15 |
| 49 | 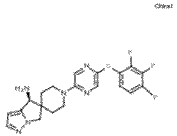 | (S)-1-(5-((2,3,4-trifluorophenyl)thio)pyrazin-2-yl)-4'H,6'H-spiro[piperidine-4,5'-pyrrolo[1,2-b]pyrazol]-4'-amine | 433.14 |
| 50 | 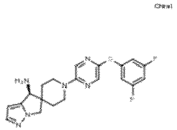 | (S)-1-(5-((3,5-difluorophenyl)thio)pyrazin-2-yl)-4'H,6'H-spiro[piperidine-4,5'-pyrrolo[1,2-b]pyrazol]-4'-amine | 415.15 |
| 51 | 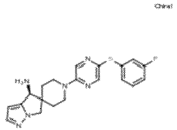 | (S)-1 -(5 -((3 -fluorophenyl)thio)pyrazin-2-yl)-4'H,6'H-spiro[piperidine-4,5'-pyrrolo[1,2-b]pyrazol]-4'-amine | 397.16 |
| 52 | 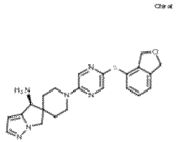 | (S)-1-(5-((1,3-dihydroisobenzofuran-4-yl)thio)pyrazin-2-yl)-4'H,6'H-spiro[piperidine-4,5'-pyrrolo[1,2-b]pyrazol]-4'-amine | 421.18 |
| 53 | 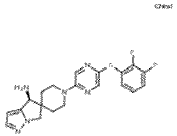 | (S)-1-(5-((2,3-difluorophenyl)thio)pyrazin-2-yl)-4'H,6'H-spiro[piperidine-4,5'-pyrrolo[1,2-b]pyrazol]-4'-amine | 415.15 |
| 54 | 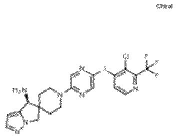 | (S)-1-(5-((3-chloro-2-(trifluoromethyl)pyridin-4-yl)thio)pyrazin-2-yl)-4'H,6'H-spiro[piperidine-4,5'-pyrrolo[1,2-b]pyrazol]-4'-amine | 482.11 |
| 55 | 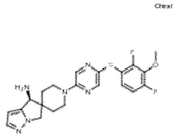 | (S)-1-(5-((2,4-difluoro-3-methoxyphenyl)thio)pyrazin-2-yl)-4'H,6'H-spiro[piperidine-4,5'-pyrrolo[1,2-b]pyrazol]-4'-amine | 445.16 |
| 56 | 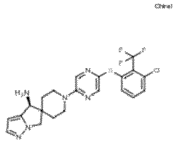 | (S)-1-(5-((3-chloro-2-(trifluoromethyl)phenyl)thio)pyrazin-2-yl)-4'H,6'H-spiro[piperidine-4,5'-pyrrolo[1,2-b]pyrazol]-4'-amine | 481.11 |
| 57 | 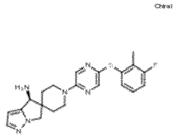 | (S)-1-(5-((3-fluoro-2-methylphenyl)thio)pyrazin-2-yl)-4'H,6'H-spiro[piperidine-4,5'-pyrrolo[1,2-b]pyrazol]-4'-amine | 411.17 |
| 58 | 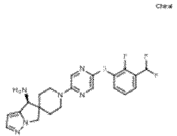 | (S)-1-(5-((3-(difluoromethyl)-2-fluorophenyl)thio)pyrazin-2-yl)-4'H,6'H-spiro[piperidine-4,5'-pyrrolo[1,2-b]pyrazol]-4'-amine | 447.15 |
| 59 | 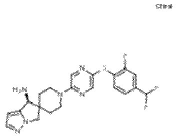 | (S)-1-(5-((4-(difluoromethyl)-2-fluorophenyl)thio)pyrazin-2-yl)-4'H,6'H-spiro[piperidine-4,5'-pyrrolo[1,2-b]pyrazol]-4'-amine | 447.15 |
| 60 | 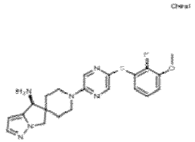 | (S)-1-(5-((2-fluoro-3-methoxyphenyl)thio)pyrazin-2-yl)-4'H,6'H-spiro[piperidine-4,5'-pyrrolo[1,2-b]pyrazol]-4'-amine | 427.17 |
| 61 | 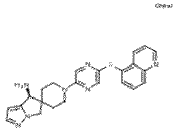 | (S)-1-(5-(quinolin-5-ylthio)pyrazin-2-yl)-4'H,6'H-spiro[piperidine-4,5'-pyrrolo[1,2-b]pyrazol]-4'-amine | 430.18 |
| 62 | 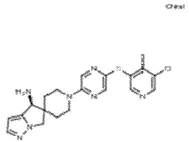 | (S)-1-(5-((5-chloro-4-methylpyridin-3-yl)thio)pyrazin-2-yl)-4'H,6'H-spiro[piperidine-4,5'-pyrrolo[1,2-b]pyrazol]-4'-amine | 428.14 |
| 63 | 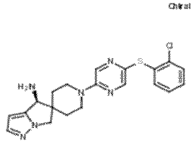 | (S)-1-(5-((2-chlorophenyl)thio)pyrazin-2-yl)-4'H,6'H-spiro[piperidine-4,5'-pyrrolo[1,2-b]pyrazol]-4'-amine | 413.13 |
| 64 | 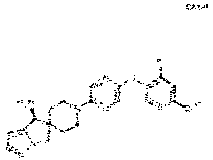 | (S)-1-(5-((2-fluoro-4-methoxyphenyl)thio)pyrazin-2-yl)-4'H,6'H-spiro[piperidine-4,5'-pyrrolo[1,2-b]pyrazol]-4'-amine | 427.17 |
| 65 | 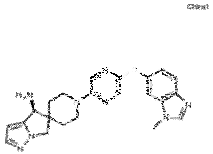 | (S)-1-(5-((1-methyl-1H-benzo[d]imidazol-6-yl)thio)pyrazin-2-yl)-4'H,6'H-spiro[piperidine-4,5'-pyrrolo[1,2-b]pyrazol]-4'-amine | 433.19 |
| 66 | 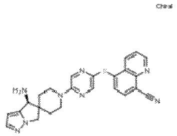 | (S)-5-((5-(4'-amino-4'H,6'H-spiro[piperidine-4,5'-pyrrolo[1,2-b]pyrazol]-1-yl)pyrazin-2-yl)thio)quinoline-8-carbonitrile | 455.17 |
| 67 | 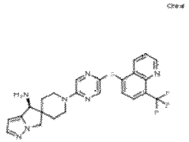 | (S)-1-(5-((8-(trifluoromethyl)quinolin-5-yl)thio)pyrazin-2-yl)-4'H,6'H-spiro[piperidine-4,5'-pyrrolo[1,2-b]pyrazol]-4'-amine | 498.16 |
| 68 | 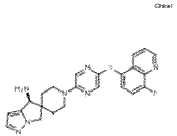 | (S)-1-(5-((8-fluoroquinolin-5-yl)thio)pyrazin-2-yl)-4'H,6'H-spiro[piperidine-4,5'-pyrrolo[1,2-b]pyrazol]-4'-amine | 448.17 |
| 69 | 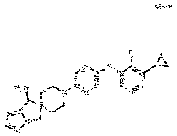 | (S)-1-(5-((3-cyclopropyl-2-fluorophenyl)thio)pyrazin-2-yl)-4'H,6'H-spiro[piperidine-4,5'-pyrrolo[1,2-b]pyrazol]-4'-amine | 437.19 |
| 70 | 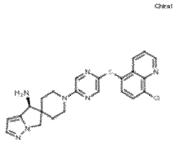 | (S)-1-(5-((8-chloroquinolin-5-yl)thio)pyrazin-2-yl)-4'H,6'H-spiro[piperidine-4,5'-pyrrolo[1,2-b]pyrazol]-4'-amine | 464.14 |
| 71 | 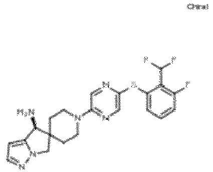 | (S)-1-(5-((2-(difluoromethyl)-3-fluorophenyl)thio)pyrazin-2-yl)-4'H,6'H-spiro[piperidine-4,5'-pyrrolo[1,2-b]pyrazol]-4'-amine | 447.15 |
| 72 | 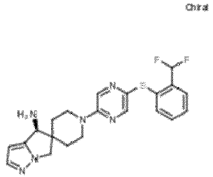 | (S)-1-(5-((2-(difluoromethyl)phenyl)thio)pyrazin-2-yl)-4'H,6'H-spiro[piperidine-4,5'-pyrrolo[1,2-b]pyrazol]-4'-amine | 429.16 |
| 73 | 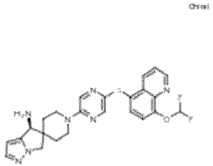 | (S)-1-(5-((8-(difluoromethoxy)quinolin-5-yl)thio)pyrazin-2-yl)-4'H,6'H-spiro[piperidine-4,5'-pyrrolo[1,2-b]pyrazol]-4'-amine | 496.17 |
| 74 | 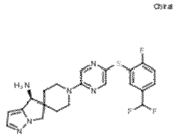 | (S)-1-(5-((5-(difluoromethyl)-2-fluorophenyl)thio)pyrazin-2-yl)-4'H,6'H-spiro[piperidine-4,5'-pyrrolo[1,2-b]pyrazol]-4'-amine | 447.15 |
| 75 | 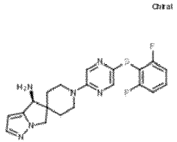 | (S)-1-(5-((2,6-difluorophenyl)thio)pyrazin-2-yl)-4'H,6'H-spiro[piperidine-4,5'-pyrrolo[1,2-b]pyrazol]-4'-amine | 415.15 |
| 76 | 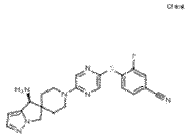 | (S)-4-((5-(4'-amino-4'H,6'H-spiro[piperidine-4,5'-pyrrolo[1,2-b]pyrazol]-1-yl)pyrazin-2-yl)thio)-3-fluorobenzonitrile | 422.15 |
| 77 | 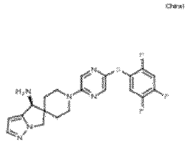 | (S)-1-(5-((2,4,5-trifluorophenyl)thio)pyrazin-2-yl)-4'H,6'H-spiro[piperidine-4,5'-pyrrolo[1,2-b]pyrazol]-4'-amine | 433.14 |
| 78 | 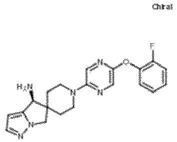 | (S)-1-(5-(2-fluorophenoxy)pyrazin-2-yl)-4'H,6'H-spiro[piperidine-4,5'-pyrrolo[1,2-b]pyrazol]-4'-amine | 381.18 |
| 79 | 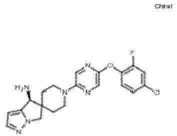 | (S)-1-(5-(4-chloro-2-fluorophenoxy)pyrazin-2-yl)-4'H,6'H-spiro[piperidine-4,5'-pyrrolo[1,2-b]pyrazol]-4'-amine | 415.14 |
| 80 | 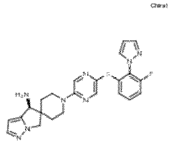 | (S)-1-(5-((3-fluoro-2-(1H-pyrazol-1-yl)phenyl)thio)pyrazin-2-yl)-4'H,6'H-spiro[piperidine-4,5'-pyrrolo[1,2-b]pyrazol]-4'-amine | 463.18 |
| 81 | 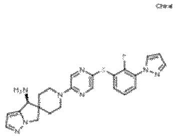 | (S)-1-(5-((2-fluoro-3-(1H-pyrazol-1-yl)phenyl)thio)pyrazin-2-yl)-4'H,6'H-spiro[piperidine-4,5'-pyrrolo[1,2-b]pyrazol]-4'-amine | 463.55 |
| 82 | 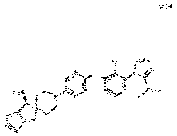 | (S)-1-(5-((2-chloro-3-(2-(difluoromethyl)-1H-imidazol-1-yl)phenyl)thio)pyrazin-2-yl)-4'H,6'H-spiro[piperidine-4,5'-pyrrolo[1,2-b]pyrazol]-4'-amine | 529.15 |
| 83 | 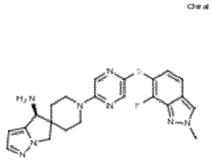 | (S)-1-(5-((7-fluoro-2-methyl-2H-indazol-6-yl)thio)pyrazin-2-yl)-4'H,6'H-spiro[piperidine-4,5'-pyrrolo[1,2-b]pyrazol]-4'-amine | 451.18 |
| 84 | 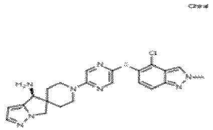 | (S)-1-(5-((4-chloro-2-methyl-2H-indazol-5-yl)thio)pyrazin-2-yl)-4'H,6'H-spiro[piperidine-4,5'-pyrrolo[1,2-b]pyrazol]-4'-amine | 467.15 |

### Intermediates 1 & 2: (R)-2-methyl-N-((S)-4'H,6'H-spiro[piperidine-4,5'-pyrrolo[1,2-b]pyrazol]-4'-yl)propane-2-sulfinamide--2,2,2-trifluoroacetaldehyde & (S)-4'H,6'H-spiro[piperidine-4,5'-pyrrolo[1,2-b]pyrazol]-4'-amine dihydrochloride

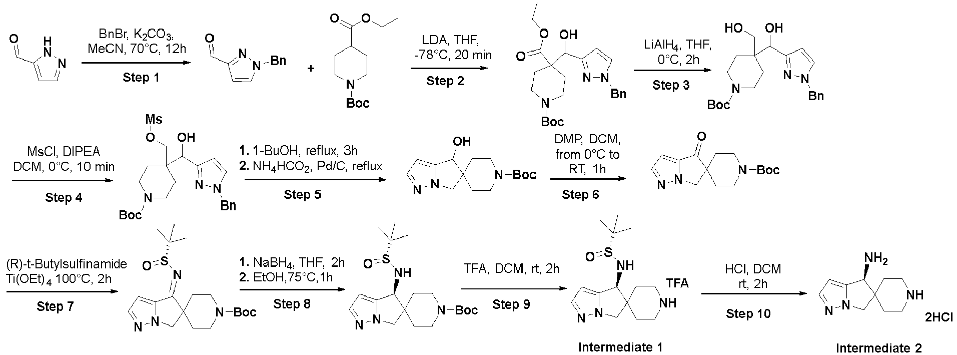

### Intermediate 1: (R)-2-methyl-N-((S)-4'H,6'H-spiro[piperidine-4,5'-pyrrolo[1,2-b]pyrazol]-4'-yl)propane-2-sulfinamide--2,2,2-trifluoroacetaldehyde

### Step 1: 1-benzyl-1H-pyrazole-3-carbaldehyde

A suspension of 1H-pyrazole-5-carbaldehyde (1.42 g, 14.78 mmol), K₂CO₃ (5.10 g, 36.95 mmol) and BnBr (3 mL, 25.26 mmol) in dry MeCN (27 mL) was heated at 70°C for 12 h. The mixture was diluted with toluene and washed with H₂O and brine. The organic layer was dried over Na₂SO₄, filtered and the solvent was removed under *vacuo.* The residue was purified by flash chromatography on silica gel (from 0% to 20% EtOAc in cyclohexane) to afford the title compound as a yellow oil (1.86 g, 68%). ¹H NMR (400 MHz, DMSO-*d*₆) δ 9.87 (s, 1H), 8.06 (s, 1H), 7.42-7.26 (m, 5H), 6.81 (s, 1H), 5.48 (s, 2H); LCMS (ES⁺) Method 1: *m*/*z* 187 (M+H)⁺, RT 1.60 min.

### Step 2: 1-(tert-butyl) 4-ethyl 4-((1-benzyl-1Hpyrazol-3yl)(hydroxy)methyl)piperidine-1,4-dicarboxylate

LDA (2N in THF; 6 mL, 12.0 mmol) was added to a stirring solution of ethyl 1-*tert-*butoxycarbonylpiperidine-4-carboxylate (2.45 g, 9.52 mmol) in dry THF (20 mL) at -78°C. After 10 min a solution of 1-benzyl-1H-pyrazole-3-carbaldehyde (1.86 g, 9.99 mmol) in dry THF (5 mL) was added slowly. After 5 min the mixture was allowed to warm up to rt. After 20 min the mixture was diluted with toluene and HCl 2N to pH=6 at 0°C. The organic layer was washed with H₂O, brine, dried over Na₂SO₄, filtered and the solvent was removed under reduced pressure to obtain the crude compound as a yellow sticky solid that was used in the next step without purification (4.35 g). ¹H NMR (400 MHz, DMSO-*d*₆) δ 7.72 (d, *J*=2.2 Hz, 1H), 7.36-7.24 (m, 3H), 7.24-7.13 (m, 2H), 6.17 (d, *J*=2.2 Hz, 1H), 5.47 (d, *J*=5.5 Hz, 1H), 5.26 (s, 2H), 4.64 (d, *J*=5.5 Hz, 1H), 4.04-3.92 (m, 2H), 3.92-3.76 (m, 2H), 2.77-2.55 (m, 2H), 1.93-1.79 (m, 1H), 1.59-1.30 (m, 12H), 1.12 (t, *J*=7.1 Hz, 3H); LCMS (ES⁺) Method 1: *m*/*z* 444 (M+H)⁺, RT 2.05 min.

### Step 3: tert-butyl 4-((1-benzyl-1H-pyrazol-3yl)(hydroxy)methyl)-4 (hydroxymethyl)piperidine-1-carboxylate

LiALH₄ (1N in THF; 7.2 mL, 14.4 mmol) was added to a stirring solution 1-(*tert*-butyl) 4-ethyl 4-((1-benzyl-1H-pyrazol-3-yl)(hydroxy)methyl)piperidine-1,4-dicarboxylate (5.28 g, 11.9 mmol) in dry THF (30 mL) at 0°C. After 1.5 h, LiAlH₄ (1N in THF; 1 mL, 2.0 mmol) was added. After 30 min the mixture was quenched with slow addition of 5% citric acid solution (to pH=6) and Rochelle salt sol. at 0°C and diluted with toluene. The organic layer was washed with H₂O (x2) and brine, dried over Na₂SO₄, filtered and the solvent removed under reduced pressure to obtain the crude product as a colorless solid that was used in the next step without purification (4.7 g). LCMS (ES⁺) Method 1: *m*/*z* 402 (M+H)⁺, RT 1.77 min.

### Step 4: tert-butyl 4-((1-benzyl-1H-pyrazol-3-yl)(hydroxy)methyl)-4-(((methylsulfonyl)oxy)methyl)piperidine-1-carboxylate

To a stirring solution of *tert-butyl* 4-((1-benzyl-1H-pyrazol-3-yl)(hydroxy)methyl)-4-(hydroxymethyl)piperidine-1-carboxylate (4.7 g, 11.71 mmol) in DCM (60 mL), dry DIPEA (3.1 mL, 17.8 mmol) and MsCl (0.95 mL, 12.3 mmol) were added at 0°C. After 5 min the mixture was diluted with DCM and 5% citric acid solution was added at 0°C. Then the organic layer was washed with H₂O and brine, dried over Na₂SO₄, filtered and the solvent removed under reduced pressure. The residue was purified by flash chromatography on silica gel (from 20% to 70% EtOAc in cyclohexane) to afford the title compound as white spongy solid (3.1 g, 56 % over 3 steps). ¹H NMR (400 MHz, DMSO-*d₆*) δ 7.76 (d, *J*=2.1 Hz, 1H), 7.36-7.23 (m, 3H), 7.23-7.16 (m, 2H), 6.23 (d, *J*=2.2 Hz, 1H), 5.42 (d, *J*=4.9 Hz, 1H), 5.29 (s, 2H), 4.63 (d, *J*=4.9 Hz, 1H), 4.32 (d, *J*=9.8 Hz, 1H), 4.18 (d, *J*=9.7 Hz, 1H), 3.51 (br dd, *J*=15.9, 13.8 Hz, 2H), 3.21-2.96 (m, 5H), 1.57-1.31 (m, 13H). LCMS (ES⁺) Method 1: *m*/*z* 480 (M+H)⁺, RT 1.93 min.

### Step 5: tert-butyl 4'-hydroxy-4'H,6'H-spiro[piperidine-4,5'-pyrrolo[1,2-b]pyrazole]-1-carboxylate

A solution of *tert-butyl* 4-((1-benzyl-1H-pyrazol-3-yl)(hydroxy)methyl)-4-(((methylsulfonyl)oxy)methyl)piperidine-1-carboxylate (3.70 g, 7.71 mmol) in 1-BuOH (64 mL) was heated at 120°C in a sealed tube for 3 h, then cooled to rt and treated with NH₄HCO₂ (1.46 g, 23.15 mmol) and Pd/C 10% wt (0.41 g, 0.39 mmol). The mixture was heated at reflux for 15 min and then left stirred at rt for 2 h. The mixture was filtered over alpha cellulose pad and washed with EtOAc and MeOH. The volatiles were removed under reduced pressure and the product was purified by flash chromatography on silica gel (from 20% to 100% EtOAc +3% MeOH in petroleum ether) to obtain the title compound as a white solid (1.40 g, 61% over 2 steps). ¹H NMR (400 MHz, DMSO-*d₆*) δ 7.42 (d, *J*=1.8 Hz, 1H), 6.11 (d, *J*=1.8 Hz, 1H), 5.52 (d, *J*=7.0 Hz, 1H), 4.61 (d, *J*=7.0 Hz, 1H), 4.04-3.95 (m, 2H), 3.66-3.54 (m, 2H), 3.15 (br d, *J*=9.3 Hz, 2H), 1.79-1.67 (m, 1H), 1.55-1.31 (m, 12H); LCMS (ES⁺) Method 1: *m*/*z* 294 (M+H)⁺, RT 1.43 min.

### Step 6: tert-butyl 4'-oxo-4'H,6'H-spiro[piperidine-4,5'-pyrrolo[1,2-b]pyrazole]-1-carboxylate

To a stirring solution of *tert-butyl* 4'-hydroxy-4'H,6'H-spiro[piperidine-4,5'-pyrrolo[1,2-b]pyrazole]-1-carboxylate (1.6 g, 5.45 mmol) in DCM (24 mL), DMP (2.5 g, 6.01 mmol) was added portion wise at 0°C. The resulting mixture was allowed to warm up to rt. After 1 h the mixture was diluted with DCM and washed with 10% Na₂S₂O₃ sol., H₂O and brine. The organic layer was dried over Na₂SO₄, filtered and the solvent was removed under reduced pressure to afford a yellow solid. The residue was purified by flash chromatography on silica gel (from 0% to 100% EtOAc in petroleum ether) to afford the title compound as off-white solid (1.25 g, 78%). ¹H NMR (400 MHz, DMSO-*d₆*) δ 7.90 (d, *J*=2.2 Hz, 1H), 6.79 (d, *J*=2.2 Hz, 1H), 4.53 (s, 2H), 3.94 (br d, *J*=13.5 Hz, 2H), 2.97 (br s, 2H), 1.75-1.59 (m, 4H), 1.42 (s, 9H); LCMS (ES⁺) Method 1: *m*/*z* 292 (M+H)⁺, RT 1.73 min.

### Step 7: tert-butyl (R,Z)-4'-((tert-butylsulfmyl)imino)-4'H,6'H-spiro[piperidine-4,5'-pyrrolo[1,2-b]pyrazole]-1-carboxylate

A solution of *tert-butyl* 4'-oxo-4'H,6'H-spiro[piperidine-4,5'-pyrrolo[1,2-b]pyrazole]-1-carboxylate (980 mg, 2.17 mmol) and (R)-(-)-*tert*-butyl sulfinamide (790 mg, 6.52 mmol) in Ti(OEt)₄ (4 mL) was heated at 100°C for 1 h. The mixture was cooled to rt, diluted with EtOAc and H₂O and the solid precipitated filtered and washed with EtOAc. The organic layer was washed with H₂O (x3), brine, dried over Na₂SO₄, filtered and the solvent removed under reduced pressure. The residue was purified by flash chromatography on silica gel (from 20% to 100% EtOAc in petroleum ether) to afford the title compound as a pale-yellow solid (740 mg, 86%). ¹H NMR (400 MHz, DMSO-*d₆*) δ 7.78 (d, *J*=2.1 Hz, 1H), 6.91 (d, *J*=2.1 Hz, 1H), 4.46 (d, *J*=2.9 Hz, 2H), 3.98 (br d, *J*=13.7 Hz, 2H), 3.09-2.80 (m, 2H), 1.63-1.86 (m, 4H), 1.42 (s, 9H), 1.20 (s, 9H); LCMS (ES⁺) Method 1: *m*/*z* 395 (M+H)⁺, RT 1.91 min.

### Step 8: tert-butyl (S)-4'-(((R)-tert-butylsulfinyl)amino)-4'H,6'H-spiro[piperidine-4,5'-pyrrolo[1,2-b]pyrazole]-1-carboxylate

NaBH₄ (0.23 g, 6 mmol) was added to a solution of *tert-butyl* (*S*, *Z*)-4'-((*tert*-butylsulfinyl)imino)-4'H,6'H-spiro[piperidine-4,5'-pyrrolo[1,2-b]pyrazole]-1-carboxylate (1.57 g, 3.97 mmol) in dry THF (15 mL) at - 50°C. After 5 min the mixture was allowed to warm up to rt. After 1.5 h, additional NaBH₄ (30 mg, 0.79 mmol) was added and after 30 min the mixture was diluted with EtOAc and H₂O at 0°C. The organic layer was washed with brine, dried over Na₂SO₄, filtered and the solvent evaporated under reduced pressure to afford a pale-yellow solid, which was dissolved in EtOH (16 mL) and heated at 75°C for 1 h (88/12 diastereomeric ratio observed by UPLC-MS). The mixture was then evaporated under reduced pressure and the product was purified by flash chromatography on silica gel (from 0% to 100% EtOAc +3% MeOH in DCM) to afford the title compound as a white solid (908 mg, 62%, diastereomeric ratio = 97/3). ¹H NMR (400 MHz, DMSO-*d₆*) δ 7.46 (d, *J*=1.7 Hz, 1H), 6.07 (d, *J*=1.6 Hz, 1H), 5.93 (d, *J*=10.0 Hz, 1H), 4.43 (d, *J*=9.9 Hz, 1H), 4.19-4.11 (m, 1H), 4.08-3.96 (m, 2H), 3.82 (br t, *J*=14.0 Hz, 2H), 3.12-2.80 (m, 2H), 1.80-1.55 (m, 3H), 1.41 (s, 9H), 1.15 (s, 9H); LCMS (ES⁺) Method 1: *m*/*z* 397 (M+H)⁺, RT 1.66 min.

### Step 9: (R)-2-methyl-N-((S)-4'H,6'H-spiro[piperidine-4,5'-pyrrolo[1,2-b]pyrazol]-4'-yl)propane-2-sulfinamide--2,2,2-trifluoroacetaldehyde

TFA (0.2 mL, 2.61 mmol) was added to a solution of *tert*-butyl-(*S*)-4'-(((*R*)-*tert*-butylsulfinyl)amino)-4'H,6'H-spiro[piperidine-4,5'-pyrrolo[1,2-b]pyrazole]-1-carboxylate (90 mg, 0.23 mmol) in dry DCM (1.5 mL) at rt. After 2 h the mixture was evaporated under reduced pressure to afford a colorless solid (95 mg, quant.). ¹H NMR (400 MHz, DMSO-*d₆*) δ 8.68-8.24 (m, 3H), 7.48 (d, *J*=1.8 Hz, 1H), 6.20-6.04 (m, 2H), 4.52 (d, *J*=10.0 Hz, 1H), 4.14 (dd, *J*=19.3, 11.3 Hz, 2H), 3.44-3.18 (m, 2H), 3.17-2.89 (m, 2H), 2.07-1.65 (m, 4H), 1.30-1.09 (m, 9H); LCMS (ES⁺) Method 1: *m*/*z* 297 (M+H) ⁺, RT 0.89 min.

### Intermediate 2: (S)-4'H,6'H-spiro[piperidine-4,5'-pyrrolo[1,2-b]pyrazol]-4'-amine dihydrochloride

### Step 10: (S)-4'H,6'H-spiro[piperidine-4,5'-pyrrolo[1,2-b]pyrazol]-4'-aminedihydrochloride

HCl (4N in 1,4 dioxane; 2.3 mL, 9.2 mmol) was added to a stirring solution of *tert*-butyl (*S*)-4'-(((*S*)-*tert-*butylsulfinyl)amino)-4'H,6'H-spiro[piperidine-4,5'-pyrrolo[1,2-b]pyrazole]-1-carboxylate (908 mg, 2.29 mmol) in DCM (5 mL) at rt. After 2 h the product was filtered, washed with DCM, and dried under reduced pressure to obtain a pale-yellow solid (463 mg, 75%). ¹H NMR (DMSO-*d₆*+TFA) δ 8.97 (br s, 2H), 8.76 (br s, 3H), 7.54 (d, *J*=1.7 Hz, 1H), 6.33 (d, *J*=1.7 Hz, 1H), 4.59-4.45 (m, 1H), 4.41-4.18 (m, 2H), 3.38 (br d, *J*=12.7 Hz, 1H), 3.29-3.10 (m, 2H), 3.10-2.87 (m, 1H), 2.17-1.80 (m, 3H), 1.80-1.65 (m, 1H); LCMS (ES⁺) Method 2: *m*/*z* 193 (M+H)⁺, RT 0.54 min.

### Intermediate 3: (S)-3'-fluoro-4'H,6'H-spiro[piperidine-4,5'-pyrrolo[1,2-b]pyrazol]-4'-amine dihydrochloride

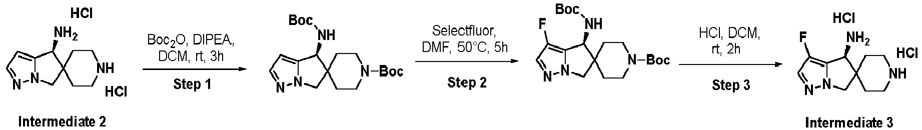

### Step 1: tert-butyl (S)-4'-((tert-butoxycarbonyl)amino)-4'H,6'H-spiro[piperidine-4,5'-pyrrolo[1,2-b]pyrazole]-1-carboxylate

Dry DIPEA (0.4 mL, 2.3 mmol) was added to a suspension **Intermediate 2** (200 mg, 0.75 mmol) and Boc₂O (412 mg, 1.89 mmol) in dry DCM (3 mL). After 3 h the mixture was diluted with DCM and washed with 5% citric acid solution, H₂O and brine. The organic layer was dried over Na₂SO₄, filtered and solvent removed under reduced pressure. The residue was purified by flash chromatography on silica gel (from 0% to 50% EtOAc in petroleum ether) to afford the title compound as colorless solid (225 mg, 76%). LCMS (ES⁺) Method 1: *m*/*z* 393 (M+H)⁺, RT 1.95 min.

### Step 2: tert-butyl (S)-4'-((tert-butoxycarbonyl)amino)-3'-fluoro-4'H,6'H-spiro[piperidine-4,5'-pyrrolo[1,2-b]pyrazole]-1-carboxylate

Selectfluor (53 mg, 0.29 mmol) was added to a stirred solution of *tert*-butyl (*S*)*-*4'*-*((*tert-*butoxycarbonyl)amino)-4'H,6'H-spiro[piperidine-4,5'-pyrrolo[1,2-b]pyrazole]-1-carboxylate (65 mg, 0.17 mmol) in dry DMF (1.3 mL) at 50°C. After 5 h, the mixture was diluted with toluene and washed with H₂O (x2) and brine. The organic layer was dried over Na₂SO₄, filtered and the solvent was removed under reduced pressure. The residue was purified by preparative HPLC (from 20% to 65% MeCN/H₂O + 0.1% TFA) to afford the title compound as pale-yellow solid (10 mg, 14%). ¹H NMR (400 MHz, DMSO-*d₆*) δ 7.57 (d, *J*=9.7 Hz, 1H), 7.44 (d, *J*=4.0 Hz, 1H), 4.85 (d, *J*=9.6 Hz, 1H), 4.01 (s, 2H), 3.69-3.48 (m, 2H), 3.26-2.97 (m, 2H), 1.66-1.47 (m, 4H), 1.37 (s, 18H). LCMS (ES⁺) Method 1: *m*/*z* 411 (M+H)⁺, RT 2.06 min.

### Step 3: (S)-3'-fluoro-4'H,6'H-spiro[piperidine-4,5'-pyrrolo[1,2-b]pyrazol]-4'-amine dihydrochloride

The title compound was prepared according to the procedure described for the synthesis of **Intermediate 2** in Step 10 starting from *tert*-butyl (*S*)-4'-((*tert*-butoxycarbonyl)amino)-3'-fluoro-4'H,6'H-spiro[piperidine-4,5'-pyrrolo[1,2-b]pyrazole]-1-carboxylate (from Step 2; 10 mg, 0.02 mmol) to afford a white powder (5 mg, 73%). LCMS (ES⁺) Method 1: *m*/*z* 211 (M+H)⁺, RT 0.34 min.

### Intermediate 4: 3-(2,3-dichlorophenyl)-6-hydroxy-2-methylpyrimidin-4(3H)-one

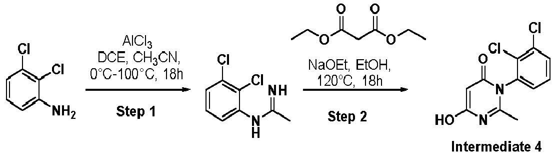

### Step 1: N-(2,3-dichlorophenyl)acetimidamide

A solution of 2,3-dichloroaniline (1.62 g, 10 mmol) in dry DCE (10 mL) and MeCN (0.78 mL) was treated with AlCl₃ (1.47 g, 11 mmol) at 0°C. The reaction mixture was stirred at 0°C for 10 min then at 100°C for 18 h. After cooling the reaction mixture was treated with ice H₂O and extracted with DCM. NaOH (2N) was added to the mixture to adjust the pH to 10 and the aqueous phase was extracted with DCM. The combined organic layers were dried over Na₂SO₄ and concentrated *in vacuo* to afford the title compound as a brown oil (1.62 g, 80%) which was used as a crude without further purification. LCMS (ES⁺) Method 1: *m*/*z* 203 (M+H)⁺, RT 1.15 min.

### Step 2: 3-(2,3-dichlorophenyl)-6-hydroxy-2-methylpyrimidin-4(3H)-one

A suspension of *N*-(2,3-dichlorophenyl)acetimidamide (1.61 g, 7.93 mmol) in EtOH (8 mL) was treated with diethyl propanedioate (2.42 mL, 15.86 mmol) and EtONa (20% solution in EtOH; 8.88 mL, 23.78 mmol) and the resulting mixture was stirred in a sealed tube at 120°C for 18 h. The reaction mixture was allowed to cool to 25°C and the volatiles were removed under reduced pressure. H₂O was added to the residue, the mixture was cooled to 0°C and acidified to pH~2 with HCl (6N). The mixture was allowed to warm to 25°C and stirred for 1 h. The solid formed was collected by *vacuum* filtration, rinsed with Et₂O, and dried under reduced pressure to afford the title compound (955 mg, 44%). LCMS (ES⁺) Method 1: *m*/*z* 271 (M+H)⁺, RT 1.08 min.

### Intermediate 5: 6-chloro-3-(2,3-dichlorophenyl)-2,5-dimethylpyrimidin-4(3H)-one

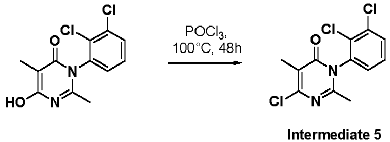

A solution of 3-(2,3-dichlorophenyl)-6-hydroxy-2,5-dimethylpyrimidin-4(3H)-one (prepared as reported in the synthesis of **Intermediate 4** using diethyl 2-methylmalonate as reagent in step 2; 360 mg, 1.26 mmol) in POCl₃ (4 mL) was heated at 100°C for 48 h. The reaction mixture was cooled to rt and concentrated under reduced pressure. Ice H₂O was added to the mixture which was extracted with DCM. The organic layer was washed with NaHCO₃ sat. sol, dried over Na₂SO₄, filtered, and concentrated *in vacuo.* The crude product was purified by flash chromatography (0-100% EtOAc in petroleum ether) to afford the title compound as a white powder (194 mg, 51%). ¹H NMR (DMSO-*d₆*) δ 7.93 (d, *J*=7.9 Hz, 1H), 7.75-7.65 (m, 2H), 2.13 (s, 6H); LCMS (ES⁺) Method 1: *m*/*z* 303 (M+H)⁺, RT 1.90 min.

### Intermediate 6: 6-chloropyrido[2,3-b]pyrazin-2-yl 4-nitrobenzenesulfonate

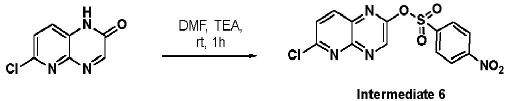

A solution of 6-chloropyrido[2,3-b]pyrazin-2(1H)-one (1.0 g, 5.5 mmol) in DMF (10 mL) was treated with TEA (0.92 mL, 6.6 mmol) and 4-nitrobenzenesulfonyl chloride (1.2 g, 5.51 mmol). The mixture was stirred at rt for 1 h then poured into H₂O and the formed precipitate was filtered off and dried to afford the title product as a brown solid (1.87 g, 92%). ¹H NMR (400 MHz, DMSO-*d₆*) δ 9.13 (s, 1H), 8.52-5.50 (m, 5H), 8.01 (d, *J*=8.7 Hz, 1H). LCMS (ES⁺) Method 1: *m*/*z* 367 (M+H)⁺, RT 1.78 min.

### Intermediate 7: sodium 5-chloropyrazine-2-thiolate

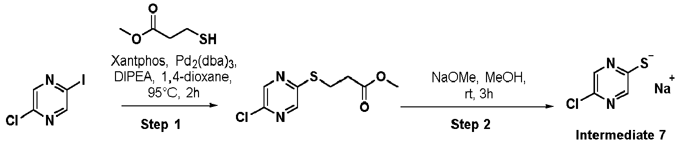

### Step 1: methyl 3-((5-chloropyrazin-2-yl)thio)propanoate

A pressure tube was charged with methyl 3-mercaptopropanoate (200 uL, 1.60 mmol), 2-chloro-5-iodopyrazine (350 mg, 1.46 mmol), DIPEA (648 uL, 3.64 mmol), Xantphos (42.1 mg, 0.07 mmol), Pd₂(dba)₃ (33.3 mg, 0.04 mmol) and 1,4-dioxane (4.4 mL) and the mixture was degassed and heated at 95°C for 2 h. After cooling the reaction mixture was concentrated *in vacuo* to get a residue which was purified by flash chromatography on silica gel (from 0% to 50% EtOAc in petroleum ether) to afford the title compound as a yellow powder (330 mg, 97%). LCMS (ES⁺) Method 1: *m*/*z* 233 (M+H)⁺, RT 1.66 min.

### Step 2: sodium 5-chloropyrazine-2-thiolate

NaOMe (20% solution w/w in MeOH; 0.21 mL, 0.85 mmol) was added to a solution of methyl 3-((5-chloropyrazin-2-yl)thio)propanoate (330 mg, 0.71 mmol) in MeOH (2 mL) and the mixture was stirred at rt for 3 h, then evaporated under reduced pressure to afford a residue which was treated with Et₂O (30 mL). The solution was left at -20°C for 18 h. The precipitate formed was filtered and washed with Et₂O and DCM giving the title compound as a yellow powder (119 mg, 99%). LCMS (ES⁺) Method 1: *m*/*z* 145 (M+H)⁻RT 0.74 min.

### Intermediate 8: 1-(2-chloro-3-iodophenyl)-1H-pyrazole

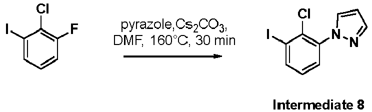

A solution of pyrazole (78 mg, 1.17 mmol) in DMF (6.0 mL) was treated with Cs₂CO₃ (380 mg, 1.17 mmol) and 2-chloro-1-fluoro-3-iodobenzene (250 mg, 0.98 mmol) and stirred under MW irradiation at 160°C for 30 min. After cooling the solvent was removed under reduced pressure and the residue was dissolved in EtOAc and washed with H₂O. The organic solvent was dried over Na₂SO₄, filtered, and concentrated *in vacuo* to afford a residue which was purified by flash chromatography on silica gel (from 0% to 100% EtOAc in petroleum ether) to furnish the title compound as a white powder (198 mg, 67%). LCMS (ES⁺) Method 1: *m*/*z* 305 (M+H)⁺, RT 1.83 min.

### Intermediate 9:-1'H,3'H-spiro[piperidine-4,2'-pyrrolizin]-1'-one hydrochloride

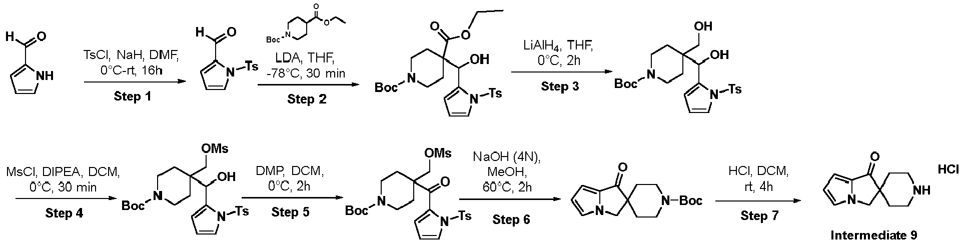

### Step 1: 1-tosyl-1H-pyrrole-2-carbaldehyde

NaH (60% wt in mineral oil; 547 mg, 13.67 mmol) was added to a solution of 1H-pyrrole-2-carbaldehyde (1.0 g, 10.52 mmol) in dry DMF (20 mL) at 0°C. After 10 min TsCl (3.1 g, 1.55 mmol) was added and mixture allowed to warm up to rt and stirred for 16 h before diluted with EtOAc and washed with citric acid (5% aq. sol.), H₂O and brine. The organic layer was dried over Na₂SO₄, filtered and the solvent removed under reduced pressure to afford the title compound as a light brown solid (2.5 g, 96%) that was used in the next step without further purification. LCMS (ES⁺) Method 1: *m*/*z* 250 (M+H)⁺, RT 1.89 min.

### Step 2: 1-(tert-butyl) 4-ethyl 4-(hydroxy(1-tosyl-1H-pyrrol-2-yl)methyl)piperidine-1,4-dicarboxylate

LDA (2N in THF; 5.2 mL, 10.4 mmol) was added to a solution of ethyl 1-*tert*-butoxycarbonylpiperidine-4-carboxylate (2.25 g, 8.7 mmol) in dry THF (28 mL) at -78°C. After 10 min a solution of 1-tosyl-1H-pyrrole-2-carbaldehyde (2.51 g, 10.1 mmol) in dry THF (7 mL) was added slowly and stirred at -78°C for 30 min. The reaction mixture was diluted with toluene and citric acid (5% aq. sol.) until pH=5 at 0°C. The organic layer was washed with H₂O and brine, dried over Na₂SO₄, filtered and the solvent removed under reduced pressure to afford the title compound as a yellow oil (4.4 g, 99%) which was used in the next step without further purification. LCMS (ES⁺) Method 1: *m*/*z* 507 (M+H)⁺, RT 2.35 min.

### Step 3: tert-butyl 4-(hydroxy(1-tosyl-1H-pyrrol-2-yl)methyl)-4-(hydroxymethyl)piperidine-1-carboxylate

LiAlH₄ (2N in THF; 6 mL, 12.0 mmol) was added to a solution of 1-(*tert*-butyl) 4-ethyl 4-(hydroxy(1-tosyl-1H-pyrrol-2-yl)methyl)piperidine-1,4-dicarboxylate (5 g, 9.8 mmol) in dry THF (24 mL) at 0°C and the reaction mixture was stirred at this temperature for 2 h before being diluted with toluene and quenched with slow addition of citric acid (5% aq. sol.) until pH=6. The aqueous phase was extracted with toluene and the organic layer was washed with H₂O and brine, dried over Na₂SO₄, filtered and the solvent removed under reduced pressure to afford the title compound as a brown sticky solid (3.9 g, 85%). LCMS (ES⁺) Method 1: *m*/*z* 465 (M+H)⁺, RT 1.99 min.

### Step 4: tert-butyl 4-(hydroxy(1-tosyl-1H-pyrrol-2-yl)methyl)-4-(((methylsulfonyl)oxy)methyl)piperidine-1-carboxylate

A solution of *tert-butyl* 4-(hydroxymethyl)-4-[oxidanyl-[1-(phenylmethyl)pyrazol-3-yl]methyl]piperidine-1-carboxylate (3.9 g, 8.4 mmol) in DCM (50 mL) was treated with dry DIPEA (1.8 mL, 10.3 mmol) and MsCl (0.65 mL, 8.4 mmol) at 0°C and stirred at this temperature for 30 min before being quenched with citric acid (5% aq. sol.) and diluted with DCM. The organic layer was washed with H₂O and brine, dried over Na₂SO₄, filtered and solvent removed under reduced pressure. The residue was purified by flash chromatography on silica gel (from 0% to 70% EtOAc in cyclohexane) to afford the title compound as a pale red spongy solid (1.5 g, 34%). LCMS (ES⁺) Method 1: *m*/*z* 543 (M+H)⁺, RT 2.06 min.

### Step 5: tert-butyl 4-(((methylsulfonyl)oxy)methyl)-4-(1-tosyl-1H-pyrrole-2-carbonyl)piperidine-1-carboxylate

A solution of *tert-butyl* 4-(hydroxy(1-tosyl-1H-pyrrol-2-yl)methyl)-4-(((methylsulfonyl)oxy)methyl)piperidine-1-carboxylate (900 mg, 1.66 mmol) in DCM (8 mL) was treated with DMP (774 mg, 1.82 mmol) at 0°C and stirred at this temperature for 1 h before being diluted with DCM and washed with NaHCO₃ (sat. sol.), H₂O and brine. The organic layer was dried over Na₂SO₄, filtered and the solvent removed under reduced pressure. The residue was purified by flash chromatography on silica gel (from 0% to 70% EtOAc in cyclohexane) to afford the title compound as a light orange spongy solid (525 mg, 59%). LCMS (ES⁺) Method 1: *m*/*z* 541 (M+H)⁺, RT 2.16 min.

### Step 6: tert-butyl 1'-oxo-1'H,3'H-spiro[piperidine-4,2'-pyrrolizine]-1-carboxylate

NaOH (4N, 10 mL, 40 mmol) was added to a stirred solution of *tert-butyl* 4-(((methylsulfonyl)oxy)methyl)-4-(((tetrahydro-2H-pyran-2-yl)oxy)(1-tosyl-1H-pyrrol-2-yl)methyl)piperidine-1-carboxylate (525 mg, 0.97 mmol) in MeOH (10 mL) and the mixture was stirred at 60°C for 2 h before being cooled to rt and diluted with EtOAc and H₂O. The organic layer was washed with H₂O, brine, dried over Na₂SO₄, filtered and the solvent was removed under reduced pressure to afford the title compound as a light-brown solid (281 mg, 99%). LCMS (ES⁺) Method 1: *m*/*z* 291 (M+H)⁺, RT 1.71 min.

### Step 7: 1'H,3'H-spiro[piperidine-4,2'-pyrrolizin]-1'-one hydrochloride

HCl (4N in 1,4-dioxane; 1.5 mL, 6 mmol) was added to a solution of 1'H,3'H-spiro[piperidine-4,2'-pyrrolizin]-1'-one hydrochloride (280 mg, 0.96 mmol) in DCM (7 mL) and the reaction mixture was stirred at rt for 4 h before being evaporated under reduced pressure to afford a residue which was suspended in Et₂O and filtered. The solvent was removed under reduced pressure to afford the title compound as a violet powder (214 mg, 98%) which was used without further purification. ¹H NMR (400 MHz, DMSO-*d₆*) δ 9.14 (br s, 1H), 8.98-8.61 (m, 1H), 7.47-7.41 (m, 1H), 6.75 (dd, *J*=1.0, 3.9 Hz, 1H), 6.62 (dd, *J*=2.2, 4.0 Hz, 1H), 4.40 (s, 2H), 3.47-3.39 (m, 2H), 3.06 (br d, *J*=9.6 Hz, 2H), 2.17-1.92 (m, 2H), 1.92-1.74 (m, 2H). LCMS (ES⁺) Method 1: *m*/*z* 191 (M+H)⁺, RT 0.62 min.

### Intermediates 10 & 11: 1-(2-fluoro-6-iodophenyl)-1H-pyrazole & 1-(2-fluoro-3-iodophenyl)-1H-pyrazole

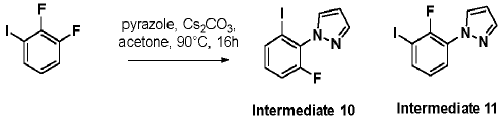

The title compounds were prepared following procedure reported for the synthesis of **Intermediate 8** using 1,2-difluoro-3-iodobenzene in acetone at 90°C for 16 h under MW irradiation. **Intermediate 10**: (32 mg, 53%); LCMS (ES⁺) Method 1: *m*/*z* 289 (M+H)⁺, RT 1.59 min. **Intermediate 11**: (9 mg, 15%); LCMS (ES⁺) Method 1: *m*/*z* 289 (M+H)⁺, RT 1.88 min.

### Intermediate 12: 1-(2-chloro-3-iodophenyl)-2-(difluoromethyl)-1H-imidazole

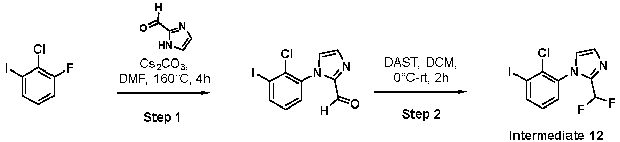

### Step 1: 1-(2-chloro-3-iodophenyl)-1H-imidazole-2-carbaldehyde

The title compound was prepared following procedure reported for the synthesis of **Intermediate 8** (32 mg, 25%). LCMS (ES⁺) Method 1: *m*/*z* 333 (M+H)⁺, RT 1.58 min.

### Step 2: 1-(2-chloro-3-iodophenyl)-2-(difluoromethyl)-1H-imidazole

A solution of 1-(2-chloro-3-iodophenyl)-1H-imidazole-2-carbaldehyde (21 mg, 0.06 mmol) in DCM (0.6 mL) was treated with DAST (26 mg, 0.16 mmol) at 0°C. The mixture was stirred at rt for 2 h before being cooled back to 0°C and treated with NaHCO₃ (sat. sol.) and DCM. The organic layer was washed with brine, dried over Na₂SO₄, filtered and the solvent removed under reduced pressure. The residue was purified by flash chromatography on silica gel (from 0% to 70% EtOAc in petroleum ether) to afford the title compound (10 mg, 45%). LCMS (ES⁺) Method 1: *m*/*z* 355 (M+H)⁺, RT 1.74 min.

### Intermediate 13: 5-bromo-4-fluoro-2-methyl-2H-indazole

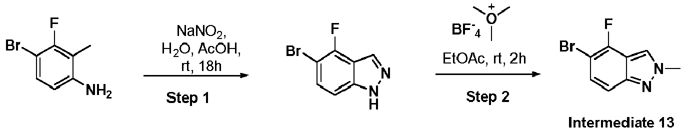

### Step 1: 5-bromo-4-fluoro-1H-indazole

A solution of NaNO₂ (84 mg, 1.23 mmol) in H₂O (1 mL) was added to an ice cooled solution of 4-bromo-3-fluoro-2-methylaniline (100 mg, 0.49 mmol) in AcOH (2.2 mL). The reaction mixture was left warming to rt and stirred for 18 h. The product was precipitated adding H₂O and washing the residue with petroleum ether to afford the title compound as an orange powder (68 mg, 65%). LCMS (ES⁺) Method 1: *m*/*z* 215 (M+H)⁺, RT 1.55 min.

### Step 2: 5-bromo-4-fluoro-2-methyl-2H-indazole

A solution of 5-bromo-4-fluoro-1H-indazole (68 mg, 0.31 mmol) in EtOAc (1.5 mL) was treated with trimethyloxonium tetrafluoroborate (51 mg, 0.34 mmol) and stirred at rt for 2 h before adding H₂O. The precipitate formed was filtered and washed with H₂O and petroleum ether to afford the title compound as a brown oil (63 mg, 88%). LCMS (ES⁺) Method 1: *m*/*z* 229-231 (M+H)⁺, RT 1.60 min.

### Intermediate 14: sodium 4-chloro-2-methyl-2H-indazole-5-thiolate

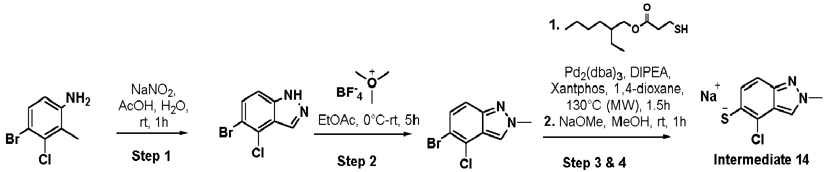

### Step 1: 5-Bromo-4-chloro-1H-indazole

A solution of 4-bromo-3-chloro-2-methylaniline (867 mg, 3.93 mmol) in AcOH (17 mL) was treated with NaNO₂ (339 mg, 4.91 mmol) in H₂O (1.5 mL). The mixture was stirred at rt for 1 h. The solvent was concentrated under reduced pressure and the residue was suspended in H₂O, filtered and washed with H₂O and *n*-heptane. The phases were separated, and the organic phase was concentrated under reduced pressure to give a residue which was purified by flash chromatography (gradient elution 0-30% EtOAc in cyclohexane) to afford the title compound as a light orange solid (328 mg, 36%). LCMS (ES⁺) Method 1: *m*/*z* 231 (M+H)⁺, RT 1.59 min.

### Step 2: 5-Bromo-4-chloro-2-methyl-2H-indazole

A solution of 5-bromo-4-chloro-1H-indazole (1.49 g, 6.45 mmol) in EtOAc (32 mL) at 0 °C was treated with trimethyloxonium tetrafluoroborate (1.43 g, 9.65 mmol). The resultant mixture was allowed to warm to rt and stirred for 5 h. After completion, the mixture was quenched with NaHCO₃ sat. sol. and extracted with EtOAc. The organic layer was washed with brine, dried over Na₂SO₄, filtered, and concentrated *in vacuo.* The crude product was purified by flash chromatography (gradient elution 0-30% EtOAc in *n-*heptane) to afford the title compound as an orange solid (0.9 g, 57%). ¹H NMR (500 MHz, DMSO-*d₆*): δ 8.49 (s, 1H), 7.52 (d, *J*=9.0 Hz, 1H), 7.44 (d, *J*=9.0 Hz, 1H), 4.16 (s, 3H). LCMS (ES⁺) Method 1: *m*/*z* 245 (M+H)⁺, RT 1.69 min.

### Step 3: 2-ethylhexyl 3-((4-chloro-2-methyl-2H-indazol-5-yl)thio)propanoate

A solution of 2-ethylhexyl 3-mercaptopropanoate (139 uL, 0.61 mmol), 5-bromo-4-chloro-2-methyl-2H-indazole (100 mg, 0.41 mmol), DIPEA (145 uL, 0.81 mmol), Xantphos (24 mg, 0.04 mmol) and Pd₂(dba)₃ (22 mg, 0.02 mmol) in 1,4-dioxane (1 mL) was irradiated in a MW apparatus at 130°C for 1.5 h. After cooling the mixture was concentrated under reduced pressure to afford a residue which was purified by flash chromatography (gradient elution 0-50% EtOAc in petroleum ether) to afford the title compound as a yellow oil (150 mg, 96%). LCMS (ES⁺) Method 1: *m*/*z* 383 (M+H)⁺, RT 2.58 min.

### Step 4: sodium 4-chloro-2-methyl-2H-indazole-5-thiolate

A solution of 2-ethylhexyl 3-((4-chloro-2-methyl-2H-indazol-5-yl)thio)propanoate (125 mg, 0.33 mmol) in MeOH (6 mL) was treated with NaOMe (25% wt in MeOH; 328 uL, 0.36 mmol) and stirred at rt for 1 h before being evaporated under reduced pressure to afford a residue which was dissolved in H₂O and washed with Et₂O. The aqueous phase was diluted with MeCN and lyophilized to afford the title compound as a yellow powder (72 mg, 99%). LCMS (ES⁺) Method 1: *m*/*z* 199 (M+H)⁺, RT 1.59 min.

### Examples

### Example 1: (S)-6-(4'-amino-3'-fluoro-4'H,6'H-spiro[piperidine-4,5'-pyrrolo[1,2-b]pyrazol]-1-yl)-3-(2,3-dichlorophenyl)-2-methylpyrimidin-4(3H)-one

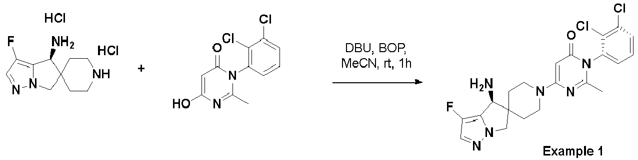

BOP (16 mg, 0.04 mmol) and DBU (0.01 mL, 0.09 mmol) were added to a suspension of **Intermediate 3** (9 mg, 0.03 mmol) and **Intermediate 4** (8 mg, 0.03 mmol) in dry MeCN (0.1 mL). The mixture was stirred at rt for 1 h before being concentrated under reduced pressure to afford a residue which purified by preparative HPLC-MS (from 15% to 100% MeCN/ H₂O + 0.1% TFA) to obtain the title compound as a white powder (3 mg, 23%). ¹H NMR (400 MHz, DMSO-*d₆*) δ 8.55 (br s, 3H), 7.81 (d, *J*=7.7 Hz, 1H), 7.62 (d, *J*=3.7 Hz, 1H), 7.58-7.51 (m, 2H), 5.51 (s, 1H), 4.67 (br s, 1H), 4.36 (br d, *J*=11.6 Hz, 1H), 4.23 (br d, *J*=11.8 Hz, 1H), 4.18-4.14 (m, 1H), 3.33-3.27 (m, 1H), 3.15-3.08 (m, 1H), 2.01 (s, 3H), 1.84-1.56 (m, 4H). LCMS (ES⁺) Method 1: *m*/*z* 463 (M+H)⁺, RT 1.21 min.

### Example 2: (S)-1-(5-(2,3-dichlorophenoxy)pyrazin-2-yl)-4'H,6'H-spiro[piperidine-4,5'-pyrrolo[1,2-b]pyrazol]-4'-amine

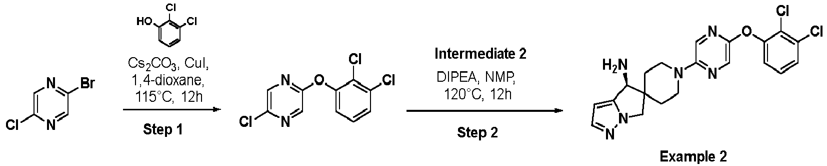

### Step 1: 2-chloro-5-(2,3-dichlorophenoxy)pyrazine

A mixture of 2-bromo-5-chloropyrazine (33 mg, 0.17 mmol), 2,3-dichlorophenol (31 mg, 0.19 mmol), Cs₂CO₃ (78 mg, 0.24 mmol), CuI (3 mg, 0.017 mmol) and 2-(dimethylamino)acetic acid (2 mg, 0.017 mmol) in 1,4-dioxane (0.9 ml) was degassed and heated at 115°C for 12 h. After cooling the reaction mixture was filtered and concentrated under reduced pressure to afford a residue which was purified by flash chromatography on silica gel (from 0% to 100% EtOAc in petroleum ether) to afford the title compound as a white powder (28 mg, 60%). LCMS (ES⁺) Method 1: *m*/*z* 275 (M+H)⁺, RT 2.34 min.

### Step 2: (S)-1-(5-(2,3-dichlorophenoxy)pyrazin-2yl)-4'H,6'H-spiro[piperidine-4,5'-pyrrolo[1,2-b]pyrazol]-4'-amine

A solution of **Intermediate 2** (32 mg, 0.12 mmol), 2-chloro-5-(2,3-dichlorophenoxy)pyrazine (28 mg, 0.1 mmol) and DIPEA (0.09 mL, 0.51 mmol) in NMP (0.5 mL) was heated at 120°C for 12 h. After cooling the reaction mixture was concentrated under reduced pressure to afford a residue which was purified by flash chromatography on silica gel (from 0% to 100% EtOAc in petroleum ether to 0% to 100% MeOH in EtOAc) to furnish a residue which was purified by preparative HPLC-MS (from 25% to 100% MeCN/ H₂O + 0.1% TFA) to obtain the title compound as a white powder (1.4 mg, 33%). ¹H NMR (400 MHz, DMSO-*d₆*) δ 8.32 (br s, 3H), 8.13 (s, 1H), 7.90 (s, 1H), 7.51 (s, 1H), 7.44 (d, *J*=8.1 Hz, 1H), 7.32 (t, *J*=8.2 Hz, 1H), 7.12 (d, *J*=8.1 Hz, 1H), 6.24 (s, 1H), 4.41-4.36 (m, 1H), 4.29 (br d, *J*=11.4 Hz, 1H), 4.15 (br d, *J*=11.2 Hz, 1H), 4.11-4.01 (m, 1H), 3.18-3.12 (m, 1H), 3.03-2.97 (m, 1H), 1.81-1.54 (m, 5H). LCMS (ES⁺) Method 1: *m*/*z* 431 (M+H)⁺, RT 1.54 min.

### Example 3: (S)-6-(4'-amino-4'H,6'H-spiro[piperidine-4,5'-pyrrolo[1,2-b]pyrazol]-1-yl)-3-(2,3-dichlorophenyl)-2,5-dimethylpyrimidin-4(3H)-one

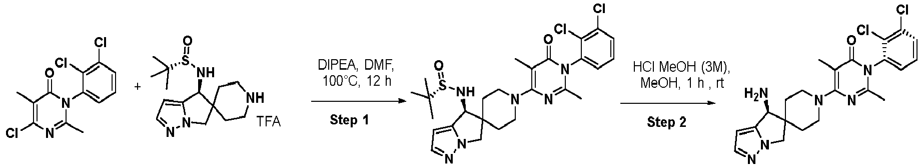

### Step 1: (S)-N-((S)-1-(1-(2,3-dichlorophenyl)-2,5-dimethyl-6-oxo-1,6-dihydropyrimidin-4yl)-4'H,6'H spiro[piperidine-4,5'-pyrrolo[1,2-b]pyrazol)-4'-yl)-2-methylpropane-2-sulfinamide

A solution of **Intermediate 5** (90 mg, 0.3 mmol), **Intermediate 1** (182 mg, 0.35 mmol) and DIPEA (0.3 mL, 1.77 mmol) in DMF (1 mL) was heated at 100°C for 12 h. The reaction mixture was filtered on a pad of celite, washed with EtOAc and the solvent was concentrated under reduced pressure. The residue was used in the next step without further purification (105 mg). LCMS (ES⁺) Method 1: *m*/*z* 579 (M+H)⁺, RT 1.90 min.

### Step 2: (S)-6-(4'-amino-4H,6'H-spiro[piperidine-4,5'-pyrrolo[1,2-b]pyrazol]-1-yl)-3-(2,3-dichlorophenyl)-2,5-dimethylpyrimidin-4(3H)-one

A solution of (*S*)-*N*-((*S*)-1-(1-(2,3-dichlorophenyl)-2,5-dimethyl-6-oxo-1,6-dhhydropyrimidin-4-yl)-4'H,6'H-spiro[piperidine-4,5'-pyrrolo[1,2-b]pyrazol]-4'-yl)-2-methylpropane-2-sulfinamide in MeOH (1 mL) was treated with HCl in MeOH (3M, 0.5 mL) for 1 h. The solvent was evaporated under reduced pressure and the residue was directly purified by reverse phase chromatography (from 15% to 35% MeCN/H₂O + 0.1% TFA) to afford the title compound as white solid (35 mg, 25%). The above mixture of atropoisomers was separated by chiral SFC on a SFC^{™} Prep 15 Waters using a Chiralpak^{®} IA (1×25 cm) column (flow: 10 ml/min, T_{col} 40 °C, P_{col}: 120 bar, modifier: 30% MeOH (+0.1% TEA) 3 min, 30-35% 6 min, 35-40% 11 min, 40-30% 2 min, 30% 2 min; using CO₂ as supercritical eluent); the title compound was obtained as a second eluted (RT= 14.9 min) as a white powder (5.8 mg; 18%). ¹H NMR (400 MHz DMSO-*d₆*) δ 7.81 (t, *J*=5.2 Hz, 1H), 7.55 (d, *J*=4.6 Hz, 2H), 7.40 (s, 1H), 6.05 (br s, 1H), 4.15 (br d, *J*=11.0 Hz, 1H), 3.99-3.94 (m, 2H), 3.77-3.69 (m, 2H), 3.20-3.14 (m, 2H), 1.99 (s, 3H), 1.98 (m, 1H), 1.92 (s, 3H), 1.92-1.90 (m, 2H), 1.75-1.61 (m, 3H), 1.52-1.48 (m, 1H). LCMS (ES⁺) Method 1: *m*/*z* 459 (M+H)⁺, RT 1.09 min.

### Example 4: (S)-1-(5-((3-fluoro-2-methylpyridin-4-yl)thio)-6-methylpyrazin-2-yl)-4'H,6'H-spiro[piperidine-4,5'-pyrrolo[1,2-b]pyrazol]-4'-amine

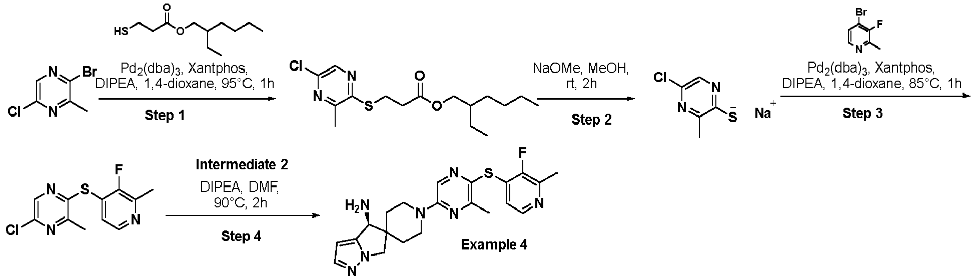

### Step 1: 2-ethylhexyl 3-((5-chloro-3-methylpyrazin-2-yl)thio)propanoate

A suspension of Pd₂(dba)₃ (11 mg, 0.01 mmol), 2-bromo-5-chloro-3-methylpyrazine (100 mg, 0.48 mmol), 2-ethylhexyl 3-mercaptopropanoate (0.12 mL, 0.53 mmol), Xantphos (13.9 mg, 0.024 mmol) and DIPEA (0.17 mL, 0.96 mmol) in 1,4-dioxane (2.4 mL) was degassed and stirred at 95°C for 1 h before being cooled and filtered on a pad of celite which was washed with EtOAc. The solvent was concentrated under reduced pressure to afford a residue which was purified by flash chromatography on silica gel (from 0% to 100% EtOAc in petroleum ether) to furnish the title compound (166 mg, 99%). LCMS (ES⁺) Method 1: *m*/*z* 345 (M+H)⁺, RT 2.50 min.

### Step 2: sodium 5-chloro-3-methylpyrazine-2-thiolate

NaOMe (20% solution w/w in MeOH; 0.58 mL, 0.58 mmol) was added to a solution of 2-ethylhexyl 3-((5-chloro-3-methylpyrazin-2-yl)thio)propanoate (166 mg, 0.48 mmol) in MeOH (1.4 mL) and the mixture was stirred at rt for 2 h before being evaporated under reduced pressure. A mixture of Et₂O and H₂O (20 mL) was added to the residue and the aqueous phase was separated and lyophilized to afford the title compound as a yellow powder (87 mg, 99%). LCMS (ES⁺) Method 1: *m*/*z* 161 (M+H)⁺, RT 1.08 min.

### Step 3: 5-chloro-2-((3-fluoro-2-methylpyridin-4-yl)thio)-3-methylpyrazine

A suspension of 4-bromo-3-fluoro-2-methylpyridine (25 mg, 0.13 mmol), sodium 5-chloro-3-methylpyrazine-2-thiolate (48 mg, 0.13 mmol), Pd₂(dba)₃ (3 mg, 0.03 mmol), Xantphos (4 mg, 0.01 mmol) and DIPEA (0.05 mL, 0.26 mmol) in 1,4-dioxane (0.7 mL) was degassed and heated at 85°C for 1 h before being cooled and filtered on a pad of solka floc which was washed with EtOAc/MeOH. The solvent was concentrated under reduced pressure to afford a residue which was purified by flash chromatography on silica gel (from 0% to 100% MeOH in EtOAc) to furnish the title compound (12 mg, 34%). LCMS (ES⁺) Method 1: *m*/*z* 270 (M+H)⁺, RT 1.98 min.

### Step 4: (S)-1-(5-((3-fluoro-2-methylpyridin-4-yl)thio)-6-methylpyrazin-2-yl)-4'H,6'H-spiro[piperidine-4,5'-pyrrolo[1,2-b]pyrazol]-4'-amine

A solution of **Intermediate 2** (15 mg, 0.04 mmol), 5-chloro-2-((3-fluoro-2-methylpyridin-4-yl)thio)-3-methylpyrazine (12 mg, 0.04 mmol) and DIPEA (0.04 mL, 0.22 mmol) in DMF (0.2 mL) was heated at 90°C for 2 h. After cooling the reaction mixture was concentrated under reduced pressure to afford a residue which was purified by preparative HPLC-MS (from 15% to 100% MeCN/ H₂O + 0.1% TFA) to obtain the title compound as a yellow powder (4.5 mg, 24%). ¹H NMR (400 MHz, DMSO-*d₆*) δ 8.40 (br s, 3H), 8.35 (s, 1H), 8.09 (d, *J*=5.0 Hz, 1H), 7.60 (s, 1H), 6.64 (t, *J*=5.6 Hz, 1H), 6.32 (s, 1H), 4.48-4.24 (m, 5H), 3.37-3.29 (m, 1H), 3.24-3.16 (m, 1H), 2.45 (s, 3H), 2.43 (s, 3H), 1.85-1.66 (m, 4H). LCMS (ES⁺) Method 1: *m*/*z* 426 (M+H)⁺, RT 1.20 min.

The following examples were synthesized using the above procedure with the corresponding starting materials: **Example 6, Example 17** (starting from 5-bromo-2-chloro-3-methylpyrazine in step 1)

| **Example #** | **¹H NMR (400 MHz, DMSO-*d₆*)** |
|---|---|
| 6 | δ 8.24 (br s, 3H), 8.16 (s, 1H), 7.44 (s, 1H), 7.34 (d, *J*=8.1 Hz, 1H), 7.12-7.08 (m, 1H), 6.64 (d, *J*=7.9 Hz, 1H), 6.16 (s, 1H), 4.33-4.08 (m, 5H), 3.17-3.12 (m, 1H), 3.03-2.98 (m, 1H), 2.26 (s, 3H), 1.69-1.49 (m, 4H). |
| 17 | δ 8.42 (br s, 3H), 8.20 (s, 1H), 7.59-7.56 (m, 2H), 7.31 (d, *J*=8.0 Hz, 1H), 7.09 (d, *J*=7.9 Hz, 1H), 6.32 (s, 1H), 4.51 (br s, 1H), 4.36 (br d, *J*=11.2 Hz, 1H), 4.21 (br d, *J*=11.4 Hz, 1H), 3.72 (br d, *J*=13.4 Hz, 1H), 3.65-3.61 (m, 1H), 3.21-3.15 (m, 1H), 3.04-2.98 (m, 1H), 2.48 (s, 3H), 2.03-1.67 (m, 4H). |

### Example 5: (S)-2-(4'-amino-4'H,6'H-spiro[piperidine-4,5'-pyrrolo[1,2-b]pyrazol]-1-yl)-5-(2,3-difluorophenyl)-3-methylpyrimidin-4(3H)-one

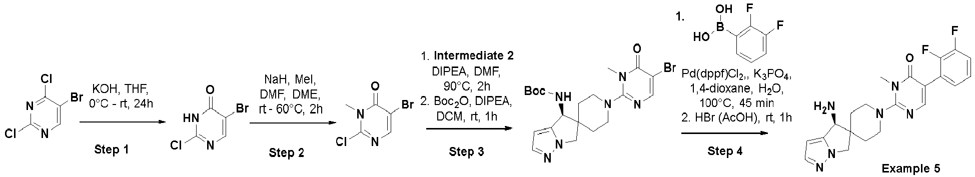

### Step 1: 5-bromo-2-chloropyrimidin-4(3H)-one

KOH (2N; 2.4 mL, 4.8 mmol) was added to a solution of 5-bromo-2,4-dichloro-pyrimidine (545 mg, 2.39 mmol) in THF (8 mL) at 0°C. The mixture was allowed to warm up to rt and stirred at this temperature for 24 h before being quenched with HCl (6N) to pH=1 at 0°C and then extracted with EtOAc (2x). The organic layers were combined, dried over Na₂SO₄, filtered and the solvent removed under reduced pressure to afford the title compound as a brown solid (480 mg, 95%) which was used without further purification. LCMS (ES⁺) Method 1: *m*/*z* 209 (M+H)⁺, RT 0.81 min.

### Step 2: 5-bromo-2-chloro-3-methylpyrimidin-4(3H)-one

NaH (60% wt in mineral oil; 101 mg, 2.5 mmol) was added to a solution of 5-bromo-2-chloropyrimidin-4(3H)-one (480 mg, 2.29 mmol) in dry DME (6 mL)/DMF (1.5 mL) at 0°C. The mixture was allowed to warm up to rt, treated with MeI (0.29 mL, 4.58 mmol) and heated at 60°C for 2 h. After cooling the reaction mixture was diluted with brine and EtOAc. The organic layer was dried over Na₂SO₄, filtered and the solvent removed under reduced pressure to afford a residue which was purified by flash chromatography on silica gel (from 0% to 40% EtOAc in petroleum ether) to afford the title compound as an off-white powder (300 mg, 58%). LCMS (ES⁺) Method 1: *m*/*z* 223 (M+H)⁺, RT 1.01 min.

### Step 3: tert-butyl (S)-(1-(5-bromo-1-methyl-6-oxo-1,6-dihydropyrimidin-2-yl)-4'H,6'H-spiro[piperidine-4,5'-pyrrolo[1,2-b]pyrazol]-4'-yl)carbamate

A solution of **Intermediate 2** (32 mg, 0.11 mmol), 5-bromo-2-chloro-3-methylpyrimidin-4(3H)-one (20 mg, 0.09 mmol) and DIPEA (0.12 mL, 0.72 mmol) in DMF (0.45 mL) was heated at 90°C for 2 h. After cooling the solvent was concentrated under reduced pressure and the residue was dissolved in DCM (0.4 mL) and treated with Boc₂O (39 mg, 0.18 mmol) and DIPEA (23 mg, 0.18 mmol) and stirred at rt for 1 h. The solvent was concentrated under reduced pressure to afford a residue which was purified by flash chromatography on silica gel (from 0% to 100% EtOAc +10% MeOH in petroleum ether) to furnish the title compound as a pale-yellow oil (16 mg, 37%). LCMS (ES⁺) Method 1: *m*/*z* 479 (M+H)⁺, RT 1.66 min.

### Step 4: (S)-2-(4'-amino-4'H,6H-spiro[piperidine-4,5'-pyrrolo[1,2-b]pyrazol]-1-yl)-5-(2,3-difluorophenyl)-3-methylpyrimidin-4(3H)-one

A suspension of *tert*-butyl (*S*)-(1-(5-bromo-1-methyl-6-oxo-1,6-dihydropyrimidin-2-yl)-4'H,6'H-spiro[piperidine-4,5'-pyrrolo[1,2-b]pyrazol]-4'-yl)carbamate (16 mg, 0.03 mmol), (2,3-difluorophenyl)boronic acid (10.5 mg, 0.07 mmol), Pd(dppf)Cl₂ (4.9 mg, 0.01 mmol) and K₃PO₄ (17.7 mg, 0.08 mmol) in a mixture of 1,4-dioxane (0.12 mL) and H₂O (0.06 mL) was degassed and stirred at 100°C for 45 min before being cooled and concentrated under reduced pressure. The residue was treated with HBr (33% in AcOH; 0.3 mL) and stirred at rt for 1 h. The mixture was concentrated under reduced pressure to afford a residue which purified by preparative HPLC-MS (from 10% to 100% MeCN/H₂O +0.1% TFA) to obtain the title compound as a white powder (0.4 mg, 2.8%). LCMS (ES⁺) Method 1: *m*/*z* 413 (M+H)⁺, RT 0.97 min.

### Example 7: (S)-1-(5-(4-fluorophenoxy)pyrazin-2-yl)-4'H,6'H-spiro[piperidine-4,5'-pyrrolo[1,2-b]pyrazol]-4'-amine

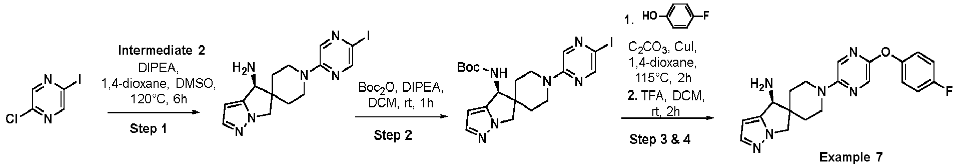

### Step 1: (S)-1-(5-iodopyrazin-2-yl)-4'H,6'H-spiro[piperidine-4,5'-pyrrolo[1,2-b]pyrazol]-4'-amine

A solution of **Intermediate 2** (79 mg, 0.3 mmol), 2-chloro-5-((3-fluoro-2-methylpyridin-4-yl)thio)pyrazine (60 mg, 0.25 mmol) and DIPEA (0.22 mL, 1.25 mmol) in a mixture of 1,4-dioxane (0.5 mL) and DMSO (0.3 mL) was heated at 120°C for 6 h. After cooling the reaction mixture was evaporated under reduced pressure to afford the title compound which was used as a crude in the next step. LCMS (ES⁺) Method 1: *m*/*z* 397 (M+H)⁺, RT 1.06 min.

### Step 2: tert-butyl (S)-(1-(5-iodopyrazin-2-yl)-4'H,6'H-spiro[piperidine-4,5'-pyrrolo[1,2-b]pyrazol]-4'-yl)carbamate

A solution of (*S*)-1-(5-iodopyrazin-2-yl)-4'H,6'H-spiro[piperidine-4,5'-pyrrolo[1,2-b]pyrazol]-4'-amine (80 mg, 0.2 mmol) in DCM (1 mL) was treated with Boc₂O (132 mg, 0.61 mmol) and DIPEA (209 mg, 1.61 mmol) and stirred at rt for 1 h. The solvent was removed under reduced pressure to afford a residue which was purified by flash chromatography on silica gel (from 0% to 100% EtOAc in petroleum ether) to afford the title compound (17 mg, 11%). LCMS (ES⁺) Method 1: *m*/*z* 497 (M+H)⁺, RT 2.22 min.

### Step 3: tert-butyl (S)-(1-(5-(4-fluorophenoxy)pyrazin-2-yl)-4'H,6'H-spiro[piperidine-4,5'-pyrrolo[1,2-b]pyrazol]-4'-yl)carbamate

A mixture of *tert*-butyl (*S*)-(1-(5-iodopyrazin-2-yl)-4'H,6'H-spiro[piperidine-4,5'-pyrrolo[1,2-b]pyrazol]-4'-yl)carbamate (17 mg, 0.03 mmol), 4-fluorophenol (4.2 mg, 0.04 mmol), Cs₂CO₃ (15.6 mg, 0.05 mmol), CuI (0.7 mg, 0.003 mmol) and 2-(dimethylamino)acetic acid (0.4 mg, 0.003 mmol) in 1,4-dioxane (0.17 ml) was degassed and heated at 115°C for 2 h. After cooling the reaction mixture was filtered on a pad of solka floc and concentrated under reduce pressure to afford a residue which was purified by flash chromatography on silica gel (from 0% to 100% EtOAc in petroleum ether) to afford the title compound as a white powder (16 mg, 97%). LCMS (ES⁺) Method 1: *m*/*z* 481 (M+H)⁺, RT 2.37 min.

### Step 4: (S)-1-(5-(4-fluorophenoxy)pyrazin-2-yl)-4'H,6'H-spiro[piperidine-4,5'-pyrrolo[1,2-b]pyrazol]-4'-amine

A solution of *tert*-butyl (*S*)-(1-(5-(4-fluorophenoxy)pyrazin-2-yl)-4'H,6'H-spiro[piperidine-4,5'-pyrrolo[1,2-b]pyrazol]-4'-yl)carbamate (16 mg, 0.03 mmol) in DCM (0.5 mL) was treated with TFA (0.2 mL) and stirred at rt for 2 h. The solvent was removed under reduced pressure to afford a residue which purified by preparative HPLC-MS (from 13% to 100% MeCN/ H₂O + 0.1% TFA) to obtain the title compound as a white powder (0.8 mg, 6%). ¹H NMR (400 MHz, DMSO-*d₆*) δ 8.24 (br s, 3H), 8.09 (s, 1H), 8.01(s, 1H), 7.58 (s, 1H), 7.22 (t, *J*=8.7 Hz, 2H), 7.12-7.08 (m, 2H), 6.30 (s, 1H), 4.43 (s, 1H), 4.34 (d, *J*=11.4 Hz, 1H), 4.23-4.08 (m, 3H), 3.24-3.19 (m, 1H), 3.10-3.05 (m, 1H), 1.91-1.75 (m, 3H), 1.65-1.62 (m, 1H). LCMS (ES⁺) Method 1: *m*/*z* 381 (M+H)⁺, RT 1.13 min.

### Example 8: (S)-1-(6-(2,3-difluorophenyl)pyrido[2,3-b]pyrazin-2-yl)-4'H,6'H-spiro[piperidine-4,5'-pyrrolo[1,2-b]pyrazol]-4'-amine

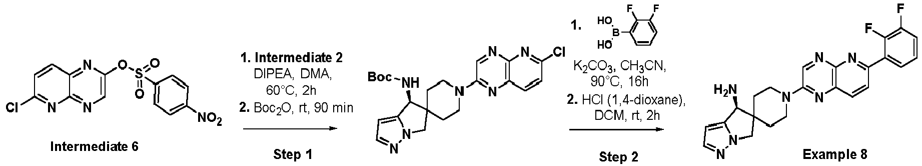

### Step 1: tert-butyl (S)-(1-(6-chloropyrido[2,3-b]pyrazin-2-yl)-4'H,6'H-spiro[piperidine-4,5'-pyrrolo[1,2-b]pyrazol]-4'-yl)carbamate

A solution of **Intermediate 6** (100 mg, 0.27 mmol) and **Intermediate 2** (90 mg, 0.3 mmol) in DMA (2 mL) was treated with DIPEA (0.24 mL, 1.36 mmol) and heated at 60 °C for 2 h before being cooled to rt and treated with Boc₂O (119 mg, 0.55 mmol) and stirred for 90 min. The mixture was then diluted with EtOAc and washed with H₂O, brine, and dried over Na₂SO₄ and the solvent was removed under reduced pressure to afford a residue which was purified by flash chromatography on silica gel (from 0% to 7.5% MeOH in DCM) to afford the title compound as a yellow oil (47 mg, 38%). LCMS (ES⁺) Method 1: *m*/*z* 456 (M+H)⁺, RT 1.83 min.

### Step 2: (S)-1-(6-(2,3-difluorophenyl)pyrido[2,3-b]pyrazin-2-yl)-4'H,6'H-spiro[piperidine-4,5'-pyrrolo[1,2-b]pyrazol]-4'-amine

A mixture of *tert*-butyl (*S*)-(1-(6-chloropyrido[2,3-b]pyrazin-2-yl)-4'H,6'H-spiro[piperidine-4,5'-pyrrolo[1,2-b]pyrazol]-4'-yl)carbamate (23 mg, 0.05 mmol), (2,3-difluorophenyl)boronic acid (16 mg, 0.1 mmol), Pd(dppf)Cl₂ (8.3 mg, 0.01 mmol) and K₂CO₃ (28 mg, 0.2 mmol) in MeCN (0.5 mL) was heated at 90 °C for 16 h before being cooled, diluted with EtOAc, filtered and evaporated under reduced pressure to afford a residue which was purified by flash chromatography on silica gel (from 0% to 10% MeOH in DCM) to afford the Boc-protected intermediate (10 mg). The material was dissolved in DCM (1 mL) and treated with HCl (4N in 1,4-dioxane; 0.13 mL) and stirred at rt for 2 h. Volatiles were removed in *vacuo* and the residue was purified by preparative HPLC-MS (from 15% to 100% MeCN/H₂O + 0.1% TFA) to obtain the title compound as a yellow powder (5.5 mg, 25%). ¹H NMR (400 MHz, DMSO-*d₆*) δ 9.09 (s, 1H), 8.34 (br s, 3H), 8.08 (d, *J*=8.6 Hz, 1H), 7.99-7.97 (m, 1H), 7.77 (br t, *J*=7.3 Hz, 1H), 7.53 (s, 1H), 7.51-7.47 (m, 1H), 7.35-7.30 (m, 1H), 6.25 (s, 1H), 4.57 (br d, *J*=13.8 Hz, 1H), 4.48 (d, *J*=14.3 Hz, 1H), 4.42 (br s, 1H), 4.35 (br d, *J*=11.4 Hz, 1H), 4.23 (br d, *J*=11.4 Hz, 1H), 3.44-3.25 (m, 2H), 1.87-1.66 (m, 4H). LCMS (ES⁺) Method 1: *m*/*z* 434 (M+H)⁺, RT 1.19 min.

The following examples were synthesized using the above procedure with the corresponding starting materials: **Example 9**

| **Example #** | **¹H NMR (400 MHz, DMSO-*d₆*)** |
|---|---|
| 9 | δ 9.07 (s, 1H), 8.33 (br s, 3H), 8.06 (d, *J*=8.6 Hz, 1H), 7.82 (d, *J*=8.6 Hz, 1H), 7.71 (d, *J*=7.7 Hz, 1H), 7.56-7.53 (m, 2H), 7.48-7.44 (m, 1H), 6.24 (s, 1H), 4.57 (br d, *J*=14.5 Hz, 1H), 4.48 (d, *J*=14.0 Hz, 1H), 4.41 (br s, 1H), 4.36-4.33 (m, 1H), 4.26-4.22 (m, 1H), 3.44-3.25 (m, 2H), 1.86-1.65 (m, 4H). |

### Example 10: (S)-1-(5-((2-chloro-3-(1H-pyrazol-1-yl)phenyl)thio)pyrazin-2-yl)-4'H,6'H-spiro[piperidine-4,5'-pyrrolo[1,2-b]pyrazol]-4'-amine

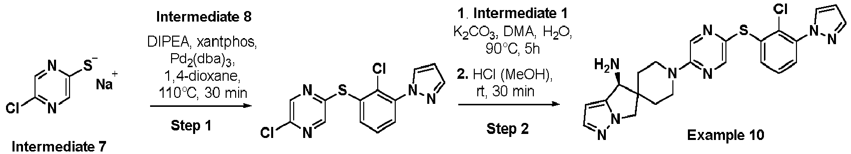

### Step 1: 2-chloro-5-((2-chloro-3-(1H-pyrazol-1-yl)phenyl)thio)pyrazine

A solution of **Intermediate 7** (37 mg, 0.07 mmol), **Intermediate 8** (20 mg, 0.07 mmol), Pd₂(dba)₃ (2.4 mg, 0.003 mmol), Xantphos (3 mg, 0.01 mmol) and DIPEA (0.04 mL, 0.21 mmol) in 1,4-dioxane (0.4 mL) was degassed and heated at 110°C for 30 min. After cooling the solvent was removed under reduced pressure to afford a residue which was purified by flash chromatography on silica gel (from 0% to 100% EtOAc in petroleum ether) to furnish the title compound as a brown oil (21 mg, 99%). LCMS (ES⁺) Method 1: *m*/*z* 323 (M+H)⁺, RT 1.95 min.

### Step 2: (S)-1-(5-((2-chloro-3-(1H-pyrazol-1-yl)phenyl)thio)pyrazin-2-yl)-4'H,6'H-spiro[piperidine-4,5'-pyrrolo[1,2-b]pyrazol]-4'-amine

A solution of **Intermediate 1** (40 mg, 0.08 mmol) and K₂CO₃ (45 mg, 0.32 mmol) in a mixture of DMA (0.5 mL) / H₂O (0.5 mL) was heated at 90°C for 5 h. After cooling the reaction mixture was filtered and the residue washed with MeOH then the solvent was concentrated under reduced pressure to get a residue which was treated with HCl (3N in MeOH; 0.4 mL). The mixture was stirred for 30 min at rt then the solvent was concentrated under reduced pressure to get a residue which purified by preparative HPLC-MS (from 15% to 100% MeCN / H₂O + 0.1% TFA) to obtain the title compound as a pale-yellow powder (4.3 mg, 14%). ¹H NMR (400 MHz, DMSO-*d₆*) δ 8.55 (s, 1H), 8.41 (br s, 3H), 8.36 (s, 1H), 8.14 (s, 1H), 7.77 (s, 1H), 7.60 (s, 1H), 7.41-7.39 (m, 2H), 7.02 (dd, *J₁*=6.5 Hz, *J₂*=2.5 Hz, 1H), 6.55 (s, 1H), 6.32 (s, 1H), 4.49-4.25 (m, 5H), 3.40-3.37 (m, 1H), 3.25-3.18 (m, 1H), 1.85-1.67 (m, 4H). LCMS (ES⁺) Method 1: *m*/*z* 479 (M+H)⁺, RT 1.24 min.

### Example 11: (S)-6-(4'-amino-4'H,6'H-spiro[piperidine-4,5'-pyrrolo[1,2-b]pyrazol]-1-yl)-3-(2,3-dichlorophenyl)-1-methylpyridin-2(1H)-one

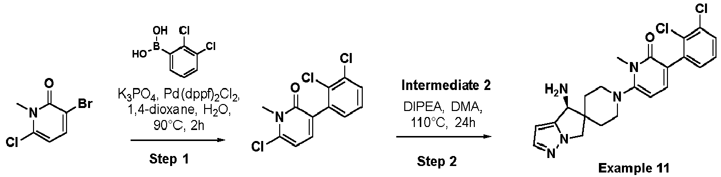

### Step 1: 6-chloro-3-(2,3-dichlorophenyl)-1-methylpyridin-2(1H)-one

A solution of 3-bromo-6-chloro-1-methylpyridin-2(1H)-one (20 mg, 0.09 mmol), (2,3-dichlorophenyl)boronic acid (34.3 mg, 0.18 mmol), Pd(dppf)Cl₂ (14.7 mg, 0.02 mmol) and K₃PO₄ (114.4 mg, 0.54 mmol) in a mixture of 1,4-dioxane (0.5 mL)/H₂O (0.1 mL) was degassed and heated at 90°C for 2 h. After cooling the reaction mixture was filtered on a pad of solka floc and washed with EtOAc. The solvent was concentrated under reduced pressure to afford a residue which was purified by flash chromatography on silica gel (from 0% to 100% EtOAc in petroleum ether) to afford the title compound as a yellow solid (16 mg, 62%). LCMS (ES⁺) Method 1: *m*/*z* 288 (M+H)⁺, RT 1.87 min.

### Step 2: (S)-6-(4'-amino-4'H,6'H-spiro[piperidine-4,5'-pyrrolo[1,2-b]pyrazol]-1-yl)-3-(2,3-dichlorophenyl)-1-methylpyridin-2(1H)-one

A solution of 6-chloro-3-(2,3-dichlorophenyl)-1-methylpyridin-2(1H)-one (16 mg, 0.07 mmol), **Intermediate 2** (26 mg, 0.09 mmol) and DIPEA (0.05 mL, 0.29 mmol) in DMA (0.45 mL) was heated at 110°C for 24 h before being concentrated under reduced pressure to get a residue which purified by preparative HPLC-MS (from 15% to 100% MeCN/ H₂O + 0.1% TFA) to obtain the title compound as a brown powder (6.7 mg, 21%). ¹H NMR (400 MHz, DMSO-*d₆*) δ 8.50 (br s, 3H), 7.62-7.60 (m, 2H), 7.45 (d, *J*=7.5 Hz, 1H), 7.37 (t, *J*=7.8 Hz, 1H), 7.28 (d, *J*=7.7 Hz, 1H), 6.33 (s, 1H), 5.99 (d, *J*=7.7 Hz, 1H), 4.55 (br s, 1H), 4.34 (br d, *J*=11.2 Hz, 1H), 4.21 (d, *J*=11.4 Hz, 1H), 3.50 (s, 3H), 3.30-3.28 (m, 1H), 3.22-3.19 (m, 1H), 3.00-2.95 (m, 1H), 2.86-2.80 (m, 1H), 2.07-1.85 (m, 3H), 1.74-1.71 (m, 1H). LCMS (ES⁺) Method *1: m*/*z* 444 (M+H)⁺, RT 1.16 min.

### Example 12: (S)-1-(3-(2-fluoro-3-methylphenyl)imidazo[1,5-a]pyrazin-8-yl)-4'H,6'H-spiro[piperidine-4,5'-pyrrolo[1,2-b]pyrazol]-4'-amine

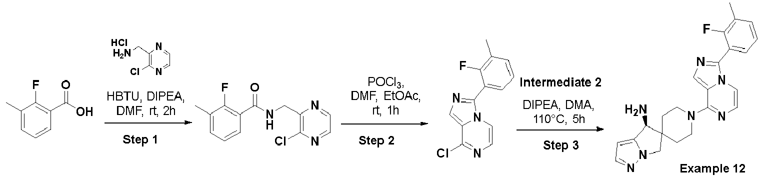

### Step 1: N-((3-chloropyrazin-2-yl)methyl)-2-fluoro-3-methylbenzamide

A solution of (3-chloropyrazin-2-yl)methanamine hydrochloride (46 mg, 0.25 mmol), 2-fluoro-3-methylbenzoic acid (30 mg, 0.2 mmol), DIPEA (0.14 mL, 0.78 mmol) and HBTU (89 mg, 0.24 mmol) in DMF (0.7 mL) was stirred at rt for 2 h then the solvent was evaporated under reduced pressure and the residue was dissolved in EtOAc and washed with HCl (1N), NaHCO₃ (sat. sol.) and brine. The organic phase was dried over Na₂SO₄ and concentrated under reduced pressure to give the title compound as a white solid (54 mg, 99%) which was used in the next step without further purification. LCMS (ES⁺) Method 1: *m*/*z* 280 (M+H)⁺, RT 1.52 min.

### Step 2: 8-chloro-3-(2-fluoro-3-methylphenyl)imidazo[1,5-a]pyrazine

A suspension of *N*-((3-chloropyrazin-2-yl)methyl)-2-fluoro-3-methylbenzamide (48 mg, 0.17 mmol) in a mixture of DMF (0.28 mL) and EtOAc (0.58 mL) was treated with POCl₃ (0.08 mL, 0.85 mmol) and stirred at rt for 1 h before being concentrated under reduced pressure. The residue was diluted with EtOAc (3 mL) and ice H₂O (1 mL) and washed with saturated NaHCO₃ aq. sol. The organic phase was isolated, dried over Na₂SO₄, and concentrated *in vacuo* to give the title compound as a white powder (44 mg, 99%) which was used in the next step without further purification. LCMS (ES⁺) Method 1: *m*/*z* 262 (M+H)⁺, RT 1.75 min.

### Step 3: (S)-1-(3-(2-fluoro-3-methylphenyl)imidazo[1,5-a]pyrazin-8-yl)-4'H,6'H-spiro[piperidine-4,5'-pyrrolo[1,2-b]pyrazol]-4'-amine

A solution of 8-chloro-3-(2-fluoro-3-methylphenyl)imidazo[1,5-a]pyrazine (15 mg, 0.06 mmol), **Intermediate 2** (21 mg, 0.07 mmol) and DIPEA (0.03 mL, 0.17 mmol) in DMA (0.4 mL) was stirred at 110°C for 5 h before being cooled at rt and concentrated under reduced pressure to get a residue which purified by preparative HPLC-MS (from 15% to 100% MeCN/ H₂O + 0.1% TFA) to obtain the title compound as a white powder (18 mg, 75%). ¹H NMR (400 MHz, DMSO-*d₆*) δ 8.42 (br s, 3H), 8.11 (s, 1H), 7.60 (s, 1H), 7.55-7.47 (m, 2H), 7.39-7.37 (m, 1H), 7.32 (t, *J*=7.2 Hz, 1H), 7.17 (d, *J*=5.0 Hz, 1H), 6.33 (s, 1H), 4.54-4.43 (m, 4H), 4.29 (d, *J*=11.4 Hz, 1H), 3.58-3.52 (m, 1H), 3.42-3.36 (m, 1H), 2.36 (s, 3H), 2.00-1.86 (m, 3H), 1.73-1.69 (m, 1H). LCMS (ES⁺) Method 1: *m*/*z* 418 (M+H)⁺, RT 0.77 min.

The following examples were synthesized using the above procedure with the corresponding starting materials: **Example 14, Example 15, Example 16**

| **Example** # | **¹H NMR (400 MHz, DMSO-*d₆*)** |
|---|---|
| 14 | δ 8.42 (br s, 3H), 8.12 (s, 1H), 7.83 (t, *J*=7.5 Hz, 1H), 7.67 (t, *J* =6.7 Hz, 1H), 7.60 (s, 1H), 7.50-7.44 (m, 2H), 7.21 (d, *J*=4.8 Hz, 1H), 6.32 (s, 1H), 4.55-4.43 (m, 4H), 4.29 (d, *J*=11.2 Hz, 1H), 3.56-3.50 (m, 1H), 3.41-3.34 (m, 1H), 1.98-1.85 (m, 3H), 1.72-1.69 (m, 1H). |
| 15 | δ 8.43 (br s, 3H), 8.13 (s, 1H), 7.68 (dd, *J₁*=6.4 Hz, *J₂*=2.4 Hz, 1H), 7.60 (s, 1H), 7.44-7.42 (m, 2H), 7.24 (d, *J*=4.8 Hz, 1H), 7.12 (d, *J*=4.8 Hz, 1H), 6.33 (s, 1H), 4.55-4.43 (m, 4H), 4.29 (d, *J*=11.4 Hz, 1H), 3.60-3.53 (m, 1H), 3.44-3.40 (m, 1H), 2.16 (s, 3H), 1.99-1.87 (m, 3H), 1.74-1.70 (m, 1H). |
| 16 | δ 8.42 (br s, 3H), 8.15 (s, 1H), 7.71-7.68 (m, 1H), 7.60 (s, 1H), 7.41 (t, *J*=8.4 Hz, 1H), 7.22-7.16 (m, 2H), 6.33 (s, 1H), 4.58-4.43 (m, 4H), 4.30-4.27 (m, 1H), 3.47-3.31 (m, 2H), 2.42 (s, 3H), 1.99-1.85 (m, 3H), 1.77-1.70 (m, 1H). |

### Example 13: (S)-1-(5-((4-fluorophenyl)thio)pyrazin-2-yl)-4'H,6'H-spiro[piperidine-4,5'-pyrrolo[1,2-b]pyrazol]-4'-amine

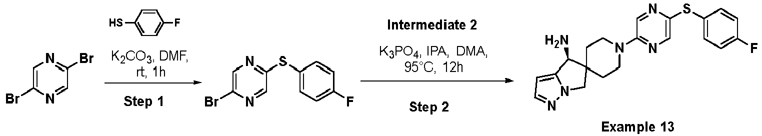

### Step 1: 2-bromo-5-((4-fluorophenyl)thio)pyrazine

A solution of 4-fluorobenzenethiol (0.01 mL, 0.08 mmol), 2,5-dibromopyrazine (20 mg, 0.08 mmol) and K₂CO₃ (12 mg, 0.08 mmol) in DMF (0.5 mL) was stirred at rt for 1 h before being diluted in DCM and H₂O. The organic phase was evaporated under reduce pressure to afford a residue which was purified by flash chromatography on silica gel (from 0% to 100% EtOAc in petroleum ether) to furnish the title compound as a yellow powder (14 mg, 58%). LCMS (ES⁺) Method 1: *m*/*z* 285 (M+H)⁺, RT 2.11 min.

### Step 2: (S)-1-(5-((4-fluorophenyl)thio)pyrazin-2-yl)-4'H,6H-spiro[piperidine-4,5'-pyrrolo[1,2-b]pyrazol]-4'-amine

A solution of **Intermediate 2** (19 mg, 0.07 mmol), 2-bromo-5-((4-fluorophenyl)thio)pyrazine (14 mg, 0.05 mmol) and K₃PO₄ (36 mg, 0.17 mmol) in a mixture of IPA (0.5 mL)/DMA (0.2 mL) was heated at 95°C for 12 h before being cooled, filtered on a pad of cellulose and washed with MeOH. The solvent was evaporated under reduced pressure to afford a residue which was purified by preparative HPLC-MS (from 25% to 100% MeCN/ H₂O + 0.1% TFA) to obtain the title compound as a yellow powder (5.6 mg, 29%). ¹H NMR (400 MHz, DMSO-*d₆*) δ 8.40 (br s, 4H), 8.18 (s, 1H), 7.59 (s, 1H), 7.40 (dd, *J₁*=8.6 Hz, *J₂*=5.5 Hz, 2H), 7.22 (t, *J*=8.9 Hz, 2H), 6.31 (s, 1H), 4.46 (br s, 1H), 4.38-4.30 (m, 2H), 4.25-4.21 (m, 2H), 3.32-3.26 (m, 1H), 3.18-3.12 (m, 1H), 1.83-1.72 (m, 3H), 1.66-1.62 (m, 1H). LCMS (ES⁺) Method 1: *m*/*z* 397 (M+H)⁺, RT 1.19 min.

### Example 18: (S)-1-(5-((4-(trifluoromethoxy)phenyl)thio)pyrazin-2-yl)-4'H,6'H-spiro[piperidine-4,5'-pyrrolo[1,2-b]pyrazol]-4'-amine

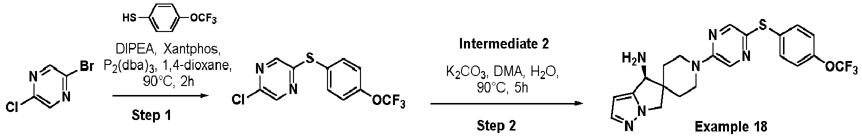

### Step 1: 2-chloro-5-((4-(trifluoromethoxy)phenyl)thio)pyrazine

A solution of 2-bromo-5-chloropyrazine (30 mg, 0.16 mmol), 4-(trifluoromethoxy)benzenethiol (30 mg, 0.16 mmol), DIPEA (0.05 mL, 0.31 mmol), Pd₂(dba)₃ (14 mg, 0.02 mmol) and Xantphos (18 mg, 0.03 mmol) in 1,4-dioxane (0.8 mL) was degassed and heated at 90 °C for 2 h before being cooled to rt, filtered and evaporated under reduce pressure to afford a residue which was purified by flash chromatography on silica gel (from 0% to 20% EtOAc in petroleum ether) to afford the title compound as a colorless oil (22 mg, 46%). LCMS (ES⁺) Method 1: *m*/*z* 307 (M+H)⁺, RT 2.32 min.

### Step 2: (S)-1-(5-((4-(trifluoromethoxy)phenyl)thio)pyrazin-2-yl)-4'H,6'H-spiro[piperidine-4,5'-pyrrolo[1,2-b]pyrazol]-4'-amine

A solution of 2-chloro-5-((4-(trifluoromethoxy)phenyl)thio)pyrazine (22 mg, 0.07 mmol), **Intermediate 2** (27 mg, 0.09 mmol) and K₂CO₃ (50 mg, 0.36 mmol) in DMA (240 uL) and H₂O (160 uL) was degassed and then heated at 90°C for 5 h. The mixture was cooled to rt, filtered, and evaporated under reduced pressure to afford a residue which was purified by preparative HPLC-MS (from 25% to 100% MeCN/ H₂O + 0.1% TFA) to obtain the title compound as a yellow powder (3.5 mg, 11%). ¹H NMR (400 MHz, DMSO-*d₆*) δ 8.44 (s, 1H), 8.38 (m, 2H), 8.26 (s, 1H), 7.58 (s, 1H), 7.40-7.34 (m, 4H), 6.30 (s, 1H), 4.44 (br s, 1H), 4.37-4.33 (m, 2H), 4.26-4.23 (m, 2H), 3.31-3.27 (m, 1H), 3.21-3.15 (m, 1H), 1.84-1.73 (m, 3H), 1.67-1.64 (m, 1H). LCMS (ES⁺) Method 1: *m*/*z* 463 (M+H)⁺, RT 1.44 min.

The following examples were synthesized using the above procedure with the corresponding starting materials: **Example 19; Example 20; Example 21; Example 22; Example 23; Example 24; Example 25; Example 26; Example 27.**

| **Example** # | **¹H NMR (400 MHz, DMSO-*d₆*)** |
|---|---|
| 19 | δ 8.50 (s, 1H), 8.44 (br s, 3H), 8.33 (s, 1H), 7.66 (d, *J*=8.3 Hz, 2H), 7.59 (s, 1H), 7.38 (d, *J*=8.1 Hz, 2H), 6.32 (s, 1H), 4.48 (br s, 1H), 4.39 (br d, *J*=11.4 Hz, 2H), 4.31-4.25 (m, 2H), 3.37-3.31 (m, 1H), 3.23-3.17 (m, 1H), 1.85-1.75 (m, 3H), 1.69-1.66 (m, 1H). |
| 20 | δ 8.30 (s, 1H), 8.22 (br s, 3H), 8.12 (s, 1H), 7.42 (s, 1H), 7.22-7.12 (m, 3H), 7.06 (d, *J*=7.5 Hz, 1H), 6.15 (s, 1H), 4.30 (br s, 1H), 4.21 (br d, *J*=11.2 Hz, 2H), 4.09 (br d, *J*=11.2 Hz, 2H), 3.23-3.18 (m, 1H), 3.05-3.00 (m, 1H), 1.71-1.58 (m, 3H), 1.52-1.48 (m, 1H). |
| 21 | δ 8.39 (s, 1H), 8.37 (br s, 3H), 8.20 (s, 1H), 7.58 (s, 1H), 7.40-7.36 (m, 1H), 7.32-7.26 (m, 2H), 7.21-7.17 (m, 1H), 6.30 (s, 1H), 4.45 (br s, 1H), 4.37-4.31 (m, 2H), 4.26-4.21 (m, 2H), 3.30-3.26 (m, 1H), 3.18-3.13 (m, 1H), 1.86-1.73 (m, 3H), 1.66-1.63 (m, 1H). |
| 22 | δ 8.50 (s, 1H), 8.43 (br s, 3H), 8.29 (s, 1H), 7.71 (s, 1H), 7.59 (s, 1H), 7.36 (br d, *J* =8.6 Hz, 1H), 7.01 (d, *J* =8.6 Hz, 1H), 6.32 (s, 1H), 4.48 (br s, 1H), 4.38 (br d, *J*=11.4 Hz, 2H), 4.30-4.25 (m, 2H), 3.34-3.30 (m, 1H), 3.22-3.17 (m, 1H), 1.88-1.76 (m, 3H), 1.69-1.66 (m, 1H). |
| 23 | δ 8.47 (s, 1H), 8.44 (br s, 3H), 8.29 (s, 1H), 7.60-7.59 (br m, 2H), 7.51 (s, 1H), 7.22 (br d, *J* =8.6 Hz, 1H), 6.31 (s, 1H), 4.47 (br s, 1H), 4.37 (br d, *J*=11.4 Hz, 2H), 4.29-4.24 (m, 2H), 3.33-3.29 (m, 1H), 3.21-3.16 (m, 1H), 1.87-1.75 (m, 3H), 1.68-1.65 (m, 1H). |
| 24 | δ 8.45 (br s, 3H), 8.42 (s, 1H), 8.20 (s, 1H), 7.59 (s, 1H), 7.37-7.25 (m, 5H), 6.31 (s, 1H), 4.46 (br s, 1H), 4.39-4.32 (m, 2H), 4.25 (br d, *J*=11.4 Hz, 2H), 3.33-3.27 (m, 1H), 3.19-3.13 (m, 1H), 1.87-1.74 (m, 3H), 1.67-1.63 (m, 1H). |
| 25 | δ 8.56 (br s, 3H), 8.50 (br s, 3H), 8.39 (s, 1H), 7.59 (br s, 1H), 7.36 (br s, 2H), 6.32 (s, 1H), 4.49-4.31 (m, 5H), 3.45-3.32 (m, 1H), 3.30-3.18 (m, 1H), 1.92-1.77 (m, 3H), 1.73-1.65 (m, 1H). |
| 26 | δ 8.51 (br s, 3H), 8.40 (s, 1H), 8.09 (s, 1H), 7.58 (s, 1H), 7.30-7.28 (m, 1H), 7.21-7.15 (m, 3H), 6.31 (s, 1H), 4.46 (br s, 1H), 4.38-4.30 (m, 2H), 4.26-4.20 (m, 2H), 3.31-3.25 (m, 1H), 3.17-3.11 (m, 1H), 2.35 (s, 3H), 1.87-1.74 (m, 3H), 1.66-1.63 (m, 1H). |
| 27 | δ 8.47 (br s, 3H), 8.39 (s, 1H), 8.07 (s, 1H), 7.58 (s, 1H), 7.38 (d, *J*=7.9 Hz, 1H), 7.30 (t, *J*=7.2 Hz, 1H), 7.20-7.13 (m, 2H), 6.31 (s, 1H), 4.46 (br s, 1H), 4.37-4.19 (m, 4H), 3.53-3.46 (m, 1H), 3.30-3.24 (m, 1H), 3.16-3.11 (m, 1H), 1.86-1.73 (m, 3H), 1.65-1.62 (m, 1H), 1.20 (d, *J*=6.8 Hz, 6H). |

### Example 28: (S)-1-(5-((2,3-dichlorophenyl)thio)pyrazin-2-yl)-1'H,3'H-spiro[piperidine-4,2'-pyrrolizin]-1'-amine

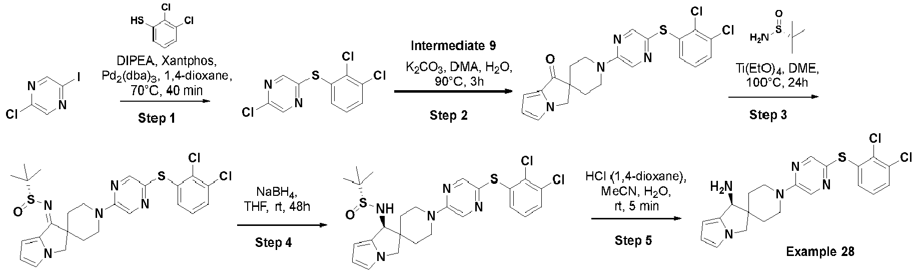

### Step 1: 2-chloro-5-((2,3-dichlorophenyl)thio)pyrazine

The title compound was prepared according to the procedure reported for the synthesis of Example 18 in step 1 starting from 2-chloro-5-iodopyrazine (180 mg, 0.6 mmol) to afford the title compound as an off-white powder (144 mg, 88%). LCMS (ES⁺) Method 1: *m*/*z* 291-293 (M+H)⁺, RT 2.31 min.

### Step 2: 1-(5-((2,3-dichlorophenyl)thio)pyrazin-2-yl)-1'H,3'H-spiro[piperidine-4,2'-pyrrolizin]-1'-one

A mixture of **Intermediate 9** (50 mg, 1.07 mmol), 2-chloro-5-((2,3-dichlorophenyl)thio)pyrazine (60 mg, 0.21 mmol) and K₂CO₃ (86 mg, 0.62 mmol) in a mixture of DMA/H₂O (6/4 v/v; 1 mL) was heated at 90°C for 3 h before being cooled to rt, diluted with EtOAc and washed with H₂O, citric acid (5% aq. sol.) and brine. The organic layer was dried over Na₂SO₄, filtered and the solvent was removed under reduced pressure to afford a residue which was purified by flash chromatography on silica gel (from 10% to 100% EtOAc in petroleum ether) to afford the title compound as an off-white powder (54 mg, 59%). LCMS (ES⁺) Method 1: *m*/*z* 445 (M+H)⁺, RT 2.34 min.

### Step 3: (R,Z)-N-(1-(5-((2,3-dichlorophenyl)thio)pyrazin-2-yl)-1'H,3H-spiro[piperidine-4,2'-pyrrolizin]-1'-ylidene)-2-methylpropane-2-sulfinamide

A solution of 1-(5-((2,3-dichlorophenyl)thio)pyrazin-2-yl)-1'H,3'H-spiro[piperidine-4,2'-pyrrolizin]-1'-one (53 mg, 0.12 mmol) and (*R*)-(-)-t-butylsulfinamide (44 mg, 0.36 mmol) in dry DME (1 mL) was treated with Ti(EtO)₄ (1 mL) and heated at 100°C for 24 h. After cooling the reaction mixture was diluted with EtOAc and H₂O. The precipitate was filtered off and the organic layer was washed with H₂O, brine, dried over Na₂SO4, filtered and the solvent removed under reduced pressure to afford a residue which was purified by flash chromatography on silica gel (from 0% to 100% EtOAc in petroleum ether) to furnish the title compound as an off-white powder (47 mg, 72%). LCMS (ES⁺) Method 1: *m*/*z* 548 (M+H)⁺, RT 2.47 min.

### Step 4: (R)-N-((S)-1-(5-((2,3-dichlorophenyl)thio)pyrazin-2-yl)-1'H,3H-spiro[piperidine-4,2'-pyrrolizin]-1'-yl)-2-methylpropane-2-sulfinamide

NaBH₄ (32 mg, 0.85 mmol) was added to a solution of (*R*,*Z*)-*N*-(1-(5-((3-chloro-2-(1H-pyrazol-1-yl)pyridin-4-yl)thio)pyrazin-2-yl)-1'H,3'H-spiro[piperidine-4,2'-pyrrolizin]-1'-ylidene)-2-methylpropane-2-sulfinamide (46 mg, 0.08 mmol) in THF (1 mL) and the reaction mixture was stirred at rt for 48 h before being quenched with H₂O at 0°C. The reaction mixture was stirred at this temperature for 20 min then treated with EtOAc and the mixture was allowed to warm up to rt. The organic layer was dried over Na₂SO₄, filtered and the solvent removed under reduced pressure to afford the title compound as a pale-yellow solid (46 mg; 99%) which was used without further purification in the next step. LCMS (ES⁺) Method 1: *m*/*z* 550 (M+H)⁺, RT 2.39 min.

### Step 5: (S)-1-(5-((2,3-dichlorophenyl)thio)pyrazin-2-yl)-1'H,3'H-spiro[piperidine-4,2'-pyrrolizin]-1'-amine

HCl (4N in 1,4 dioxane; 0.7 mL, 2.8 mmol) was added to a solution of (*R*)*-N-*((*S*)-1-(5-((2,3-dichlorophenyl)thio)pyrazin-2-yl)-1'H,3'H-spiro[piperidine-4,2'-pyrrolizin]-1'-yl)-2-methylpropane-2-sulfonamide (46 mg, 0.08 mmol) in a mixture of MeCN/H₂O (1.2/1 v/v; 2.2 mL) and the mixture was stirred at rt for 5 min before being diluted with H₂O and EtOAc and basified to pH=10 with KOH (10 N) at 0°C. The aqueous phase was extracted with EtOAc and the organic layers were combined, dried over Na₂SO₄, filtered and the solvent removed under reduced pressure to afford a residue which was purified by flash chromatography on silica gel (from 0% to 10% MeOH in EtOAc) to furnish the title compound as a yellow solid (11.5 mg; 30%). ¹H NMR(400 MHz, DMSO-*d₆*) δ 8.49 (d, *J*=1.1 Hz, 1H), 8.37-8.10 (m, 4H), 7.46 (dd, *J*=1.3, 8.0 Hz, 1H), 7.24 (t, *J*=8.0 Hz, 1H), 6.88 (dd, *J*=1.4, 8.1 Hz, 1H), 6.80 (dd, *J*=1.1, 2.5 Hz, 1H), 6.19-6.14 (m, 1H), 6.11-6.06 (m, 1H), 4.41-4.15 (m, 4H), 4.14-4.01 (m, 1H), 3.44-3.32 (m, 1H), 3.30-3.15 (m, 1H), 1.88-1.64 (m, 3H), 1.63-1.52 (m, 1H). LCMS (ES⁺) Method 1: *m*/*z* 446 (M+H)⁺, RT 1.69 min.

The following examples were synthesized using the above procedure with the corresponding starting materials: **Example 29.**

| **Example** # | **¹H NMR (400 MHz, DMSO-*d₆*)** |
|---|---|
| 29 | δ 8.49 (d, *J*=1.2 Hz, 1H), 8.45-8.18 (m, 4H), 7.47 (dd, *J*=1.3, 8.0 Hz, 1H), 7.24 (t, *J*=8.1 Hz, 1H), 6.88 (dd, *J*=1.4, 8.1 Hz, 1H), 6.71 (t, *J*=3.2 Hz, 1H), 5.98 (d, *J*=2.9 Hz, 1H), 4.58-4.45 (m, 1H), 4.30-3.97 (m, 4H), 3.43 (br t, *J*=10.7 Hz, 1H), 3.34-3.17 (m, 1H), 1.87-1.58 (m, 3H), 1.55-1.45 (m, 1H). |

### Example 30: (S)-1-(5-((2-chloro-3-fluorophenyl)thio)pyrazin-2-yl)-4'H,6'H-spiro[piperidine-4,5'-pyrrolo[1,2-b]pyrazol]-4'-amine

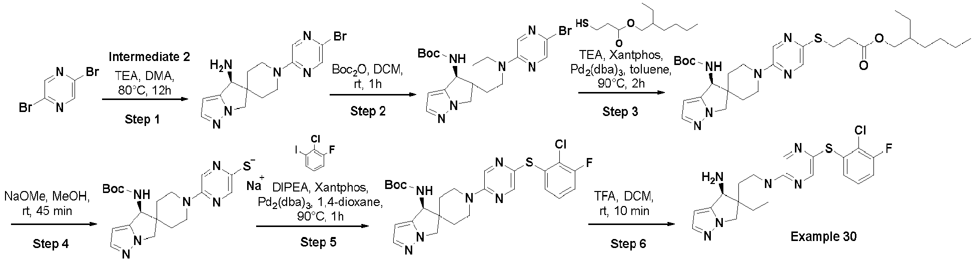

### Step 1: (S)-1-(5-bromopyrazin-2-yl)-4H,6'H-spiro[piperidine-4,5'-pyrrolo[1,2-b]pyrazol]-4'-amine

A solution of **Intermediate 2** (47 mg, 0.18 mmol), 2,5-dibromopyrazine (35 mg, 0.15 mmol) and TEA (0.1 mL, 0.74 mmol) in DMA (0.6 mL) was heated at 80°C for 12 h before being cooled to rt and diluted with EtOAc and NaOH (1N). The organic phase was washed with H₂O, dried over Na₂SO₄ and evaporated under reduced pressure to get a residue which was purified by flash chromatography on silica gel (from 0% to 5% MeOH in DCM) to furnish the title compound (24 mg, 47%). LCMS (ES⁺) Method 1: *m*/*z* 349 (M+H)⁺, RT 0.83 min.

### Step 2: tert-butyl (S)-(1-(5-bromopyrazin-2-yl)-4'H,6'H-spiro[piperidine-4,5'-pyrrolo[1,2-b]pyrazol]-4'-yl)carbamate

A mixture of (*S*)-1-(5-bromopyrazin-2-yl)-4'H,6'H-spiro[piperidine-4,5'-pyrrolo[1,2-b]pyrazol]-4'-amine (24 mg, 0.07 mmol) and Boc₂O (30 mg, 0.14 mmol) in DCM (0.3 mL) was stirred at rt for 1 h before being washed with H₂O, brine, dried over Na₂SO₄ and evaporated under reduced pressure to afford the title compound (30 mg, 99%). LCMS (ES⁺) Method 1: *m*/*z* 449 (M+H)⁺, RT 1.89 min.

### Step 3: 2-ethylhexyl 3-((5-((S)-4'-((tert-butoxycarbonyl)amino)-4'H,6'H-spiro[piperidine-4,5'-pyrrolo[1,2-b]pyrazol]-1-yl)pyrazin-2-yl)thio)propanoate

A suspension of *tert-butyl* (*S*)-(1-(5-bromopyrazin-2-yl)-4'H,6'H-spiro[piperidine-4,5'-pyrrolo[1,2-b]pyrazol]-4'-yl)carbamate (30 mg, 0.07 mmol), 2-ethylhexyl 3-mercaptopropanoate (0.02 mL, 0.08 mmol), Pd₂(dba)₃ (6.1 mg, 0.01 mmol), Xantphos (7.7 mg, 0.01 mmol) and TEA (0.03 mL, 0.2 mmol) in toluene (0.8 mL) was degassed and heated at 90°C for 2 h before being cooled to rt and evaporated under reduced pressure to get a residue which was purified by flash chromatography on silica gel (from 0% to 100% EtOAc in petroleum ether) to afford the title compound as a yellow oil (34 mg, 87%). LCMS (ES⁺) Method 1: *m*/*z* 587 (M+H)⁺, RT 2.65 min.

### Step 4: sodium (S)-5-(4'-((tert-butoxycarbonyl)amino)-4'H,6'H-spiro[piperidine-4,5'-pyrrolo[1,2-b]pyrazol]-1-yl)pyrazine-2-thiolate

A solution of 2-ethylhexyl 3-((5-((*S*)-4'-((tert-butoxycarbonyl)amino)-4'H,6'H-spiro[piperidine-4,5'-pyrrolo[1,2-b]pyrazol]-1-yl)pyrazin-2-yl)thio)propanoate (34 mg, 0.06 mmol) in MeOH (0.5 mL) was treated with NaOMe (25% wt in MeOH; 53 uL, 0.06 mmol) and stirred at rt for 45 min before being concentrated under reduced pressure to get a residue which was triturated with a mixture of petroleum ether/EtOAc (10/1 v/v) to get the title compound as a pale yellow powder (24 mg, 99%). LCMS (ES⁺) Method 1: *m*/*z* 403 (M+H)⁺, RT 1.32 min.

### Step 5: tert-butyl (S)-(1-(5-((2-chloro-3-fluorophenyl)thio)pyrazin-2-yl)-4'H,6'H-spiro[piperidine-4,5'-pyrrolo[1,2-b]pyrazol]-4'-yl)carbamate

A suspension of 2-chloro-1-fluoro-3-iodobenzene (18 mg, 0.07 mmol), sodium (*S*)-5*-*(4'-((*tert-*butoxycarbonyl)amino)-4'H,6'H-spiro[piperidine-4,5'-pyrrolo[1,2-b]pyrazol]-1-yl)pyrazine-2-thiolate (25 mg, 0.06 mmol), Pd₂(dba)₃ (3 mg, 0.003 mmol), DIPEA (0.02 mL, 0.12 mmol) and Xantphos (3.4 mg, 0.01 mmol) in 1,4-dioxane (0.6 mL) was degassed and heated at 90°C for 1 h before being cooled to rt and evaporated under reduced pressure to afford a residue which was purified by flash chromatography on silica gel (from 0% to 100% EtOAc in petroleum ether) to furnish the title compound as a yellow solid (6 mg, 19%). LCMS (ES⁺) Method 1: *m*/*z* 532 (M+H)⁺, RT 2.28 min.

### Step 6: (S)-1-(5-((2-chloro-3-fluorophenyl)thio)pyrazin-2-yl)-4'H,6'H-spiro[piperidine-4,5'-pyrrolo[1,2-b]pyrazol]-4'-amine

A solution of (*R*)-*N*((*S*)-1-(5-((2-chloro-3-fluorophenyl)thio)pyrazin-2-yl)-4'H,6'H-spiro[piperidine-4,5'-pyrrolo[1,2-b]pyrazol]-4'-yl)-2-methylpropane-2-sulfinamide (6 mg, 0.011 mmol) in DCM (0.5 mL) was treated with TFA (0.2 mL) and stirred at rt for 10 min before being evaporated under reduced pressure to afford a residue which was purified by preparative HPLC-MS (from 20% to 100% MeCN/ H₂O + 0.1% TFA) to obtain the title compound as a yellow powder (4 mg, 82%). ¹H NMR (400 MHz, DMSO-*d*₆) δ 8.51 (s, 1H), 8.38 (br s, 3H), 8.32 (s, 1H), 7.59 (s, 1H), 7.31-7.28 (m, 2H), 6.84-6.77 (m, 1H), 6.31 (s, 1H), 4.50-4.24 (m, 5H), 3.40-3.20 (m, 2H), 1.90-1.64 (m, 4H). LCMS (ES⁺) Method 1: *m*/*z* 431 (M+H)⁺, RT 1.31 min.

### Example 31: (S)-1-(5-(2,3-dichlorophenyl)pyrazin-2-yl)-4'H,6'H-spiro[piperidine-4,5'-pyrrolo[1,2-b]pyrazol]-4'-amine

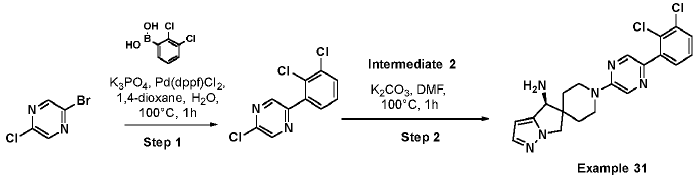

### Step 1: 2-chloro-5-(2,3-dichlorophenyl)pyrazine

A mixture of 2-bromo-5-chloro-pyrazine (70 mg, 0.36 mmol), (2,3-dichlorophenyl)boronic acid (69 mg, 0.36 mmol), Pd(dppf)Cl₂ (26 mg, 0.036 mmol) and K₃PO₄ (230 mg, 1.09 mmol) in a mixture of 1,4-dioxane (0.9 mL) and H₂O (90 µL) was degassed and heated at 100°C for 1 h before being cooled and filtered on a pad of cellulose which was washed with EtOAc. The solvent was removed under reduced pressure to afford a residue which was purified by flash chromatography on silica gel (from 0% to 100% EtOAc in petroleum ether) to afford the title compound (20 mg, 21%). LCMS (ES⁺) Method 1: *m*/*z* 259 (M+H)⁺, RT 2.17 min.

### Step 2: (S)-1-(5-(2,3-dichlorophenyl)pyrazin-2-yl)-4'H,6'H-spiro[piperidine-4,5'-pyrrolo[1,2-b]pyrazol]-4'-amine

A solution of 2-chloro-5-(2,3-dichlorophenyl)pyrazine (20 mg, 0.08 mmol) in DMF (0.4 mL) was treated with **Intermediate 2** (20 mg, 0.08 mmol) and K₂CO₃ (53 mg, 0.39 mmol) and stirred at 100°C for 1 h before being cooled and filtered on a pad of cellulose which was washed with a mixture of EtOAc/MeOH. The solvent was removed under reduced pressure to afford a residue which purified by preparative HPLC-MS (from 17% to 45% MeCN/ H₂O + 0.1% TFA) to obtain the title compound as a yellow powder (9.6 mg, 30%). ¹H NMR (400 MHz, DMSO-*d₆*) δ 8.52 (s, 1H), 8.43 (s, 1H), 8.40 (br s, 3H), 7.70 (d, *J*=8.1 Hz, 1H), 7.60 (s, 1H), 7.55-7.53 (m, 1H), 7.49-7.45 (m, 1H), 6.32 (s, 1H), 4.48-4.25 (m, 5H), 3.37-3.31 (m, 1H), 3.23-3.17 (m, 1H), 1.89-1.78 (m, 3H), 1.69-1.66 (m, 1H). LCMS (ES⁺) Method 1: *m*/*z* 415 (M+H)⁺, RT 1.22 min.

### Example 32: (S)-1-(5-((2,4-difluorophenyl)thio)pyrazin-2-yl)-4'H,6'H-spiro[piperidine-4,5'-pyrrolo[1,2-b]pyrazol]-4'-amine

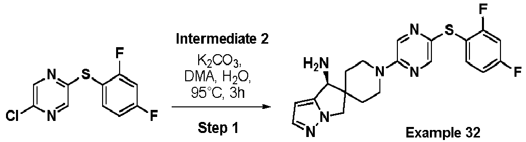

### Step 1: (S)-1-(5-((2,4-difluorophenyl)thio)pyrazin-2-yl)-4'H,6'H-spiro[piperidine-4,5'-pyrrolo[1,2-b]pyrazol]-4'-amine

A solution of 2-chloro-5-(2,4-difluorophenyl)sulfanyl-pyrazine (prepared following the same procedure reported for the synthesis of **Example 10 in** step 1; 25 mg, 0.1 mmol), Intermediate 2 (35 mg, 0.12 mmol) and K₂CO₃ (67 mg, 0.48 mmol) in DMA (320 µL) and H₂O (180 µL) was heated at 95°C for 3 h before being cooled, filtered, washed with a minimum amount of DMSO and purified by preparative HPLC-MS (from 17% to 45% MeCN/ H₂O + 0.1% TFA) to obtain the title compound as a pale yellow powder (3.4 mg, 8%). ¹H NMR (400 MHz, DMSO-*d₆*) δ 8.36 (s, 1H), 8.31 (br s, 3H), 8.18 (s, 1H), 7.58 (s, 1H), 7.52-7.46 (m, 1H), 7.39 (t, *J*=9.1 Hz, 1H), 7.16-7.12 (m, 1H), 6.30 (s, 1H), 4.44 (s, 1H), 4.37-4.19 (m, 4H), 3.30-3.25 (m, 1H), 3.17-3.11 (m, 1H), 1.83-1.70 (m, 3H), 1.65-1.62 (m, 1H). LCMS (ES⁺) Method 1: *m*/*z* 415 (M+H)⁺, RT 1.27 min.

The following examples were synthesized using the above procedure with the corresponding starting materials: **Example 33; Example 34; Example 35; Example 36; Example 37; Example 38; Example 39; Example 40; Example 41; Example 42; Example 43; Example 44; Example 45; Example 46; Example 47; Example 48; Example 49; Example 50; Example 75; Example 76; Example 77; Example 80; Example 81; Example 82**

| **Example #** | **¹H NMR (400 MHz, DMSO-*d₆*)** |
|---|---|
| 33 | δ 8.43 (s, 1H), 8.39 (br s, 3H), 8.19 (s, 1H), 7.59 (s, 1H), 7.38 (d, *J*=7.7 Hz, 1H), 7.17 (t, *J*=7.9 Hz, 1H), 7.09 (d, *J*=7.9 Hz, 1H), 6.31 (s, 1H), 4.46 (br s, 1H), 4.39-4.32 (m, 2H), 4.24 (br d, *J*=11.4 Hz, 2H), 3.30-3.27 (m, 1H), 3.19-3.13 (m, 1H), 1.83-1.73 (m, 3H), 1.67-1.63 (m, 1H). |
| 34 | δ 8.45 (s, 1H), 8.43 (br s, 3H), 8.23 (s, 1H), 7.59 (s, 1H), 7.57 (br s, 1H), 7.21 (d, *J*=6.8 Hz, 2H), 6.31 (s, 1H), 4.47 (br s, 1H), 4.39-4.34 (m, 2H), 4.25 (br d, *J*=11.4 Hz, 2H), 3.34-3.28 (m, 1H), 3.20-3.14 (m, 1H), 1.87-1.74 (m, 3H), 1.68-1.64 (m, 1H). |
| 35 | δ 8.50 (s, 1H), 8.40 (br s, 3H), 8.26 (s, 1H), 7.59 (s, 1H), 7.21 (d, *J*=6.9 Hz, 1H), 7.15 (t, *J*=7.6 Hz, 1H), 6.79 (br d, *J*=7.7 Hz, 1H), 6.32 (s, 1H), 4.48 (br s, 1H), 4.38 (br d, *J*=10.7 Hz, 2H), 4.31-4.25 (m, 2H), 3.31-3.29 (m, 1H), 3.22-3.17 (m, 1H), 2.36 (s, 3H), 1.84-1.75 (m, 3H), 1.69-1.65 (m, 1H). |
| 36 | δ 8.40 (s, 1H), 8.34 (br s, 3H), 8.19 (s, 1H), 7.58 (s, 1H), 7.26-7.22 (m, 1H), 7.07 (br d, *J*=5.3 Hz, 2H), 6.30 (s, 1H), 4.44 (br s, 1H), 4.38-4.30 (m, 2H), 4.25-4.21 (m, 2H), 3.29-3.25 (m, 1H), 3.20-3.16 (m, 1H), 2.25 (s, 3H), 1.84-1.73 (m, 3H), 1.67-1.63 (m, 1H). |
| 37 | δ 8.61 (s, 1H), 8.60 (br s, 1H), 8.43 (br s, 4H), 7.77-7.75 (m, 1H), 7.70-7.66 (m, 1H), 7.60 (s, 1H), 7.37 (br d, *J*=8.1 Hz, 1H), 7.01 (d, *J*=5.0 Hz, 1H), 6.33 (s, 1H), 4.49-4.27 (m, 5H), 4.01 (s, 3H), 3.39-3.35 (m, 1H), 3.27-3.22 (m, 1H), 1.88-1.78 (m, 3H), 1.72-1.69 (m, 1H). |
| 38 | δ 8.41 (br s, 4H), 8.17 (s, 1H), 7.59 (s, 1H), 7.06 (t, *J*=8.0 Hz, 1H), 6.69 (d, *J*=7.9 Hz, 1H), 6.62 (d, *J*=7.7 Hz, 1H), 6.31 (s, 1H), 4.55 (t, *J*=8.7 Hz, 2H), 4.47 (br s, 1H), 4.39-4.31 (m, 2H), 4.24 (br d, *J*=11.2 Hz, 2H), 3.32-3.27 (m, 1H), 3.19-3.13 (m, 1H), 3.09 (t, *J*=8.8 Hz, 2H), 1.85-1.74 (m, 3H), 1.67-1.64 (m, 1H). |
| 39 | δ 8.68 (d, *J*=4.8 Hz, 1H), 8.59 (s, 1H), 8.44 (br s, 4H), 8.22 (d, *J*=8.3 Hz, 1H), 8.07 (d, *J* =8.6 Hz, 1H), 7.88 (t, *J*=7.7 Hz, 1H), 7.74 (t, *J*=7.8 Hz, 1H), 7.60 (s, 1H), 6.98 (d, *J*=4.8 Hz, 1H), 6.33 (s, 1H), 4.49-4.26 (m, 5H), 3.41-3.35 (m, 1H), 3.27-3.21 (m, 1H), 1.89-1.78 (m, 3H), 1.72-1.68 (m, 1H). |
| 40 | δ 8.48 (br s, 3H), 8.40 (s, 1H), 8.15 (s, 1H), 7.58 (s, 1H), 7.40 (s, 1H), 7.23 (d, *J*=8.3 Hz, 1H), 7.13 (d, *J*=8.3 Hz, 1H), 6.31 (s, 1H), 4.46 (br s, 1H), 4.38-4.21 (m, 4H), 3.32-3.26 (m, 1H), 3.18-3.12 (m, 1H), 2.34 (s, 3H), 1.87-1.74 (m, 3H), 1.66-1.63 (m, 1H). |
| 41 | δ 8.47 (s, 1H), 8.43 (br s, 3H), 8.25 (s, 1H), 7.79 (d, *J*=7.7 Hz, 1H), 7.59-7.55 (m, 2H), 7.47-7.43 (m, 1H), 7.27 (d, *J*=8.1 Hz, 1H), 6.31 (s, 1H), 4.47 (br s, 1H), 4.39-4.35 (m, 2H), 4.29-4.24 (m, 2H), 3.36-3.30 (m, 1H), 3.21-3.16 (m, 1H), 1.85-1.74 (m, 3H), 1.68-1.65 (m, 1H). |
| 42 | δ 8.43 (br s, 3H), 8.33 (s, 1H), 8.00 (s, 1H), 7.58 (s, 1H), 7.37-7.21 (m, 2H), 6.78 (d, *J*=8.1 Hz, 1H), 6.30 (s, 1H), 4.56 (t, *J*=8.8 Hz, 2H), 4.45 (br s, 1H), 4.38-4.16 (m, 4H), 3.33-3.08 (m, 4H), 1.84-1.60 (m, 4H). |
| 43 | δ 8.55 (s, 1H), 8.45 (br s, 3H), 8.38 (s, 1H), 8.30 (s, 1H), 7.95 (d, *J*=8.3 Hz, 1H), 7.60 (s, 1H), 7.20 (d, *J*=8.3 Hz, 1H), 6.32 (s, 1H), 4.49 (br s, 1H), 4.44-4.26 (m, 4H), 3.37-3.34 (m, 1H), 3.26-3.21 (m, 1H), 1.90-1.77 (m, 3H), 1.71-1.68 (m, 1H). |
| 44 | δ .77 (br s, 1H), 8.48 (s, 1H), 8.42 (br s, 3H), 8.28 (s, 1H), 7.77 (s, 1H), 7.52 (br s, 2H), 7.45 (d, *J*=7.9 Hz, 1H), 7.38 (t, *J*=7.9 Hz, 1H), 7.02 (d, *J*=7.9 Hz, 1H), 6.25 (s, 1H), 4.42 (br s, 1H), 4.36-4.19 (m, 4H), 3.32-3.26 (m, 1H), 3.17-3.12 (m, 1H), 1.83-1.69 (m, 3H), 1.64-1.60 (m, 1H). |
| 45 | δ 8.16 (br s, 4H), 7.96 (s, 1H), 7.39 (s, 1H), 7.13-7.07 (m, 1H), 6.92-6.87 (m, 1H), 6.10 (s, 1H), 4.25 (br s, 1H), 4.18-4.00 (m, 4H), 3.09-3.05 (m, 1H), 2.98-2.91 (m, 1H), 1.97 (s, 3H), 1.67-1.53 (m, 3H), 1.46-1.43 (m, 1H). |
| 46 | δ 8.40 (s, 1H), 8.11 (s, 1H), 7.57 (s, 1H), 7.15 (d, *J*=6.9 Hz, 1H), 7.10 (t, *J* =7.6 Hz, 1H), 6.94 (d, *J*=7.7 Hz, 1H), 6.29 (br s, 1H), 4.43 (br s, 1H), 4.35-4.19 (m, 4H), 3.28-3.23 (m, 1H), 3.20-3.13 (m, 1H), 2.93-2.90 (m, 2H), 2.81 (t, *J*=7.2 Hz, 2H), 2.02 (t, *J*=7.2 Hz, 2H), 1.85-1.72 (m, 3H), 1.66-1.62 (m, 1H). |
| 47 | δ 8.38 (br s, 4H), 8.03 (s, 1H), 7.59 (s, 1H), 7.14-7.11 (m, 1H), 7.06-7.04 (m, 2H), 6.31 (s, 1H), 4.45 (br s, 1H), 4.38-4.19 (m, 4H), 3.29-3.24 (m, 1H), 3.16-3.11 (m, 1H), 2.31 (s, 3H), 2.28 (s, 3H), 1.83-1.73 (m, 3H), 1.65-1.62 (m, 1H). |
| 48 | δ 8.47 (s, 1H), 8.34 (br s, 3H), 8.31 (s, 1H), 7.61 (s, 1H), 7.40-7.35 (m, 1H), 7.24-7.20 (m, 1H), 7.08-7.05 (m, 1H), 6.33 (s, 1H), 4.48 (br s, 1H), 4.41-4.35 (m, 2H), 4.27 (br d, *J*=11.2 Hz, 2H), 3.35-3.33 (m, 1H), 3.24-3.18 (m, 1H), 1.89-1.77 (m, 3H), 1.70-1.67 (m, 1H). |
| 49 | δ 8.40 (br s, 3H), 8.37 (s, 1H), 8.24 (s, 1H), 7.59 (s, 1H), 7.40-7.26 (m, 2H), 6.31 (s, 1H), 4.45 (br s, 1H), 4.38-4.20 (m, 4H), 3.31-3.26 (m, 1H), 3.18-3.12 (m, 1H), 1.85-1.72 (m, 3H), 1.66-1.62 (m, 1H). |
| 50 | δ 8.50 (s, 1H), 8.47 (br s, 3H), 8.32 (s, 1H), 7.59 (s, 1H), 7.10 (brt, *J*=9.3 Hz, 1H), 6.92 (br d, *J*=6.6 Hz, 2H), 6.32 (s, 1H), 4.48 (br s, 1H), 4.40-4.25 (m, 4H), 3.37-3.31 (m, 1H), 3.23-3.18 (m, 1H), 1.88-1.76 (m, 3H), 1.69-1.66 (m, 1H). |
| 75 | δ 8.20 (s, 1H), 8.10 (s, 1H), 7.48 (br s, 1H), 7.16 (br t, *J*=7.9 Hz, 2H), 6.44 (s, 1H), 6.19 (s, 1H), 4.28-4.06 (m, 5H), 3.25-3.18 (m, 1H), 3.10-3.04 (m, 1H), 1.76-1.61 (m, 3H), 1.56-1.53 (m, 1H). |
| 76 | δ 8.50 (s, 1H), 8.46 (br s, 3H), 8.35 (s, 1H), 7.91 (d, *J*=9.9 Hz, 1H), 7.62-7.59 (m, 2H), 7.17 (t, *J*=7.9 Hz, 1H), 6.32 (s, 1H), 4.48 (br s, 1H), 4.38 (br d, *J*=11.2 Hz, 2H), 4.31-4.25 (m, 2H), 3.32-3.27 (m, 1H), 3.24-3.18 (m, 1H), 1.86-1.74 (m, 3H), 1.69-1.66 (m, 1H). |
| 77 | δ 8.32 (s, 1H), 8.30 (br s, 3H), 8.17 (s. 1H), 7.66-7.61 (m, 1H), 7.52 (s, 1H), 7.47-7.40 (m, 1H), 6.24 (s, 1H), 4.38 (br s, 1H), 4.31-4.23 (m, 2H), 4.17 (br d, *J*=11.4 Hz, 2H), 3.25-3.19 (m, 1H), 3.11-3.06 (m, 1H), 1.75-1.65 (m, 3H), 1.59-1.55 (m, 1H). |
| 80 | δ 8.46 (s, 1H), 8.42 (br s, 3H), 8.18 (s, 1H), 8.05 (s, 1H), 7.79 (s, 1H), 7.59 (s, 1H), 7.46-7.41 (m, 1H), 7.31 (br t, *J*=9.0 Hz, 1H), 6.90 (d, *J*=7.9 Hz, 1H), 6.56 (s, 1H), 6.31 (s, 1H), 4.47 (br s, 1H), 4.38 (br d, *J*=11.2 Hz, 2H), 4.29-4.24 (m, 2H), 3.35-3.30 (m, 1H), 3.21-3.16 (m, 1H), 1.84-1.73 (m, 3H), 1.68-1.65 (m, 1H). |
| 81 | δ 8.43 (s, 1H), 8.33 (br s, 3H), 8.29 (s, 1H), 8.22 (br s, 1H), 7.81 (s, 1H), 7.73 (brt, *J*=6.9 Hz, 1H), 7.58 (s, 1H), 7.34-7.24 (m, 2H), 6.58 (s, 1H), 6.30 (s, 1H), 4.45 (br s, 1H), 4.38-4.32 (m, 2H), 4.24 (br d, *J*=11.6 Hz, 2H), 3.33-3.28 (m, 1H), 3.20-3.14 (m, 1H), 1.84-1.73 (m, 3H), 1.67-1.64 (m, 1H). |
| 82 | δ 8.56 (s, 1H), 8.43 (br s, 3H), 8.35 (s, 1H), 7.59 (br s, 2H), 7.47-7.39 (m, 2H), 7.25 (s, 1H), 6.99 (t, *J*=51.9 Hz, 1H), 7.07 (br d, *J*=7.7 Hz, 1H), 6.32 (s, 1H), 4.49-4.26 (m, 5H), 3.38-3.34 (m, 1H), 3.26-3.20 (m, 1H), 1.87-1.76 (m, 3H), 1.71-1.67 (m, 1H). |

### Example 51: (S)-1-(5-((3-fluorophenyl)thio)pyrazin-2-yl)-4'H,6'H-spiro[piperidine-4,5'-pyrrolo[1,2-b]pyrazol]-4'-amine

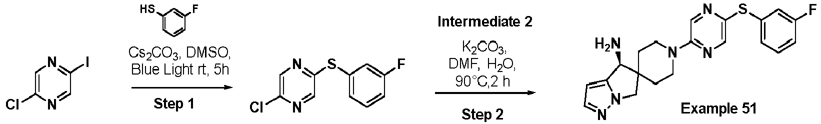

### Step 1: 2-chloro-5-((3-fluorophenyl)thio)pyrazine

A solution of 2-chloro-5-iodo-pyrazine (20 mg, 0.08 mmol) in DMSO (0.8 mL) was treated with Cs₂CO₃ (27 mg, 0.08 mmol) and 3-fluorobenzenethiol (11 mg, 0.08 mmol), degassed and irradiated with the Blue Light (Penn Photoreactor) at rt for 5 h. The solvent was then evaporated under reduced pressure to afford a residue which was purified by flash chromatography on silica gel (from 0% to 100% EtOAc in petroleum ether) to afford the title compound (6 mg, 30%). LCMS (ES⁺) Method 1: *m*/*z* 241 (M+H)⁺, RT 2.07 min.

### Step 2: (S)-1-(5-((3-fluorophenyl)thio)pyrazin-2-yl)-4'H,6'H-spiro[piperidine-4,5'-pyrrolo[1,2-b]pyrazol]-4'-amine

The title compound was prepared following procedure reported for the synthesis of Example 32 in step 1 and obtained as a white powder (2.4 mg, 24%). ¹H NMR (400 MHz, DMSO-*d₆*) δ 8.47 (s, 1H), 8.30 (bs, 3H), 8.27 (s, 1H), 7.58 (s, 1H), 7.41-7.35 (m, 1H), 7.10-7.05 (m, 3H), 6.29 (s, 1H), 4.43 (br s, 1H), 4.35 (br d, *J*=12.5 Hz, 2H), 4.28-4.23 (m, 2H), 3.35-3.32 (m, 1H), 3.23-3.17 (m, 1H), 1.88-1.73 (m, 3H), 1.68-1.64 (m, 1H). LCMS (ES⁺) Method 1: *m*/*z* 397 (M+H)⁺, RT 1.22 min.

### Example 52: (S)-1-(5-((1,3-dihydroisobenzofuran-4-yl)thio)pyrazin-2-yl)-4'H,6'H-spiro[piperidine-4,5'-pyrrolo[1,2-b]pyrazol]-4'-amine

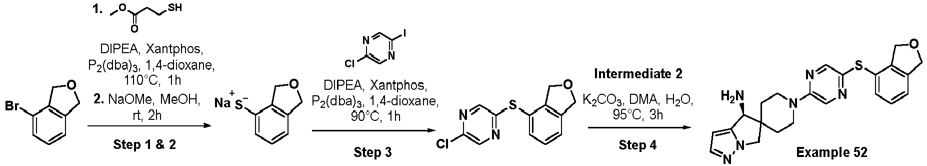

### Step 1: methyl 3-((1,3-dihydroisobenzofuran-4-yl)thio)propanoate

The title compound was prepared as reported in the synthesis of **Intermediate 7** in step 1 and obtained as a yellow powder (31 mg, 65%). LCMS (ES⁺) Method 1: *m*/*z* 239 (M-H)⁻, RT 1.63 min.

### Step 2: sodium 1,3-dihydroisobenzofuran-4-thiolate

A solution of methyl 3-(1,3-dihydroisobenzofuran-4-ylsulfanyl)propanoate (31 mg, 0.13 mmol) in MeOH (0.37 mL) was treated with NaOMe (25% in MeOH; 34 mg, 0.16 mmol) and stirred at rt for 2 h before being concentrated under reduced pressure to get a residue which was treated with Et₂O. After stirring for 30 min the precipitate was isolated by filtration and dried to get the title compound as a yellow powder (22.6 mg, 99%). LCMS (ES⁺) Method 1: *m*/*z* 153 (M+H)⁺, RT 1.51 min.

### Step 3: 2-chloro-5-((1,3-dihydroisobenzofuran-4-yl)thio)pyrazine

The title compound was prepared according to the procedure reported in the synthesis of **Example 18** in step 1 starting from 2-chloro-5-iodopyrazine (15 mg, 91%). LCMS (ES⁺) Method 1: *m*/*z* 265 (M+H)⁺, RT 1.89 min.

### Step 4: (S)-1-(5-((1,3-dihydroisobenzofuran-4-yl)thio)pyrazin-2-yl)-4'H,6'H-spiro[piperidine-4,5'-pyrrolo[1,2-b]pyrazol]-4'-amine

The title compound was prepared according to the procedure reported in the synthesis of **Example 18** in step 2 as a yellow powder (6.2 mg, 26%). ¹H NMR (400 MHz, DMSO-*d₆*) δ 8.35 (br s, 3H), 8.28 (s, 1H), 8.07 (s, 1H), 7.48 (s, 1H), 7.20-7.15 (m, 2H), 7.06 (br d, *J*=7.2 Hz, 1H), 6.20 (s, 1H), 4.95 (s, 2H), 4.81 (s, 2H), 4.35 (br s, 1H), 4.27-4.10 (m, 4H), 3.21-3.15 (m, 1H), 3.07-3.02 (m, 1H), 1.75-1.63 (m, 3H), 1.55-1.52 (m, 1H). LCMS (ES⁺) Method 1: *m*/*z* 421 (M+H)⁺, RT 1.09 min.

The following examples were synthesized using the above procedure with the corresponding starting materials: **Example 53; Example 54; Example 55; Example 56; Example 57; Example 58; Example 59; Example 60; Example 61; Example 62; Example 63; Example 64; Example 65; Example 66; Example 67; Example 68; Example 69; Example 70; Example 71; Example 72; Example 73; Example 74; Example 83; Example 84**

| **Example** # | **¹H NMR (400 MHz, DMSO-*d₆*)** |
|---|---|
| 53 | δ 8.41 (br s, 4H), 8.27 (s, 1H), 7.59 (s, 1H), 7.44-7.37 (m, 1H), 7.23-7.17 (m, 1H), 7.12-7.08 (m, 1H), 6.31 (s, 1H), 4.46 (br s, 1H), 4.39-4.32 (m, 2H), 4.24 (br d, *J*=11.6 Hz, 2H), 3.33-3.28 (m, 1H), 3.19-3.14 (m, 1H), 1.86-1.74 (m, 3H), 1.67-1.63 (m, 1H). |
| 54 | δ 8.62 (s, 1H), 8.46-8.40 (m, 5H), 7.60 (s, 1H), 7.07 (d, *J*=5.3 Hz, 1H), 6.33 (s, 1H), 4.50-4.27 (br m, 5H), 3.40-3.37 (m, 1H), 3.28-3.23 (m, 1H), 1.87-1.76 (m, 3H), 1.72-1.69 (m, 1H). |
| 55 | δ 8.42 (br s, 3H), 8.37 (s, 1H), 8.19 (s, 1H), 7.58 (s, 1H), 7.20-7.08 (m, 2H), 6.31 (s, 1H), 4.46 (br s, 1H), 4.38-4.20 (m, 4H), 3.93 (s, 3H), 3.31-3.25 (m, 1H), 3.17-3.12 (m, 1H), 1.82-1.72 (m, 3H), 1.65-1.62 (m, 1H). |
| 56 | δ 8.52 (s, 1H), 8.48 (br s, 3H), 8.33 (s, 1H), 7.59 (s, 1H), 7.55-7.46 (m, 2H), 7.07 (br d, *J*=7.9 Hz, 1H), 6.32 (s, 1H), 4.48 (br s, 1H), 4.42-4.37 (m, 2H), 4.32-4.25 (m, 2H), 3.38-3.32 (m, 1H), 3.24-3.18 (m, 1H), 1.89-1.76 (m, 3H), 1.70-1.66 (m, 1H). |
| 57 | δ 8.41 (s, 1H), 8.17 (s, 1H), 7.52 (br s, 1H), 7.21-7.16 (m, 1H), 7.12-7.08 (m, 1H), 6.94 (br d, *J*=7.9 Hz, 1H), 6.22 (br s, 1H), 4.34-4.17 (m, 5H), 3.34-3.30 (m, 1H), 3.22-3.16 (m, 1H), 2.27 (s, 3H), 1.86-1.63 (m, 4H). |
| 58 | δ 8.42 (s, 1H), 8.37 (br s, 3H), 8.28 (s, 1H), 7.59 (br s, 2H), 7.47-7.43 (m, 1H), 7.37-7.30 (m, 1H), 7.23 (t, *J*= 55.8 Hz, 1H), 6.31 (s, 1H), 4.45 (br s, 1H), 4.38-4.23 (m, 4H), 3.30-3.27 (m, 1H), 3.21-3.15 (m, 1H), 1.84-1.63 (m, 4H). |
| 59 | δ 8.42 (s, 1H), 8.29 (s, 1H), 7.52-7.50 (br m, 4H), 7.39-7.37 (br m, 1H), 7.32-7.28 (m, 1H), 7.03 (t, *J*= 56.1 Hz, 1H), 6.51 (s, 1H), 4.38-4.15 (m, 5H), 3.41-3.35 (m, 1H), 3.27-3.21 (m, 1H), 1.85-1.64 (m, 4H). |
| 60 | δ 8.39 (br s, 3H), 8.19 (s, 1H), 7.59 (s, 1H), 7.14-7.08 (m, 2H), 6.84-6.78 (m, 1H), 6.49 (s, 1H), 6.31 (s, 1H), 4.45 (br s, 1H), 4.38-4.23 (m, 4H), 3.85 (s, 3H), 3.30-3.27 (m, 1H), 3.21-3.15 (m, 1H), 1.83-1.57 (m, 4H). |
| 61 | δ 8.98 (br d, *J*=3.5 Hz, 1H), 8.75 (d, *J*=8.6 Hz, 1H), 8.41 (br s, 3H), 8.34 (s, 1H), 8.20 (s, 1H), 8.05 (d, *J*=8.3 Hz, 1H), 7.79-7.75 (m, 1H), 7.72-7.70 (m, 1H), 7.65 (dd, *J₁*=8.6 Hz, *J₂*=4.2 Hz, 1H), 7.58 (s, 1H), 6.30 (s, 1H), 4.44 (br s, 1H), 4.36-4.17 (m, 4H), 3.28-3.23 (m, 1H), 3.14-3.09 (m, 1H), 1.83-1.60 (m, 4H). |
| 62 | δ 8.53 (s, 1H), 8.43 (br s, 3H), 8.39 (s, 1H), 8.31 (s, 1H), 8.26 (s, 1H), 7.58 (s, 1H), 6.31 (s, 1H), 4.46 (br s, 1H), 4.38-4.20 (m, 4H), 3.33-3.26 (m, 1H), 3.18-3.13 (m, 1H), 2.46 (s, 3H), 1.85-1.62 (m, 4H). |
| 63 | δ 8.41 (s, 1H), 8.19 (s, 1H), 7.48 (br s, 1H), 7.45-7.43 (m, 1H), 7.20-7.18 (m, 2H), 6.94-6.92 (m, 1H), 6.19 (s, 1H), 4.29-4.15 (m, 5H), 3.29-3.25 (m, 1H), 3.18-3.12 (m, 1H), 1.80-1.57 (m, 4H). |
| 64 | δ 8.39 (br s, 3H), 8.31 (s, 1H), 8.04 (s, 1H), 7.58 (s. 1H), 7.45 (t, *J*=8.7 Hz, 1H), 6.97 (dd, *J*₁=11.2 Hz, *J*₂=2.4 Hz, 1H), 6.84 (br d, *J*=9.2 Hz, 1H), 6.30 (s, 1H), 4.45 (br s, 1H), 4.35 (d, *J*=11.4 Hz, 1H), 4.29-4.15 (m, 3H), 3.80 (s, 3H), 3.28-3.22 (m, 1H), 3.14-3.08 (m, 1H), 1.81-1.70 (m, 3H), 1.63-1.60 (m, 1H). |
| 65 | δ 8.36 (br s, 5H), 8.11 (s, 1H), 7.79 (br s, 1H), 7.68-7.66 (m, 1H), 7.58 (s, 1H), 7.32-7.25 (m, 1H), 6.31 (s, 1H), 4.45 (br s, 1H), 4.37-4.18 (m, 4H), 3.86 (s, 3H), 3.27-3.24 (m, 1H), 3.16-3.10 (m, 1H), 1.82-1.71 (m, 3H), 1.65-1.61 (m, 1H). |
| 66 | δ 9.17 (br s, 1H), 8.79 (br d, *J*=8.6 Hz, 1H), 8.50 (s, 1H), 8.42 (br s, 3H), 8.40 (s, 1H), 8.24 (d, *J*=7.9 Hz, 1H), 7.83 (dd, *J₁*=8.6 Hz, *J*₂=4.2 Hz, 1H), 7.59 (s, 1H), 7.36 (d. *J*=7.7 Hz, 1H), 6.32 (s, 1H), 4.48 (br s, 1H), 4.38 (br d, *J*=11.0 Hz, 2H), 4.31-4.25 (m, 2H), 3.30-3.26 (m, 1H), 3.24-3.18 (m, 1H), 1.86-1.74 (m, 3H), 1.70-1.66 (m, 1H). |
| 67 | δ 9.13 (br d, *J*=4.0 Hz, 1H), 8.80 (br d, *J*=8.6 Hz, 1H), 8.46 (br s, 4H), 8.37 (s, 1H), 8.10 (d, *J*=7.9 Hz, 1H), 7.80 (dd, *J₁*=8.8 Hz, *J*₂=4.2 Hz, 1H), 7.59 (s, 1H), 7.50 (d. *J*=7.9 Hz, 1H), 6.32 (s, 1H), 4.47 (br s, 1H), 4.39-4.32 (m, 2H), 4.29-4.24 (m, 2H), 3.35-3.29 (m, 1H), 3.22-3.16 (m, 1H), 1.84-1.73 (m, 3H), 1.68-1.65 (m, 1H). |
| 68 | δ 9.03 (br d, *J*=4.0 Hz, 1H), 8.78 (br d, *J*=8.6 Hz, 1H), 8.39 (br s, 3H), 8.29 (s, 1H), 8.17 (s, 1H), 7.83 (dd, *J₁*=8.0 Hz, *J*₂=4.9 Hz, 1H), 7.75 (dd, *J₁*=8.7 Hz, *J*₂=4.1 Hz, 1H), 7.64 (dd, *J₁*=10.5 Hz, *J*₂=8.3 Hz, 1H), 7.57 (br s, 1H), 6.29 (s, 1H), 4.43 (br s, 1H), 4.33 (br d, *J*=11.4 Hz, 1H), 4.27-4.14 (m, 3H), 3.26-3.20 (m, 1H), 3.12-3.07 (m, 1H), 1.81-1.68 (m, 3H), 1.62-1.59 (m, 1H). |
| 69 | δ 8.43 (br s, 3H), 8.40 (s, 1H), 8.19 (s, 1H), 7.59 (s, 1H), 7.08-7.02 (m, 2H), 6.94 (t, *J*=6.8 Hz, 1H), 6.31 (s, 1H), 4.46 (br s, 1H), 4.38-4.31 (m, 2H), 4.26-4.21 (m, 2H), 3.32-3.26 (m, 1H), 3.18-3.13 (m, 1H), 2.08-2.01 (m, 1H), 1.86-1.73 (m, 3H), 1.66-1.63 (m, 1H), 1.01-0.96 (m, 2H), 0.75-0.71 (m, 2H). |
| 70 | δ 9.10 (br d, *J*=4.0 Hz, 1H), 8.80 (br d, *J*=8.6 Hz, 1H), 8.45 (br s, 3H), 8.33 (s, 1H), 8.24 (s, 1H), 7.96 (d, *J*=7.9 Hz, 1H), 7.76 (dd, *J₁*=8.6 Hz, *J*₂=4.2 Hz, 1H), 7.65 (d, *J*=8.1 Hz, 1H), 7.57 (br s, 1H), 6.30 (s, 1H), 4.44 (br s, 1H), 4.36-4.17 (m, 4H), 3.28-3.23 (m, 1H), 3.14-3.09 (m, 1H), 1.81-1.72 (m, 3H), 1.64-1.60 (m, 1H). |
| 71 | δ 8.42 (br s, 1H), 8.39 (br s, 3H), 8.28 (s, 1H), 7.59 (s, 1H), 7.57-7.52 (m, 1H), 7.47 (s, 1H), 7.36-7.31 (m, 1H), 7.19 (br d, *J*=7.9 Hz, 1H), 6.31 (s, 1H), 4.46 (br s, 1H), 4.38-4.33 (m, 2H), 4.24 (br d, *J*=11.4 Hz, 2H), 3.30-3.28 (m, 1H), 3.19-3.14 (m, 1H), 1.85-1.73 (m, 3H), 1.67-1.63 (m, 1H). |
| 72 | δ **8.40 (br s, 1H), 8.38 (br s, 3H), 8.22 (s, 1H), 7.69 (br d, J=6.8 Hz, 1H), 7.59 (s, 1H), 7.52-7.47 (m, 2H), 7.40 (br d**, *J*=7.2 Hz, 1H), 7.34-7.20 (m, 1H), 6.31 (s, 1H), 4.46 (br s, 1H), 4.38-4.31 (m, 2H), 4.24 (br d, *J*=11.6 Hz, 2H), 3.32-3.26 (m, 1H), 3.18-3.13 (m, 1H), 1.82-1.72 (m, 3H), 1.66-1.63 (m, 1H). |
| 73 | δ 9.04 (br d, *J*=4.0 Hz, 1H), 8.79 (br d, *J*=8.6 Hz, 1H), 8.39 (br s, 3H), 8.31 (s, 1H), 8.19 (s, 1H), 7.81-7.77 (m, 2H), 7.59-7.57 (m, 2H), 7.50 (t, *J*= 67.9 Hz, 1H), 6.30 (s, 1H), 4.43 (br s, 1H), 4.34 (br d, *J*=11.2 Hz, 1H), 4.27-4.15 (m, 3H), 3.27-3.21 (m, 1H), 3.13-3.07 (m, 1H), 1.81-1.69 (m, 3H), 1.63-1.59 (m, 1H). |
| 74 | δ **8.44 (br** s, 3H), 8.43 (s, 1H), 8.28 (s, 1H), 7.59 (br s, 2H), 7.50-7.43 (m, 2H), 7.01 (t, J= 56.1 Hz, 1H), 6.31 (s, 1H), 4.47 (br s, 1H), 4.38-4.35 (m, 2H), 4.25 (br d, *J*=11.6 Hz, 2H), 3.34-3.28 (m, 1H), 3.19-3.14 (m, 1H), 1.86-1.77 (m, 3H), 1.67-1.63 (m, 1H). |
| 83 | δ **8.51** (s, 1H), 8.23 (br s, 3H), 8.00 (s, 1H), 7.49 (br s, 1H), 7.39-7.37 (m, 1H), 7.16 (t, *J*=7.2 Hz, 1H), 6.43 (br s, 1H), 6.21 (br s, 1H), 4.34-4.07 (br m, 5H), 4.12 (s, 3H), 3.21-3.14 (m, 1H), 3.08-3.01 (m, 1H), 1.73-1.62 (m, 3H), 1.55-1.52 (m, 1H). |
| 84 | δ **8.49** (s, 1H), 8.38 (s, 1H), 8.30 (br s, 3H), 8.13 (s, 1H), 7.59-7.54 (m, 2H), 7.12 (d, *J*=8.8 Hz, 1H), 6.28 (s, 1H), 4.40 (bs, 1H), 4.32 (bs, 2H), 4.28-4.17 (m, 5H), 3.15-3.05 (m, 2H), 1.78-1.65 (m, 4H). |

### Example 78: (S)-1-(5-(3-fluorophenoxy)pyrazin-2-yl)-4'H,6'H-spiro[piperidine-4,5'-pyrrolo[1,2-b]pyrazol]-4'-amine

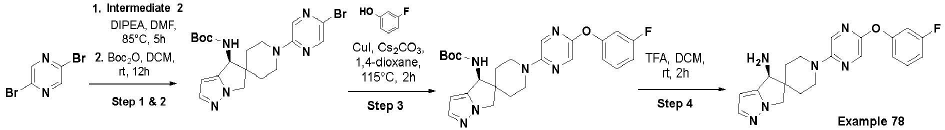

### Step 1: (S)-1-(5-bromopyrazin-2-yl)-4'H, 6'H-spiro[piperidine-4,5'-pyrrolo[1,2-b]pyrazol]-4'-amine

A solution of 2,5-dibromopyrazine (100 mg, 0.42 mmol), **Intermediate 2** (111 mg, 0.42 mmol) and DIPEA (0.37 mL, 2.1 mmol) in DMF (2.1 mL) was stirred for 5 h at 85°C before being cooled, diluted in EtOAc and washed with NaOH (1N). After removal of the solvent under reduced pressure the title compound was obtained and used as a crude (146 mg, 99%). LCMS (ES⁺) Method 1: *m*/*z* 349 (M+H)⁺, RT 1.28 min.

### Step 2: tert-butyl (S)-(1-(5-bromopyrazin-2-yl)-4'H,6'H-spiro[piperidine-4,5'-pyrrolo[1,2-b]pyrazol]-4'-yl)carbamate

A solution of (*S*)-1-(5-bromopyrazin-2-yl)-4'H,6'H-spiro[piperidine-4,5'-pyrrolo[1,2-b]pyrazol]-4'-amine (146 mg, 0.42 mmol) and BoC₂O (182 mg, 0.84 mmol) in DCM (1.7 mL) was stirred for 12 h at rt. The mixture was then washed with H₂O, brine, dried over Na₂SO₄, filtered and the solvent was then evaporated under reduced pressure to afford a residue which was purified by flash chromatography on silica gel (from 0% to 100% EtOAc in petroleum ether) to afford the title compound (40 mg, 21%). LCMS (ES⁺) Method 1: *m*/*z* 449 (M+H)⁺, RT 1.89 min.

### Step 3: tert-butyl (S)-(1-(5-(3-fluorophenoxy)pyrazin-2-yl)-4'H,6'H-spiro[piperidine-4,5'-pyrrolo[1,2-b]pyrazol]-4'-yl)carbamate

A solution of *tert*-butyl (*S*)-(1-(5-bromopyrazin-2-yl)-4'H,6'H-spiro[piperidine-4,5'-pyrrolo[1,2-b]pyrazol]-4'-yl)carbamate (20 mg, 0.04 mmol), 2-fluorophenol (5 mg, 0.04 mmol), *N*,*N*-dimethylglycine (0.5 mg, 0.004 mmol), CuI (0.8 mg, 0.004 mmol) and Cs₂CO₃ (20 mg, 0.06 mmol) in 1,4-dioxane (0.22 mL) was stirred for 2 h at 115°C before being cooled, filtered on a pad of solka floc and washed with EtOAc. The solvent was then evaporated under reduced pressure to afford a residue which was purified by flash chromatography on silica gel (from 0% to 100% EtOAc in petroleum ether) to afford the title compound (16 mg, 75%). LCMS (ES⁺) Method 1: *m*/*z* 481 (M+H)⁺, RT 2.07 min.

### Step 4: (S)-1-(5-(3-fluorophenoxy)pyrazin-2-yl)-4'H,6'H-spiro[piperidine-4,5'-pyrrolo[1,2-b]pyrazol]-4'-amine

A solution of *tert-butyl* (*S*)-(1-(5-(3-fluorophenoxy)pyrazin-2-yl)-4'H,6'H-spiro[piperidine-4,5'-pyrrolo[1,2-b]pyrazol]-4'-yl)carbamate (16 mg, 0.03 mmol) in DCM (0.2 mL) was treated with TFA (67 µL) and stirred at rt for 2 h before being evaporated and purified by preparative HPLC-MS (from 15% to 30% MeCN/ H₂O + 0.1% TFA) to obtain the title compound as a pale yellow powder (5.3 mg, 42%). ¹H NMR (400 MHz, DMSO-*d₆*) δ 8.33 (br s, 3H), 8.11 (s, 1H), 7.88 (s, 1H), 7.52 (s, 1H), 7.31-7.28 (m, 1H), 7.21-7.16 (m, 3H), 6.25 (s, 1H), 4.39 (br s, 1H), 4.30 (br d, *J*=11.4 Hz, 1H), 4.17-4.10 (m, 2H), 4.02 (br d, *J*=14.5 Hz, 1H), 3.18-3.12 (m, 1H), 3.03-2.97 (m, 1H), 1.80-1.69 (m, 3H), 1.58-1.55 (m, 1H). LCMS (ES⁺) Method 1: *m*/*z* 381 (M+H)⁺, RT 1.13 min.

**Example 79** was synthesized using the above procedure with the corresponding starting materials:

| **Example #** | **¹H NMR (400 MHz, DMSO-*d₆***) |
|---|---|
| 79 | δ 8.40 (br s, 3H), 8.19 (s, 1H), 7.94 (s, 1H), 7.63-7.58 (m, 2H), 7.32 (br s, 2H), 6.31 (s, 1H), 4.46 (br s, 1H), 4.36 (br d, *J*=1 1.4 Hz, 1H), 4.24-4.17 (m, 2H), 4.09 (br d, *J*=14.3 Hz, 1H), 3.24-3.18 (m, 1H), 3.10-3.04 (m, 1H), 1.86-1.75 (m, 3H), 1.65-1.61 (m, 1H). |

### Biology

### SHP2 inhibition enzymatic assay

The assay was performed as described in YP Chen et al, Nature (535) 2016. Assay volume of 20µL/well was assembled in 384 well black polystyrene low-binding microplates (Greiner), using the following buffer: 60 mM HEPES pH 7.2, 75 mM NaCl, 75 mM KCl, 1 mM EDTA pH 8, 0.05% tween-20, 5 mM DTT. The SHP-2 enzyme (synthetized by Origene, Met1 - Leu525, cat#TP750155) was used at a final concentration of 0.5 nM. The enzyme was activated by 500 nM IRS1 peptide (sequence: H2N-LN(pY)IDLDLV(dPEG8)LST(pY)ASINFQK-amide; Wang et al., iScience 25,104009, April 15, 2022, https://doi.org/10.1016/j.isci.2022.104009) and incubated with 75 µM DiFMUP (Sigma) as substrate. Briefly, DMSO serially diluted testing compounds were transferred to the bottom of the assay plate. SHP2 was then added together with the IRS1 peptide 30 min post incubation, the DiFMUP substrate was added to the reaction and incubated 30 min at rt. Finally 5 µL of 160 µM bpV (potassium bisperoxo[1,10-phenanthroline]oxovanadate [V], Sigma) were added to stop and quench the reaction. The fluorescence was detected by a microplate reader (Envision, PerkinElmer) according to the DiFMUP excitation and emission wavelength. The lower the fluorescence the higher the SHP2 inhibition.

The activity of each compound dilution was calculated as percentage of inhibition between vehicle (DMSO, 0% inhibition) and no enzyme (100% inhibition). The percentage inhibition is fitted against the compound dilutions with a four-parameter logistic regression. The inflection point (i.e. the concentration at which half-maximal inhibition is achieved) is the IC₅₀.

The IC₅₀ results of the compounds of the invention in the SHP2 inhibition enzymatic assay are shown in **Table 2.** Legend: A indicates IC₅₀ less or equal to 0.05 µM; B indicates IC₅₀ greater than 0.05 µM and lower or equal to 0.3 µM.

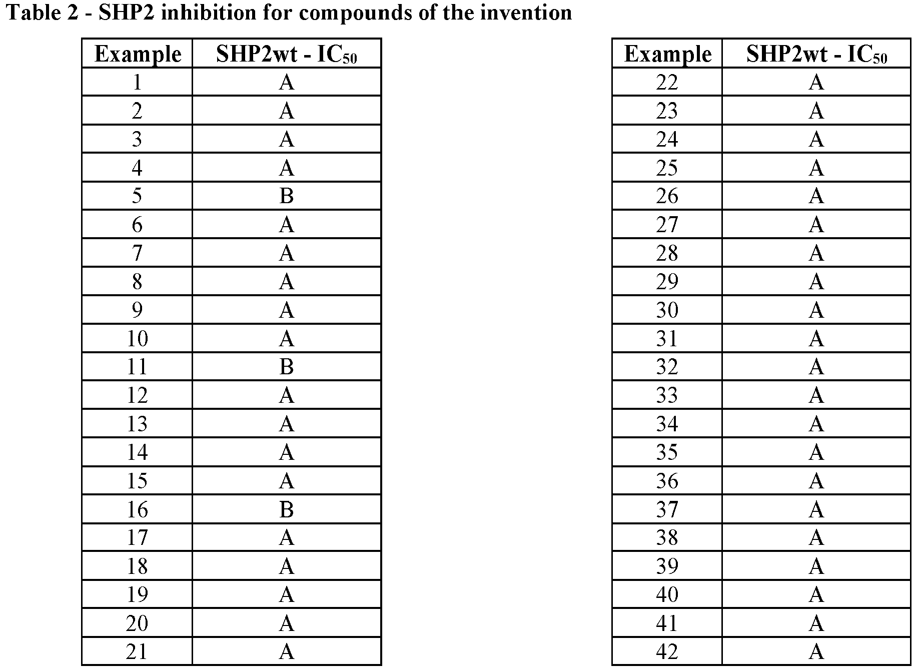

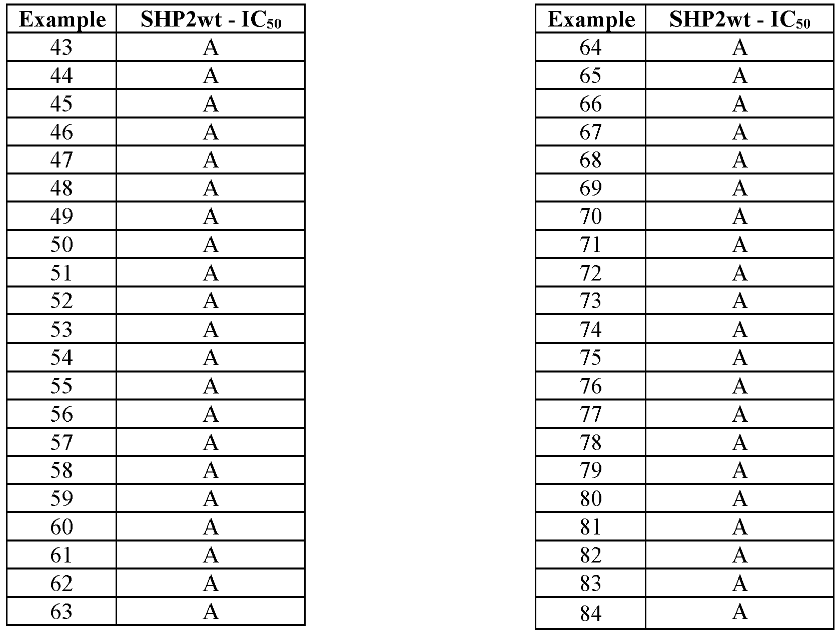

### Phospho-ERK cellular assay

ERK phosphorylation was detected using the "Advanced phospho-ERK1/2 (Thr202/Tyr204)" TR-FRET kit (Cisbio, Cat #64AERPEG/H), following the manufacturer reagents and instructions.

Briefly, 20.000/well KYSE-520 cells (DSMZ ACC 371) were plated in 6 µL RPMI-1640 (Invitrogen) growth medium, into 384 white low-volume high base TC microplates (Greiner). After an overnight incubation, cells were treated with DMSO serial diluted compounds and incubated for 2 h at 37 °C. After incubation, 2 µE/well of 4X Lysis Buffer (Cisbio 64KL1FDF), were added and incubated with cells for 30 min on gentle shaking. Finally, lysates were added with 2 µL/well of Eu cryptate (donor) and D2 (acceptor) conjugated antibodies (as provided by the Cisbio kit #64AERPEG/H) diluted 1:100 in Detection Buffer (as provided by the Cisbio kit #64AERPEG/H). Plates were then sealed and incubated at rt in the dark. After an overnight incubation, the TR-FRET signal was detected on a suitable reader (Envision, PerkinElmer). The lower the TR-FRET signal the higher the higher the SHP2 inhibition in cells.

The activity of each compound dilution was calculated as percentage between vehicle DMSO treated cells and no cells, 0% inhibition and 100% inhibition respectively. The percentage activity is fitted against the compound dilutions with a four-parameter logistic regression. The inflection point (i.e. the concentration at which half-maximal inhibition is achieved) is the IC₅₀.

The IC₅₀ results of the compounds of the invention in the phospho-ERK cellular assay are shown in Table 3. Legend: "+" indicates IC₅₀ equal or higher than 0.5 µM; "++" indicates IC₅₀ less than 0.5 µM and higher or equal to 0.1µM; "+++" indicates IC₅₀ less than 0.1µM.

**Table 3 - pERK activity for compounds of the invention**

| **Example** | **pERK IC₅₀** |
|---|---|
| 1 | ++ |
| 2 | ++ |
| 3 | +++ |
| 4 | ++ |
| 5 | + |
| 6 | ++ |
| 7 | +++ |
| 8 | + |
| 9 | + |
| 10 | +++ |
| 11 | + |
| 12 | ++ |
| 13 | +++ |
| 14 | ++ |
| 15 | + |
| 16 | + |
| 17 | ++ |
| 18 | ++ |
| 19 | ++ |
| 20 | +++ |
| 21 | +++ |
| 22 | +++ |
| 23 | ++ |
| 24 | +++ |
| 25 | +++ |
| 26 | +++ |
| 27 | +++ |
| 28 | +++ |
| 29 | +++ |
| 30 | +++ |
| 31 | + |
| 32 | +++ |
| 33 | +++ |
| 34 | +++ |
| 35 | +++ |
| 36 | +++ |
| 37 | +++ |
| 38 | ++ |
| 39 | +++ |
| 40 | ++ |
| 41 | +++ |
| 42 | +++ |
| 43 | +++ |
| 44 | +++ |
| 45 | +++ |
| 46 | +++ |
| 47 | +++ |
| 48 | +++ |
| 49 | +++ |
| 50 | +++ |
| 51 | ++ |
| 52 | +++ |
| 53 | +++ |
| 54 | +++ |
| 55 | +++ |
| 56 | +++ |
| 57 | +++ |
| 58 | +++ |
| 59 | ++ |
| 60 | +++ |
| 61 | +++ |
| 62 | ++ |
| 63 | +++ |
| 64 | +++ |
| 65 | +++ |
| 66 | +++ |
| 67 | +++ |
| 68 | +++ |
| 69 | +++ |
| 70 | +++ |
| 71 | +++ |
| 72 | +++ |
| 73 | +++ |
| 74 | ++ |
| 75 | +++ |
| 76 | +++ |
| 77 | ++ |
| 78 | ++ |
| 79 | ++ |
| 80 | ++ |
| 81 | +++ |
| 82 | +++ |
| 83 | +++ |
| 84 | +++ |

### Binding kinetic Assay by Surface Plasmon Resonance (SPR)

A N-term biotinylated recombinant SHP2 protein (AviTag-G4SG4S-SHP2(1-525)) was custom produced by VIVA Biotech Ltd. Binding kinetic rate constants were measured by SPR, using a Biacore T200 instrument. The recombinant SHP2 protein was diluted at 5 µg/mL in HBS-P+ buffer, then captured on a series S streptavidin sensor chip up to 5000 RU. Binding of compounds was assessed by a single cycle kinetic procedure (SCK): compounds were diluted in HBS-EP+ supplemented with 2% DMSO (from 300 to 3.7 nM, 3 fold dilution), injected for 120 s over the ligand, then dissociation rate measured for 3600 s, at 50 µL/min and 25°C. Sensorgrams were analysed by Biacore T200 evaluation software: binding rate constants (kon, koff) and affinity (KD) were calculated by curve fitting according to a 1:1 Langmuir model. Each molecule was tested in triplicate on separate flow channels.

| **Examples** | **koff SPR (1/s)** | **Residence time 1/koff SPR (s)** |
|---|---|---|
| 2 | 5.14E-04 | 1944 |
| 3 | 1.24E-04 | 8065 |
| 10 | 1.10E-05 | 90909 |
| 21 | 9.09E-05 | 11001 |
| 36 | 1.87E-05 | 53571 |
| 39 | 1.83E-04 | 5474 |
| 44 | 2.93E-04 | 3417 |
| 45 | 7.90E-05 | 12664 |

## Claims

1. A compound of Formula (I): 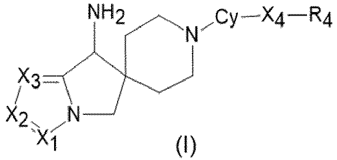 wherein:
X₁ is CR₁ or N; X₂ is CR₂; X₃ is CR₃;
R₁, R₂ and R₃ are each selected from H, halogen, OC₁₋₃alkyl or C₁₋₃alkyl; preferably R₁, R₂ and R₃ are each selected from H and halogen;
X₄ is O, S or a bond;
R₄ is an aryl, heteroaryl, partially unsaturated aryl, partially unsaturated heteroaryl ring, each of said aryl, heteroaryl, partially unsaturated aryl, partially unsaturated heteroaryl ring being optionally substituted with one or more substituents independently selected from C(O)CH₃, C(O)OCH₃, OH, halogen, NH₂, NH-C₁₋₆alkyl, N(C₁₋₆alkyl)₂, C₁₋₆alkyl, C₃₋₅cycloalkyl, haloC₁₋₆alkyl, hydroxyC₁₋₆alkyl, CONR'R", CN, haloC₁₋₆alkoxy, C₁₋₆alkoxy, saturated 5- or 6-membered heterocyclic ring, aryl or heteroaryl wherein each of said saturated 5- or 6-membered heterocyclic ring, aryl or heteroaryl is optionally substituted with one or more CH₃, NH₂, halogen or OH and wherein R' and R" are each independently H or C₁₋₆alkyl, preferably H or CH₃;
Cy-X₄-R₄ corresponds to anyone of general formulae (A) - (F) as indicated below: 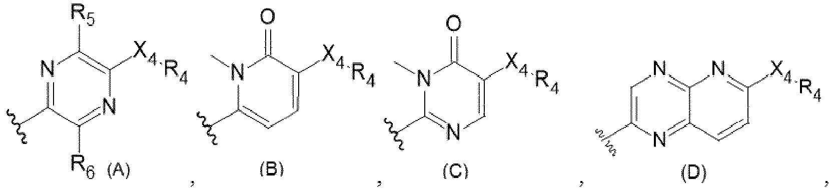 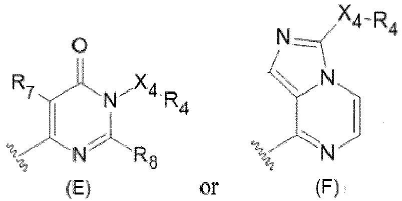 wherein:
- R₅ is selected from H, C₁₋₃alkyl and NH₂; preferably from H, CH₃ and NH₂;
- R₆ R₇, R₈ are each independently selected from H and C₁₋₃alkyl;
or a pharmaceutically acceptable salt, solvate, or stereoisomer thereof.

2. The compound of formula (I) according to claim 1 wherein R₄ is selected from: phenyl, pyridine, pyrimidine, pyrazine, quinoline, imidazo[1,2-a]pyridine, indole, 2H-indazole, 2,3-dihydroindole, 2,3,3a,4-tetrahydro-1H-pyrrolo[2,1-c][1,4]benzoxazine, 6a,7,8,9-tetrahydro-6H-pyrido[3,2-b]pyrrolo[1,2-d][1,4]oxazine, 2,3-dihydro-1H-pyrrolo[2,3-b]pyridine, 1,8-napthyridine, triazolo[4,3-a]pyridine, imidazo[1,2-a]pyridin-2(3H)-one, each of said ring being optionally substituted with one or more substituents independently selected from C(=O)CH₃, C(=O)OCH₃, OH, halogen, NH₂, CF₃, CHF₂, C₁₋₃alkyl, C₁₋₆alkoxy, C(=O)NH₂, C(=O)NHC₁₋₃alkyl, C(=O)N(C₁₋₃alkyl)₂, CN, C₃₋₅cycloalkyl, aryl or heteroaryl wherein each of said aryl or heteroaryl is optionally substituted with one or more halogen, C(=O)NH₂, C(=O)NHCH₃, C(=O)N(CH₃)₂, C₁₋₃alkyl and NHC(=O)CF₃.

3. The compound of formula (I) according to claim 2 wherein R₄ is selected from the group consisting of: 2,3-dichlorophenyl, 3-fluoro-2-methylpyridin-4-yl, 2,3-difluorophenyl, 5-(4-fluorophenyl)pyrazin-2-yl, 2-chloro-3-(1H)-pyrazolyl, 2-fluoro-3-methylphenyl, 4-fluorophenyl, 2-chloro-3-fluorophenyl, 3-chloro-2-methylphenyl, 2-chloro-6-fluoro-3 -methylphenyl, 4-(trifluoromethoxy)phenyl, 4-(trifluoromethyl)phenyl, 3-chlorophenyl, 2-fluorophenyl, 2,4-dichlorophenyl, 3,4-dichlorophenyl, tolyl, phenyl, pyridyl, 2,4-difluorophenyl, 2-chloro-4-fluorophenyl, 2-chloro-3-methylphenyl, 8-methoxyquinolin-4-yl, 2,3-dihydrobenzofuran-4-yl, 5-quinolin-4-yl, 4-chloro-2-methylphenyl, 2-(trifluoromethyl)phenyl, 2,3-dihydrobenzofuran-5-yl, trifluoromethyl-benzonitrile, 2-chloro-3-(1H-imidazol-1-yl)phenyl, 2,4-difluoro-3-methylphenyl, 2,3-dihydro-1H-inden-4-yl, 2,3-dimethylphenyl, 2,5-difluorophenyl, 2,3,4-trifluorophenyl, 3,5-difluorophenyl, 3-fluorophenyl, 1,3-dihydroisobenzofuran-4-yl, 3-chloro-2-(trifluoromethyl)pyridin-4-yl, 2,4-difluoro-3-methoxyphenyl, 3-chloro-2-(trifluoromethyl)phenyl, 3-fluoro-2-methylphenyl, 3-(difluoromethyl)-2-fluorophenyl, 4-(difluoromethyl)-2-fluorophenyl, 2-fluoro-3-methoxyphenyl, quinolinyl, 5-chloro-4-methylpyridin-3-yl, 2-chlorophenyl, 2-fluoro-4-methoxyphenyl, 1-methyl-1H-benzo[d]imidazol-6-yl, 8-fluoroquinolin-5-yl, 3-cyclopropyl-2-fluorophenyl, 8-chloroquinolin-5-yl, 2-(difluoromethyl)-3-fluorophenyl, 2-(difluoromethyl)phenyl, 8-(difluoromethoxy)quinolin-5-yl, 5-(difluoromethyl)-2-fluorophenyl, 2,6-difluorophenyl, 3-fluorobenzonitrile, 2,4,5-trifluorophenyl, 4-chloro-2-fluorophenyl, 3-fluoro-2-(1H-pyrazol-1-yl)phenyl, 2-fluoro-3-(1H-pyrazol-1-yl)phenyl, 2-chloro-3-(2-(difluoromethyl)-1H-imidazol-1-yl)phenyl, 7-fluoro-2-methyl-2H-indazol-6-yl, 4-chloro-2-methyl-2H-indazol-5-yl.

4. The compound according to any one of claims 1-3 wherein R₄ is selected from phenyl, pyridine, quinoline, each of said ring being optionally substituted with one or more substituents independently selected from C(=O)OCH₃, OH, OC₁₋₃alkyl, halogen, NH₂, CF₃, CHF₂, C₁₋₃alkyl, C(=O)NH₂, C(=O)NHC₁₋₃alkyl, C(=O)N(C₁₋₃alkyl)₂, CN, C₃₋₅cycloalkyl, heteroaryl.

5. A compound of formula (I) as defined in claim 1 selected from:
- (S)-6-(4'-amino-3'-fluoro-4'H,6'H-spiro[piperidine-4,5'-pyrrolo[1,2-b]pyrazol]-1-yl)-3-(2,3-dichlorophenyl)-2-methylpyrimidin-4(3H)-one;
- (S)-1-(5-(2,3-dichlorophenoxy)pyrazin-2-yl)-4'H,6'H-spiro[piperidine-4,5'-pyrrolo[1,2-b]pyrazol]-4'-amine;
- (S)-6-(4'-amino-4'H,6'H-spiro[piperidine-4,5'-pyrrolo[1,2-b]pyrazol]-1-yl)-3-(2,3-dichlorophenyl)-2,5-dimethylpyrimidin-4(3H)-one;
- (S)-1-(5-((3-fluoro-2-methylpyridin-4-yl)thio)-6-methylpyrazin-2-yl)-4'H,6'H-spiro[piperidine-4,5'-pyrrolo[1,2-b]pyrazol]-4'-amine;
- (S)-2-(4'-amino-4'H,6'H-spiro[piperidine-4,5'-pyrrolo[1,2-b]pyrazol]-1-yl)-5-(2,3-difluorophenyl)-3-methylpyrimidin-4(3H)-one;
- (S)-1-(5-((2,3-dichlorophenyl)thio)-6-methylpyrazin-2-yl)-4'H,6'H-spiro[piperidine-4,5'-pyrrolo[1,2-b]pyrazol]-4'-amine;
- (S)-1-(5-(4-fluorophenoxy)pyrazin-2-yl)-4'H,6'H-spiro[piperidine-4,5'-pyrrolo[1,2-b]pyrazol]-4'-amine;
- (S)-1-(6-(2,3-difluorophenyl)pyrido[2,3-b]pyrazin-2-yl)-4'H,6'H-spiro[piperidine-4,5'-pyrrolo[1,2-b]pyrazol]-4'-amine;
- (S)-1-(6-(2,3-dichlorophenyl)pyrido[2,3-b]pyrazin-2-yl)-4'H,6'H-spiro[piperidine-4,5'-pyrrolo[1,2-b]pyrazol]-4'-amine;
- (S)-1-(5-((2-chloro-3-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-6-(4'-amino-4'H,6'H-spiro[piperidine-4,5'-pyrrolo[1,2-b]pyrazol]-1-yl)-3-(2,3-dichlorophenyl)-1-methylpyridin-2(1H)-one;
- (S)-1-(3-(2-fluoro-3-methylphenyl)imidazo[1,5-a]pyrazin-8-yl)-4'H,6'H-spiro[piperidine-4,5'-pyrrolo[1,2-b]pyrazol]-4'-amine;
- (S)-1-(5-((4-fluorophenyl)thio)pyrazin-2-yl)-4'H,6'H-spiro[piperidine-4,5'-pyrrolo[1,2-b]pyrazol]-4'-amine;
- (S)-1-(3-(2-chloro-3-fluorophenyl)imidazo[1,5-a]pyrazin-8-yl)-4'H,6'H-spiro[piperidine-4,5'-pyrrolo[1,2-b]pyrazol]-4'-amine;
- (S)-1-(3-(3-chloro-2-methylphenyl)imidazo[1,5-a]pyrazin-8-yl)-4'H,6'H-spiro[piperidine-4,5'-pyrrolo[1,2-b]pyrazol]-4'-amine;
- (S)-1-(3-(2-chloro-6-fluoro-3-methylphenyl)imidazo[1,5-a]pyrazin-8-yl)-4'H,6'H-spiro[piperidine-4,5'-pyrrolo[1,2-b]pyrazol]-4'-amine;
- (S)-1-(5-((2,3-dichlorophenyl)thio)-3-methylpyrazin-2-yl)-4'H,6'H-spiro[piperidine-4,5'-pyrrolo[1,2-b]pyrazol]-4'-amine;
- (S)-1-(5-((4-(trifluoromethoxy)phenyl)thio)pyrazin-2-yl)-4'H,6'H-spiro[piperidine-4,5'-pyrrolo[1,2-b]pyrazol]-4'-amine;
- (S)-1-(5-((4-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((3-chlorophenyl)thio)pyrazin-2-yl)-4'H,6'H-spiro[piperidine-4,5'-pyrrolo[1,2-b]pyrazol]-4'-amine;
- (S)-1-(5-((2-fluorophenyl)thio)pyrazin-2-yl)-4'H,6'H-spiro[piperidine-4,5'-pyrrolo[1,2-b]pyrazol]-4'-amine;
- (S)-1-(5-((2,4-dichlorophenyl)thio)pyrazin-2-yl)-4'H,6'H-spiro[piperidine-4,5'-pyrrolo[1,2-b]pyrazol]-4'-amine;
- (S)-1-(5-((3,4-dichlorophenyl)thio)pyrazin-2-yl)-4'H,6'H-spiro[piperidine-4,5'-pyrrolo[1,2-b]pyrazol]-4'-amine;
- (S)-1-(5-(phenylthio)pyrazin-2-yl)-4'H,6'H-spiro[piperidine-4,5'-pyrrolo[1,2-b]pyrazol]-4'-amine;
- (S)-1-(5-(pyridin-4-ylthio)pyrazin-2-yl)-4'H,6'H-spiro[piperidine-4,5'-pyrrolo[1,2-b]pyrazol]-4'-amine;
- (S)-1-(5-(o-tolylthio)pyrazin-2-yl)-4'H,6'H-spiro[piperidine-4,5'-pyrrolo[1,2-b]pyrazol]-4'-amine;
- (S)-1-(5-((2-isopropylphenyl)thio)pyrazin-2-yl)-4'H,6'H-spiro[piperidine-4,5'-pyrrolo[1,2-b]pyrazol]-4'-amine;
- (S)-1-(5-((2,3-dichlorophenyl)thio)pyrazin-2-yl)-1'H,3'H-spiro[piperidine-4,2'-pyrrolizin]-1'-amine;
- (S)-1-(5-((2,3-dichlorophenyl)thio)pyrazin-2-yl)-7'-fluoro-1'H,3'H-spiro[piperidine-4,2'-pyrrolizin]-1'-amine;
- (S)-1-(5-((2-chloro-3-fluorophenyl)thio)pyrazin-2-yl)-4'H,6'H-spiro[piperidine-4,5'-pyrrolo[1,2-b]pyrazol]-4'-amine;
- (S)-1-(5-(2,3-dichlorophenyl)pyrazin-2-yl)-4'H,6'H-spiro[piperidine-4,5'-pyrrolo[1,2-b]pyrazol]-4'-amine;
- (S)-1-(5-((2,4-difluorophenyl)thio)pyrazin-2-yl)-4'H,6'H-spiro[piperidine-4,5'-pyrrolo[1,2-b]pyrazol]-4'-amine;
- (S)-1-(5-((3-chloro-2-methylphenyl)thio)pyrazin-2-yl)-4'H,6'H-spiro[piperidine-4,5'-pyrrolo[1,2-b]pyrazol]-4'-amine;
- (S)-1-(5-((2-chloro-4-fluorophenyl)thio)pyrazin-2-yl)-4'H,6'H-spiro[piperidine-4,5'-pyrrolo[1,2-b]pyrazol]-4'-amine;
- (S)-1-(5-((2-chloro-3-methylphenyl)thio)pyrazin-2-yl)-4'H,6'H-spiro[piperidine-4,5'-pyrrolo[1,2-b]pyrazol]-4'-amine;
- (S)-1-(5-((2-fluoro-3-methylphenyl)thio)pyrazin-2-yl)-4'H,6'H-spiro[piperidine-4,5'-pyrrolo[1,2-b]pyrazol]-4'-amine;
- (S)-1-(5-((8-methoxyquinolin-4-yl)thio)pyrazin-2-yl)-4'H,6'H-spiro[piperidine-4,5'-pyrrolo[1,2-b]pyrazol]-4'-amine;
- (S)-1-(5-((2,3-dihydrobenzofuran-4-yl)thio)pyrazin-2-yl)-4'H,6'H-spiro[piperidine-4,5'-pyrrolo[1,2-b]pyrazol]-4'-amine;
- (S)-1-(5-(quinolin-4-ylthio)pyrazin-2-yl)-4'H,6'H-spiro[piperidine-4,5'-pyrrolo[1,2-b]pyrazol]-4'-amine;
- (S)-1-(5-((4-chloro-2-methylphenyl)thio)pyrazin-2-yl)-4'H,6'H-spiro[piperidine-4,5'-pyrrolo[1,2-b]pyrazol]-4'-amine;
- (S)-1-(5-((2-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((2,3-dihydrobenzofuran-5-yl)thio)pyrazin-2-yl)-4'H,6'H-spiro[piperidine-4,5'-pyrrolo[1,2-b]pyrazol]-4'-amine;
- (S)-3-((5-(4'-amino-4'H,6'H-spiro[piperidine-4,5'-pyrrolo[1,2-b]pyrazol]-1-yl)pyrazin-2-yl)thio)-2-(trifluoromethyl)benzonitrile;
- (S)-1-(5-((2-chloro-3-(1H-imidazol-1-yl)phenyl)thio)pyrazin-2-yl)-4'H,6'H-spiro[piperidine-4,5'-pyrrolo[1,2-b]pyrazol]-4'-amine;
- (S)-1-(5-((2,4-difluoro-3-methylphenyl)thio)pyrazin-2-yl)-4'H,6'H-spiro[piperidine-4,5'-pyrrolo[1,2-b]pyrazol]-4'-amine;
- (S)-1-(5-((2,3-dihydro-1H-inden-4-yl)thio)pyrazin-2-yl)-4'H,6'H-spiro[piperidine-4,5'-pyrrolo[1,2-b]pyrazol]-4'-amine;
- (S)-1-(5-((2,3-dimethylphenyl)thio)pyrazin-2-yl)-4'H,6'H-spiro[piperidine-4,5'-pyrrolo[1,2-b]pyrazol]-4'-amine;
- (S)-1-(5-((2,5-difluorophenyl)thio)pyrazin-2-yl)-4'H,6'H-spiro[piperidine-4,5'-pyrrolo[1,2-b]pyrazol]-4'-amine;
- (S)-1-(5-((2,3,4-trifluorophenyl)thio)pyrazin-2-yl)-4'H,6'H-spiro[piperidine-4,5'-pyrrolo[1,2-b]pyrazol]-4'-amine;
- (S)-1-(5-((3,5-difluorophenyl)thio)pyrazin-2-yl)-4'H,6'H-spiro[piperidine-4,5'-pyrrolo[1,2-b]pyrazol]-4'-amine;
- (S)-1-(5-((3-fluorophenyl)thio)pyrazin-2-yl)-4'H,6'H-spiro[piperidine-4,5'-pyrrolo[1,2-b]pyrazol]-4'-amine;
- (S)-1-(5-((1,3-dihydroisobenzofuran-4-yl)thio)pyrazin-2-yl)-4'H,6'H-spiro[piperidine-4,5'-pyrrolo[1,2-b]pyrazol]-4'-amine;
- (S)-1-(5-((2,3-difluorophenyl)thio)pyrazin-2-yl)-4'H,6'H-spiro[piperidine-4,5'-pyrrolo[1,2-b]pyrazol]-4'-amine;
- (S)-1-(5-((3-chloro-2-(trifluoromethyl)pyridin-4-yl)thio)pyrazin-2-yl)-4'H,6'H-spiro[piperidine-4,5'-pyrrolo[1,2-b]pyrazol]-4'-amine;
- (S)-1-(5-((2,4-difluoro-3-methoxyphenyl)thio)pyrazin-2-yl)-4'H,6'H-spiro[piperidine-4,5'-pyrrolo[1,2-b]pyrazol]-4'-amine;
- (S)-1-(5-((3-chloro-2-(trifluoromethyl)phenyl)thio)pyrazin-2-yl)-4'H,6'H-spiro[piperidine-4,5'-pyrrolo[1,2-b]pyrazol]-4'-amine;
- (S)-1-(5-((3-fluoro-2-methylphenyl)thio)pyrazin-2-yl)-4'H,6'H-spiro[piperidine-4,5'-pyrrolo[1,2-b]pyrazol]-4'-amine;
- (S)-1-(5-((3-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((4-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((2-fluoro-3-methoxyphenyl)thio)pyrazin-2-yl)-4'H,6'H-spiro[piperidine-4,5'-pyrrolo[1,2-b]pyrazol]-4'-amine;
- (S)-1-(5-(quinolin-5-ylthio)pyrazin-2-yl)-4'H,6'H-spiro[piperidine-4,5'-pyrrolo[1,2-b]pyrazol]-4'-amine;
- (S)-1-(5-((5-chloro-4-methylpyridin-3-yl)thio)pyrazin-2-yl)-4'H,6'H-spiro[piperidine-4,5'-pyrrolo[1,2-b]pyrazol]-4'-amine;
- (S)-1-(5-((2-chlorophenyl)thio)pyrazin-2-yl)-4'H,6'H-spiro[piperidine-4,5'-pyrrolo[1,2-b]pyrazol]-4'-amine;
- (S)-1-(5-((2-fluoro-4-methoxyphenyl)thio)pyrazin-2-yl)-4'H,6'H-spiro[piperidine-4,5'-pyrrolo[1,2-b]pyrazol]-4'-amine;
- (S)-1-(5-((1-methyl-1H-benzo[d]imidazol-6-yl)thio)pyrazin-2-yl)-4'H,6'H-spiro[piperidine-4,5'-pyrrolo[1,2-b]pyrazol]-4'-amine;
- (S)-5-((5-(4'-amino-4'H,6'H-spiro[piperidine-4,5'-pyrrolo[1,2-b]pyrazol]-1-yl)pyrazin-2-yl)thio)quinoline-8-carbonitrile;
- (S)-1-(5-((8-(trifluoromethyl)quinolin-5-yl)thio)pyrazin-2-yl)-4'H,6'H-spiro[piperidine-4,5'-pyrrolo[1,2-b]pyrazol]-4'-amine;
- (S)-1-(5-((8-fluoroquinolin-5-yl)thio)pyrazin-2-yl)-4'H,6'H-spiro[piperidine-4,5'-pyrrolo[1,2-b]pyrazol]-4'-amine;
- (S)-1-(5-((3-cyclopropyl-2-fluorophenyl)thio)pyrazin-2-yl)-4'H,6'H-spiro[piperidine-4,5'-pyrrolo[1,2-b]pyrazol]-4'-amine;
- (S)-1-(5-((8-chloroquinolin-5-yl)thio)pyrazin-2-yl)-4'H,6'H-spiro[piperidine-4,5'-pyrrolo[1,2-b]pyrazol]-4'-amine;
- (S)-1-(5-((2-(difluoromethyl)-3-fluorophenyl)thio)pyrazin-2-yl)-4'H,6'H-spiro[piperidine-4,5'-pyrrolo[1,2-b]pyrazol]-4'-amine;
- (S)-1-(5-((2-(difluoromethyl)phenyl)thio)pyrazin-2-yl)-4'H,6'H-spiro[piperidine-4,5'-pyrrolo[1,2-b]pyrazol]-4'-amine;
- (S)-1-(5-((8-(difluoromethoxy)quinolin-5-yl)thio)pyrazin-2-yl)-4'H,6'H-spiro[piperidine-4,5'-pyrrolo[1,2-b]pyrazol]-4'-amine;
- (S)-1-(5-((5-(difluoromethyl)-2-fluorophenyl)thio)pyrazin-2-yl)-4'H,6'H-spiro[piperidine-4,5'-pyrrolo[1,2-b]pyrazol]-4'-amine;
- (S)-1-(5-((2,6-difluorophenyl)thio)pyrazin-2-yl)-4'H,6'H-spiro[piperidine-4,5'-pyrrolo[1,2-b]pyrazol]-4'-amine;
- (S)-4-((5-(4'-amino-4'H,6'H-spiro[piperidine-4,5'-pyrrolo[1,2-b]pyrazol]-1-yl)pyrazin-2-yl)thio)-3 -fluorobenzonitrile;
- (S)-1-(5-((2,4,5-trifluorophenyl)thio)pyrazin-2-yl)-4'H,6'H-spiro[piperidine-4,5'-pyrrolo[1,2-b]pyrazol]-4'-amine;
- (S)-1-(5-(2-fluorophenoxy)pyrazin-2-yl)-4'H,6'H-spiro[piperidine-4,5'-pyrrolo[1,2-b]pyrazol]-4'-amine;
- (S)-1-(5-(4-chloro-2-fluorophenoxy)pyrazin-2-yl)-4'H,6'H-spiro[piperidine-4,5'-pyrrolo[1,2-b]pyrazol]-4'-amine;
- (S)-1-(5-((3-fluoro-2-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-1-(5-((2-fluoro-3-(1H-pyrazol-1-yl)phenyl)thio)pyrazin-2-yl)-4'H,6'H-spiro[piperidine-4,5'-pyrrolo[1,2-b]pyrazol]-4'-amine;
- (S)-1-(5-((2-chloro-3-(2-(difluoromethyl)-1H-imidazol-1-yl)phenyl)thio)pyrazin-2-yl)-4'H,6'H-spiro[piperidine-4,5'-pyrrolo[1,2-b]pyrazol]-4'-amine;
- (S)-1-(5-((7-fluoro-2-methyl-2H-indazol-6-yl)thio)pyrazin-2-yl)-4'H,6'H-spiro[piperidine-4,5'-pyrrolo[1,2-b]pyrazol]-4'-amine;
- (S)-1-(5-((4-chloro-2-methyl-2H-indazol-5-yl)thio)pyrazin-2-yl)-4'H,6'H-spiro[piperidine-4,5'-pyrrolo[1,2-b]pyrazol]-4'-amine;
or a pharmaceutically acceptable salt, solvate, or stereoisomer thereof.

6. The compound or a pharmaceutically acceptable salt, solvate, or stereoisomer thereof as defined in any one of previous claims being an inhibitor of SHP2, preferably being a brain penetrant inhibitor of SHP2.

7. The compound or a pharmaceutically acceptable salt, solvate, or stereoisomer thereof as defined in any one of previous claims for medical use.

8. The compound or the pharmaceutically acceptable salt, solvate, or stereoisomer thereof according to any one of previous claims for use in the treatment and/or prevention of a disease or disorder mediated by the activity of SHP2.

9. The compound or the pharmaceutically acceptable salt, solvate, or stereoisomer thereof according to any one of the previous claims for use in the treatment and/or prevention of a disease or disorder selected from the group consisting of: cancer, cardiovascular disease, immunological disorder, autoimmune disorder, fibrosis, an ocular disorder, systemic lupus erythematosus, diabetes, neutropenia and combinations thereof.

10. The compound or the pharmaceutically acceptable salt, solvate, or stereoisomer thereof for use according to claims 8 or 9, wherein the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer, brain cancer and combinations thereof.

11. The compound or the pharmaceutically acceptable salt, solvate, or stereoisomer thereof for use according to claims 9 or 10 wherein any one of said cancers is a primary cancer or a cancer metastasis.

12. The compound or the pharmaceutically acceptable salt, solvate, or stereoisomer thereof for use according to any one of claims 8-11, wherein said use is in combination with radiotherapy or with at least one further therapeutic agent, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

13. A pharmaceutical composition comprising the compound or the pharmaceutically acceptable salt, solvate, or stereoisomer thereof as defined in anyone of claims 1-5, alone or in combination with at least one further therapeutic agent, and at least one pharmaceutically acceptable excipient, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

14. The pharmaceutical composition according to claim 13 for the use as defined in any one of claims 8-11.
